(19) Europäisches Patentamt
European Patent Office
Office européen des brevets

(11) EP 1 525 193 B1

(12) # FASCICULE DE BREVET EUROPEEN

(45) Date de publication et mention de la délivrance du brevet:
**26.12.2007 Bulletin 2007/52**

(21) Numéro de dépôt: **03750801.7**

(22) Date de dépôt: **11.07.2003**

(51) Int Cl.:
***C07D 277/56*** (2006.01) ***C07D 417/12*** (2006.01)
***A61K 31/426*** (2006.01) ***A61K 31/427*** (2006.01)
***A61K 31/4439*** (2006.01) ***A61P 25/28*** (2006.01)

(86) Numéro de dépôt international:
**PCT/FR2003/002194**

(87) Numéro de publication internationale:
**WO 2004/009565 (29.01.2004 Gazette 2004/05)**

(54) **DERIVES D'ACYLAMINOTHIAZOLE ET LEUR UTILISATION COMME INHIBITEURS DE BETA-AMYLOIDE**

ACYLAMINOTHIAZOLDERIVATE UND IHRE VEWRENDUNG ALS BETA-AMYLOID INHIBITOREN

ACYLAMINOTHIAZOLE DERIVATIVES, PREPARATION AND THERAPEUTIC USE THEREOF

(84) Etats contractants désignés:
**AT BE BG CH CY CZ DE DK EE ES FI FR GB GR HU IE IT LI LU MC NL PT RO SE SI SK TR**
Etats d'extension désignés:
**AL LT LV MK**

(30) Priorité: **17.07.2002 FR 0209061**

(43) Date de publication de la demande:
**27.04.2005 Bulletin 2005/17**

(73) Titulaire: **Sanofi-Aventis**
**75013 Paris (FR)**

(72) Inventeurs:
- **BALTZER, Sylvie**
  **F-67200 Strasbourg (FR)**
- **SCHOENTJES, Bruno**
  **F-76230 Bois-Guillaume (FR)**
- **VAN DORSSELAER, Viviane**
  **F-67100 Strasbourg (FR)**

(74) Mandataire: **Ludwig, Jacques et al**
**Sanofi-Aventis**
**174 avenue de France**
**75013 Paris (FR)**

(56) Documents cités:
**WO-A-98/22430** **US-A- 5 712 270**

- **HIGAKI J N ET AL: "A combinatorial approach to the identification of dipeptide aldehyde inhibitors of beta-amyloid production" JOURNAL OF MEDICINAL CHEMISTRY, vol. 42, no. 19, 23 septembre 1999 (1999-09-23), pages 3889-3898, XP002185434**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention a pour objet des dérivés d'acylaminothiazole leur préparation et leur utilisation en thérapeutique.

WO-A-98/22430 décrit des dérivés d'esters d'amino-acides N-acylés utiles comme inhibiteurs du peptide β-amyloïde.

La présente invention a pour premier objet des composés répondant à la formule générale (I) :

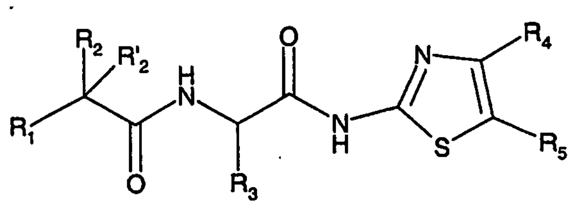

(I)

dans laquelle,

$R_1$ représente :

un $C_{1-6}$ alkyle éventuellement substitué par un à trois substituants choisis parmi un halogène, un trifluorométhyle, un hydroxy, un $C_{1-6}$ alcoxy, un $C_{1-6}$ thioalkyle, un thiophène ou un phényle ; ou
un $C_{3-7}$ cycloalkyle, un thiophène, un benzothiophène, un pyridinyle, un furanyle ou un phényle ;
les groupes phényle étant éventuellement substitués par un à trois substituants choisis parmi un atome d'halogène, un $C_{1-6}$ alkyle, un $C_{1-6}$ alcoxy, un hydroxy, un méthylènedioxy, un phénoxy ou un benzyloxy ;

$R_2$ et $R'_2$ représentent indépendamment l'un de l'autre un atome d'hydrogène, un atome d'halogène, un hydroxy, un $C_{1-3}$ alcoxy, un $C_{1-3}$ alkyle, un $C_{3-7}$ cycloalkyle, un groupe O-C(O)-$C_{1-6}$ alkyle, ou $R_2$ et $R'_2$ forment ensemble un groupe oxo ;
$R_3$ représente un atome d'hydrogène, un $C_{1-6}$ alkyle éventuellement substitué par un hydroxy ou un $C_{1-3}$ alcoxy ;
$R_4$ et $R_5$ représentent, indépendamment l'un de l'autre :

un atome d'hydrogène, un $C_{1-7}$ alkyle éventuellement substitué par un $C_{3-7}$ cycloalkyle ou phényle ; ou
un $C_{3-7}$ cycloalkyle, un phényle, un naphtyle ou un -C(X)$R_6$ ;
les groupes $C_{3-7}$ cycloalkyle et phényle étant éventuellement substitués par un ou plusieurs groupes choisis parmi un atome d'halogène, un hydroxy, un $C_{1-3}$ alkyle, un $C_{1-3}$ alcoxy, un trifluorométhyle, un trifluorométhoxy, un phényle, un phénoxy, un benzyloxy, un $-CH_2O$-phényle, un $-OCH_2$-pyridinyle ;
le groupe benzyloxy étant éventuellement substitué par un à trois groupes choisis parmi un halogène, un trifluorométhyle, un méthyle ;
à la condition qu'au moins un groupe $R_4$ ou $R_5$ représente un groupe -C(X)$R_6$;

X représente un atome d'oxygène ou un atome de soufre ;
$R_6$ représente un groupe $C_{1-6}$ alcoxy, un hydroxy ou un groupe $-NR_7R_8$; le groupe $C_{1-6}$ alcoxy étant éventuellement substitué par un phényle ;
$R_7$ et $R_8$ représentent, indépendamment l'un de l'autre :

un atome d'hydrogène, un groupe $C_{1-6}$ alkyle éventuellement substitué par un $C_{3-7}$ cycloalkyle, un $C_{3-7}$ cycloalkènyle, $C_{1-3}$ alcoxy un phényle, un morpholinyle ou un pyridinyle ; ou
un $C_{3-7}$ cycloalkyle, $C_{1-6}$ alcoxy ou un phényle ; ou

$R_7$ et $R_8$, avec l'atome d'azote qui les porte, forment un cycle aziridine, azétidine, pyrrolidine, pipéridine ou morpholine ;
les groupes $C_{3-7}$ cycloalkyle et phényle étant éventuellement substitués par un ou deux groupes choisis parmi un $C_{1-3}$ alkyle, un hydroxy, un $C_{1-3}$ alcoxy ou un atome d'halogène

Parmi les composés de formule générale (I), un premier groupe de composés préférés sont ceux pour lesquels :

$R_1$ représente :

un $C_{1-5}$ alkyle, de préférence un méthyle, éthyle, 1-méthyléthyle, 2-méthylpropyle, tert-butyle, 1-éthylpropyle, éventuellement substitué par un à trois substituants choisis parmi un fluor, un trifluorométhyle, un hydroxy, un $C_{1-4}$ thioalkyle, de préférence un thiométhyle, un thiophène ou un phényle; ou
un $C_{4-7}$ cycloalkyle, de préférence un cyclopropyle ou un cyclohexyle, un furanyle, un thiophène, un benzothiophène, un pyridinyle ou un phényle ; le phényle étant éventuellement substitué par un à trois substituants choisis parmi un atome d'halogène, de préférence un fluor ou un chlore, un hydroxy, un benzyloxy ou un méthylènedioxy; et/ou

$R_2$ et $R'_2$ représentent indépendamment l'un de l'autre un atome d'hydrogène, un atome d'halogène, de préférence un fluor, un hydroxy, un $C_{1-3}$ alkyle, de préférence un méthyle ou un éthyle, un $C_{3-7}$ cycloalkyle, de préférence un cyclohexyle, un $C_{1-3}$ alcoxy, de préférence un méthoxy, un groupe O-C(O)-$C_{1-4}$ alkyle, de préférence O-C(O)-$CH_3$, ou $R_2$ et $R'_2$ forment ensemble un groupe oxo ; et/ou
$R_3$ représente un $C_{1-4}$ alkyle, de préférence un méthyle, un éthyle, un propyle ou un butyle, éventuellement substitué par un $C_{1-3}$ alcoxy, de préférence un méthoxy ; et/ou
$R_4$ et $R_5$ représentent, indépendamment l'un de l'autre :

un atome d'hydrogène, un $C_{1-7}$ alkyle, de préférence un méthyle, éthyle, 1-méthyléthyle, n-propyle, butyle, 2-méthylpropyle, *ter*-butyle, 3-méthylbutyle, 1-éthylpropyle, 2,2-diméthylpropyle, hexyle, 5-méthylhexyle, éventuellement substitué par un phényle ou un $C_{3-7}$ cycloalkyle, de préférence un cyclohexyle; ou
un $C_{3-7}$ cycloalkyle, de préférence un cyclopropyle ou un cyclohexyle, un phényle, un naphtyle ou un -C(X)$R_6$; le phényle étant éventuellement substitué par un ou deux groupes choisis parmi un halogène, de préférence un brome, un $C_{1-3}$ alkyle, de préférence un méthyle ou un 1-méthyléthyle, un hydroxy, un $C_{1-3}$ alcoxy, de préférence un méthoxy ou éthoxy, un trifluorométhyle, un trifluorométhoxy, un phényle, un phénoxy, un benzyloxy, un -$CH_2$O-phényle, un -O$CH_2$-pyridinyle ;
le groupe benzyloxy étant éventuellement substitué par un groupe choisi parmi un halogène, de préférence un chlore ou un fluor, un trifluorométhyle ou un méthyle ;

à ta condition qu'au moins un groupe $R_4$ ou $R_5$ représente un groupe -C(X)$R_6$; et/ou
X représente un atome d'oxygène ; et/ou
$R_6$ représente un groupe $C_{1-6}$ alcoxy, de préférence un méthoxy, éthoxy, *ter*-butyloxy, un hydroxy ou un groupe -N$R_7R_8$; le groupe $C_{1-6}$ alcoxy étant éventuellement substitué par un phényle ; et/ou
$R_7$ et $R_8$ représentent, indépendamment l'un de l'autre :

un atome d'hydrogène, un groupe $C_{1-3}$ alkyle, de préférence un méthyle, éthyle ou 1-méthyléthyle, éventuellement substitué par un $C_{1-3}$ alcoxy, de préférence un méthoxy, un $C_{3-7}$ cycloalkènyle, de préférence un cyclohexènyle, un phényle, un morpholinyle ou un pyridinyle ; un $C_{1-3}$ alcoxy, de préférence un méthoxy; ou
un $C_{3-6}$cycloalkyle, de préférence un cyclohexyle ; ou
$R_7$ et $R_8$, avec l'atome d'azote qui les porte, forment un cycle azétidine, pipéridine ou morpholine ;
le groupe phényle étant éventuellement substitué par un ou deux groupes $C_{1-3}$ alcoxy, de préférence méthoxy.

Parmi les composés de formule générale (I), un second groupe de composés préférés sont ceux pour lesquels :

$R_1$ représente :

un $C_{1-6}$ alkyle éventuellement substitué par un ou deux substituants choisis parmi un hydroxy, un $C_{1-6}$ alcoxy, un $C_{1-6}$ thioalkyle, un trifluorométhyle, un thiophène, ou un phényle; ou
un $C_{3-7}$ cycloalkyle, un thiophène, un benzothiophène, un pyridinyle, un furanyle ou un phényle ;
les groupes phényle étant éventuellement substitués par un à trois substituants choisis parmi un atome d'halogène, un $C_{1-6}$ alkyle, un $C_{1-6}$ alcoxy, un hydroxy, un méthylènedioxy, un phénoxy ou un benzyloxy; et/ou

$R_2$ et $R'_2$ représentent indépendamment l'un de l'autre un atome d'hydrogène, un atome d'halogène, un hydroxy, un $C_{1-3}$ alcoxy, un $C_{1-3}$ alkyle, un $C_{3-7}$ cycloalkyle, un groupe O-C(O)-$C_{1-6}$ alkyle, ou $R_2$ et $R'_2$ forment ensemble un groupe oxo ; et/ou
$R_3$ représente un atome d'hydrogène ou un $C_{1-6}$ alkyle éventuellement substitué par un hydroxy ou un $C_{1-3}$ alcoxy ;

et/ou
$R_4$ et $R_5$ représentent, indépendamment l'un de l'autre :

un atome d'hydrogène, un $C_{1-7}$ alkyle éventuellement substitué par un $C_{3-7}$ cycloalkyle ou phényle; ou un $C_{3-7}$ cycloalkyle, un phényle, un naphtyl ou un -C(X)$R_6$;
les groupes $C_{3-7}$ cycloalkyle et phényle étant éventuellement substitués par un ou plusieurs groupes choisis parmi un atome d'halogène, un hydroxy, un $C_{1-3}$ alkyle, un $C_{1-3}$ alcoxy, un phényl, un phénoxy, un benzyloxy ;
à la condition qu'au moins un groupe $R_4$ ou $R_5$ représente un groupe -C(X)$R_6$; et/ou

X représente un atome d'oxygène ou un atome de soufre ; et/ou
$R_6$ représente un groupe $C_{1-6}$ alcoxy, un hydroxy ou un groupe -$NR_7R_8$ ; et/ou
$R_7$ et $R_8$ représentent, indépendamment l'un de l'autre :

un atome d'hydrogène, un groupe $C_{1-6}$ alkyle éventuellement substitué par un $C_{3-7}$ cycloalkyle, un $C_{3-7}$ cycloalkènyle, un phényle ou un pyridinyle; ou
un $C_{3-7}$ cycloalkyle, $C_{1-6}$ alcoxy ou un phényle ; ou
$R_7$ et $R_8$, avec l'atome d'azote qui les porte, forment un cycle aziridine, azétidine, pyrrolidine, pipéridine ou morpholine ;
les groupes $C_{3-7}$ cycloalkyle et phényle étant éventuellement substitués par un groupe $C_{1-3}$ alkyle, un hydroxy, un $C_{1-3}$ alcoxy ou un atome d'halogène.

Parmi les composés de formule générale (I), un troisième groupe de composés préférés sont ceux pour lesquels:

$R_1$ représente :

un $C_{1-5}$ alkyle, de préférence un méthyle, éthyle, 1-méthyléthyle, 2-méthylpropyle, *tert*-butyle, 1-éthylpropyle, éventuellement substitué par un ou deux substituants choisis parmi un hydroxy, un $C_{1-4}$ thioalkyle, de préférence un thiométhyle, un trifluorométhyle, un thiophène ou un phényle; ou
un $C_{4-7}$ cycloalkyle, de préférence un cyclopropyle ou un cyclohexyle ; un furanyle, un thiophène, un benzothiophène, un pyridinyle ou un phényle ; le phényle étant éventuellement substitué par un ou deux substituants choisis parmi un atome d'halogène, de préférence un fluor ou un chlore, un hydroxy, un benzyloxy ou un méthylènedioxy; et/ou

$R_2$ et $R'_2$ représentent indépendamment l'un de l'autre un atome d'hydrogène, un atome d'halogène, de préférence un fluor, un hydroxy, un $C_{1-3}$ alkyle, de préférence un méthyle ou un éthyle, un $C_{3-7}$ cycloalkyle, de préférence un cyclohexyle, un $C_{1-3}$ alcoxy, de préférence un méthoxy, un groupe O-C(O)-$C_{1-4}$ alkyle, de préférence -O-C(O)-$CH_3$, ou $R_2$ et $R'_2$ forment ensemble un groupe oxo ; et/ou
$R_3$ représente un $C_{1-4}$ alkyle, de préférence un méthyle, un éthyle, un propyle ou un butyle, éventuellement substitué par un $C_{1-3}$ alcoxy, de préférence un méthoxy ; et/ou
$R_4$ et $R_5$ représentent, indépendamment l'un de l'autre :

un atome d'hydrogène, un $C_{1-7}$ alkyle, de préférence un méthyle, éthyle, 1-méthyléthyle, n-propyle, butyle, 2-méthylpropyle, *ter*-butyle, 3-méthylbutyle, 1-éthylpropyle, 2,2-diméthylpropyle, hexyle, 5-méthylhexyle, éventuellement substitué par un phényle ou un $C_{3-7}$cycloalkyle, de préférence un cyclohexyle; ou
un $C_{3-7}$ cycloalkyle, de préférence un cyclopropyle ou un cyclohexyle ; un phényle, un naphtyle, ou un -C(X)$R_6$;
le phényle étant éventuellement substitué par un ou deux groupes choisis parmi un $C_{1-3}$ alkyle, de préférence un 1-méthyléthyle, un hydroxy, un $C_{1-3}$ alcoxy de préférence un méthoxy ou éthoxy ; un phénoxy, un $C_{1-3}$ alcoxy, de préférence un méthoxy ou un éthoxy, un hydroxy, un phénoxy ou un benzyloxy ;
à la condition qu'au moins un groupe $R_4$ ou $R_5$ représente un groupe -C(X)$R_6$; et/ou

X représente un atome d'oxygène ; et/ou
$R_6$ représente un groupe $C_{1-6}$ alcoxy, de préférence un méthoxy, éthoxy, *ter*-butyloxy, un hydroxy ou un groupe -$NR_7R_8$; et/ou
$R_7$ et $R_8$ représentent, indépendamment l'un de l'autre :

un atome d'hydrogène, un groupe $C_{1-3}$ alkyle, de préférence un méthyle, éthyle ou 1-méthyléthyle, éventuellement substitué par un $C_{3-7}$ cycloalkènyle, de préférence un cyclohexènyle, un phényle ou un pyridinyle ; ou
un $C_{1-3}$ alcoxy, de préférence un méthoxy, un $C_{3-6}$cycloalkyle, de préférence un cyclohexyle ; ou

$R_7$ et $R_8$, avec l'atome d'azote qui les porte, forment un cycle azétidine, pipéridine ou morpholine.

Les composés pour lesquels à la fois X, $R_1$, $R_2$, $R'_2$, $R_3$, $R_4$, $R_5$, $R_6$, $R_7$ et $R_8$ sont tels que définis ci-dessus dans les groupes de composés préférés, sont particulièrement préférés et plus spécifiquement parmi ceux-ci les composés pour lesquels :

$R_1$ représente un $C_{1-4}$ alkyle, de préférence un 1-méthyléthyle ou un *ter*-butyle, ou un phényle substitué par deux atomes de fluor ; et/ou
$R_2$ représente un hydroxy et $R'_2$ représente un atome d'hydrogène ; et/ou
$R_3$ représente un $C_{1-4}$ alkyle, de préférence un méthyle, éthyle ou propyte ; et/ou
X représente un atome d'oxygène.

Dans le cadre de la présente invention, on entend par :

- $C_{t-z}$ où t et z peuvent prendre les valeurs de 1 à 7, une chaîne carbonée pouvant avoir de t à z atomes de carbone, par exemple $C_{1-3}$ une chaîne carbonée qui peut avoir de 1 à 3 atomes de carbone, $C_{3-6}$ une chaîne carbonée qui peut avoir de 3 à 6 atomes de carbone, ......
- alkyle, un groupe aliphatique saturé, linéaire ou ramifié; par exemple, un groupe $C_{1-6}$ alkyle représente une chaîne carbonée de 1 à 6 atomes de carbone, linéaire ou ramifiée, plus particulièrement un méthyle, éthyle, propyle, 1-méthyléthyle, butyle, isobutyle, secbutyle, *ter*-butyle, .....de préférence un méthyle, éthyle, propyle ou 1-méthyléthyle ;
- cycloalkyle, un groupe alkyle cyclique, par exemple, un groupe $C_{3-7}$ cycloalkyle représente une chaîne carbonée cyclique de 3 à 7 atomes de carbone, plus particulièrement un cyclopropyle, cyclobutyle, cyclopentyle, cyclohexyle, cycloheptyle, de préférence un cyclopentyle ou cyclohexyle ;
- cycloalkènyle, un groupe alkyle cyclique mono- ou poly-insaturé, par exemple, un groupe $C_{3-7}$ cycloalkènyle représente une chaîne carbonée cyclique mono- ou poly-insaturée de 3 à 7 atomes de carbone, plus particulièrement un cyclopropényle, cyclobutènyle, cyclopentènyle, cyclohexènyle, cycloheptènyle, de préférence un cyclopentènyle ou cyclohexènyle ;
- thioalkyle, un groupe S-alkyle à chaîne aliphatique saturée, linéaire ou ramifiée ;
- alcoxy, un groupe alkyloxy à chaîne aliphatique saturée, linéaire ou ramifiée ;
- atome d'halogène, un fluor, un chlore, un brome ou un iode ; et
- « $R_2$ et $R_{2'}$ forment ensemble un groupe oxo », le groupe tel que :

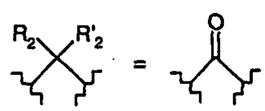

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention. Lorsque le carbone portant $R_2$ et $R'_2$ et/ou le carbone portant $R_3$ sont asymétriques, on préfère les composés de formule générale (I) pour lesquels le carbone portant $R_2$ et $R'_2$ est de configuration (S) et/ou le carbone portant $R_3$ est de configuration (S).

Les composés de formule (I) peuvent exister à l'état de bases ou de sels d'addition à des acides. De tels sels d'addition font partie de l'invention. Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

La présente invention a pour second objet des procédés de préparation des composés de formule (I).

Ainsi, ces composés peuvent être préparés par des procédés, illustrés dans les schémas qui suivent, dont les conditions opératoires sont classiques pour l'homme du métier.

On entend par groupe protecteur, un groupement permettant d'empêcher la réactivité d'une fonction ou position, lors d'une réaction chimique pouvant l'affecter, et qui restitue la molécule après clivage selon des méthodes connues de l'homme du métier. Des exemples de groupes protecteurs ainsi que les méthodes de protection et déprotection sont données, entre autres, dans Protective groups in Organic Synthesis, Greene et al., 2nd Ed. (John Wiley & Sons, Inc.,

New York).

**Schéma 1**

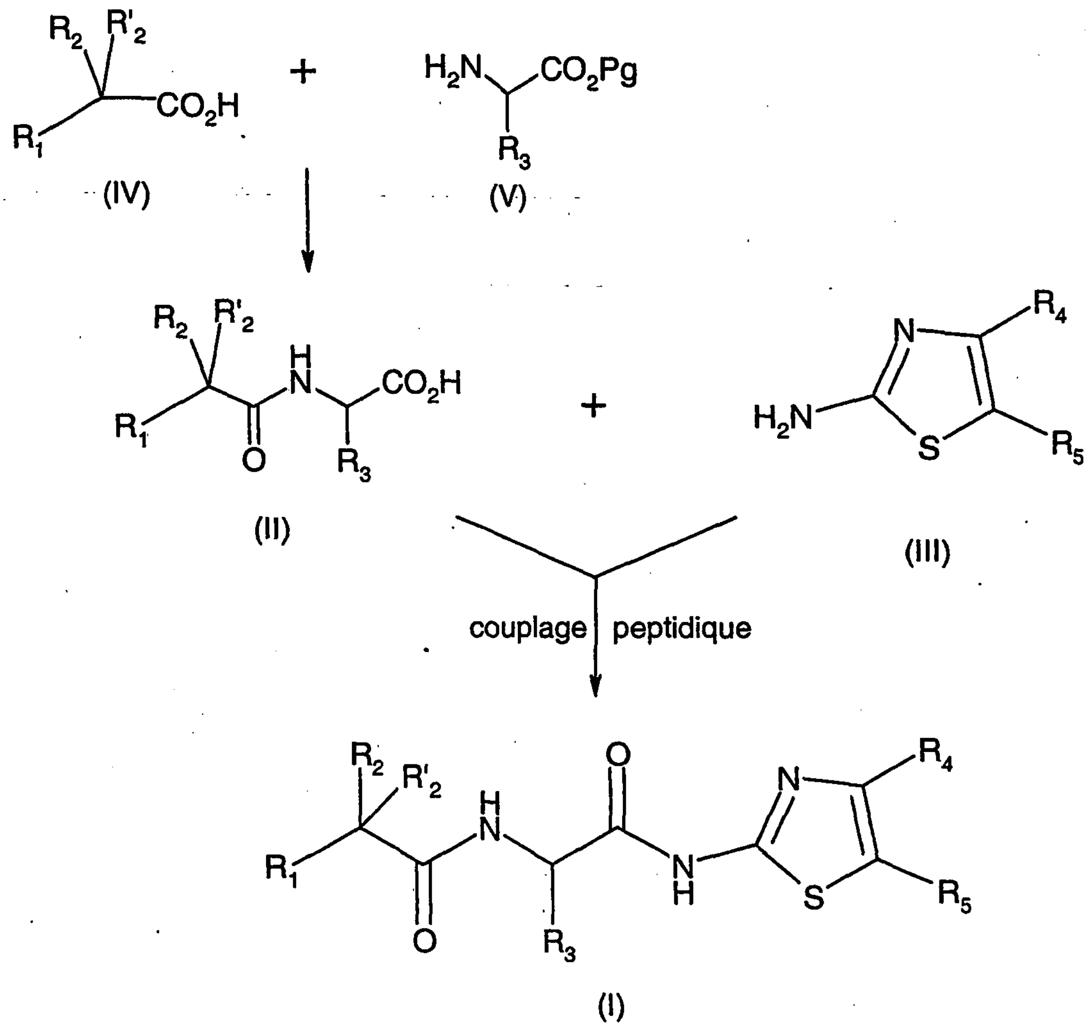


(IV) (V)

(II) (III)

(I)

Selon le schéma 1, le composé de formule (1) peut être obtenu par couplage peptidique du 2-aminothiazole de formule (III) avec l'acylaminoacide de formule (II) selon des conditions connues de l'homme du métier, par exemple en présence d'héxafluorophosphate de benzotriazol-1-yloxy-tris(pyrrolidino) phosphonium (PyBOP) ou d'héxafluorophosphate de benzotriazol-1-yloxy-tris(diméthylamino)phosphonium (BOP) et de *N*-éthylmorpholine ou *N*-méthylmdrpholine dans un solvant inerte tel que le diméthylformamide, l'acétonitrile ou le dichlorométhane à une température pouvant aller de 0°C à la température ambiante.
Le composé de formule (II) peut être obtenu par couplage peptidique du composé de formule (IV) avec l'acide protégé de formule (V), dans laquelle Pg représente un groupe protecteur, par exemple un benzyle, selon des méthodes connues de l'homme du métier telles que décrites ci-dessus.
Le composé ainsi obtenu est ensuite déprotégé. Dans le cas où la protection est un benzyle, le composé est préalablement hydrogéné en présence de palladium sur charbon dans de l'éthanol absolu à pression atmosphérique d'hydrogène, à température ambiante, pour donner le composé de formule (II).

Alternativement; le composé de formule (I) peut être préparé selon le schéma 2.

## Schéma 2

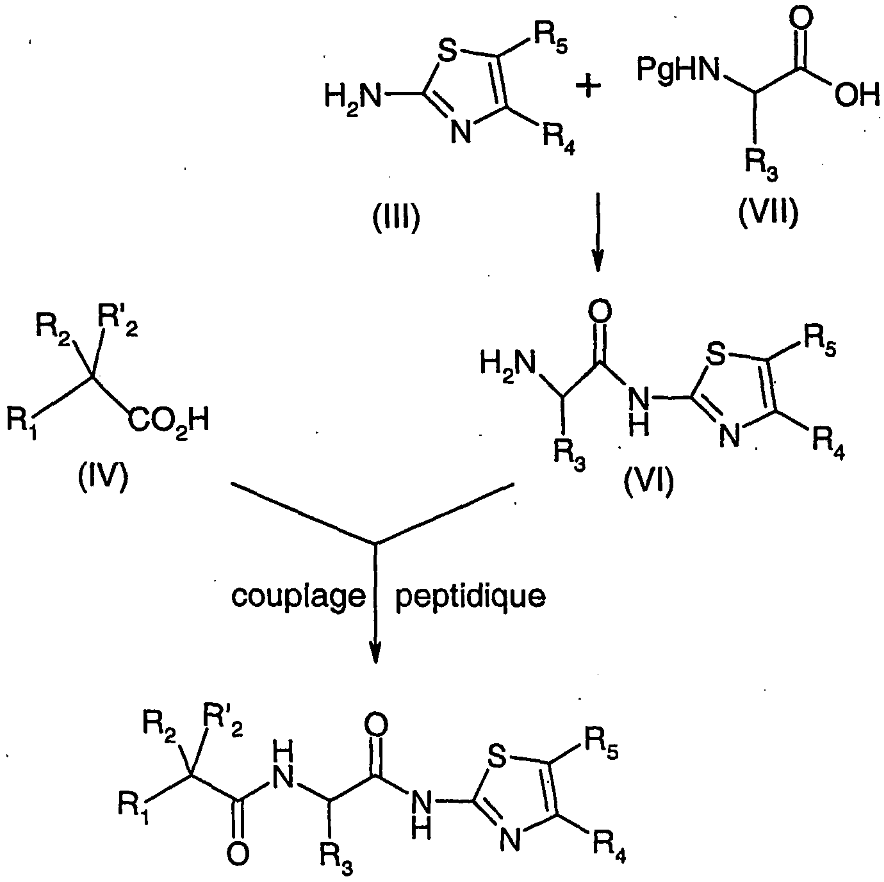


(III) (VII) (IV) (VI)

(I)

Selon ce schéma, le composé de formule (I) peut être obtenu par couplage peptidique du composé de formule (IV) avec l'amine de formule (VI), selon des méthodes connues de l'homme du métier, comme par exemple en présence de chlorhydrate d'hydroxybenzotriazole (HOBt) et de 1-éthyl-3-(3-diméthylamino-propyl)carbodiimide (EDAC, HCl).
Le composé de formule (VI) peut être obtenu par couplage peptidique du 2-aminothiazole de formule (III) avec l'amine protégée de formule (VII) selon des méthodes connues de l'homme du métier telles que décrites ci-dessus. L'amine peut être protégée par exemple au moyen d'un *N-tert*-butoxycarbonyl (Boc), selon des méthodes classiques connues de l'homme du métier puis déprotégée par hydrolyse acide, en présence d'acide chlorhydrique gazeux dissous dans un solvant anhydre ou d'acide trifluoroacétique.
Les composés de formule (1) dans laquelle $R_2$ et $R'_2$ forme un groupe oxo, peuvent être obtenu par oxydation d'un hydroxy du composé de formule (1) dans laquelle $R_2$ ou $R'_2$ représente un groupe hydroxy. La réaction peut être réalisée selon les conditions connues de l'homme du métier, par exemple avec le réactif de Dess Martin. Ces composés peuvent également être obtenus par couplage direct d'un cétoacide de formule (IV), dans laquelle $R_2$ et $R'_2$ forment ensemble un groupe oxo, avec une amine de formule (VI) selon les conditions connues de l'homme du métier. Les méthodes de préparation de tels cétoacides sont connues de l'homme du métier.

Le composé de formule (III) dans laquelle $R_4$=-C(O)-$R_6$, $R_6$ représentant un groupe $C_{1-6}$ alcoxy, peut être obtenu selon le schéma 3.

## Schéma 3

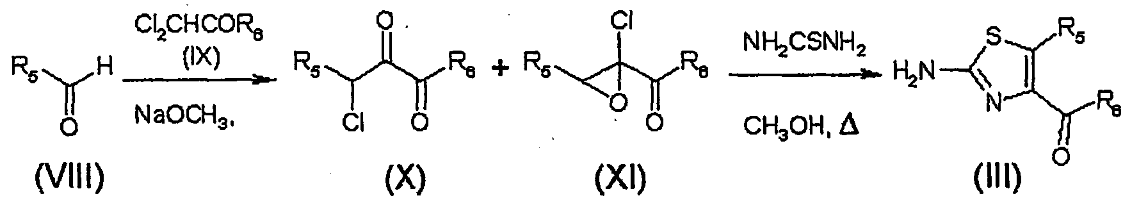


(VIII) (X) (XI) (III)

Selon ce schéma, le composé de formule (III) peut être obtenu par réaction d'un aldéhyde de formule (VIII) dans laquelle $R_5$ est tel que défini ci-dessus avec le méthyldichloroacétate de formule (IX) dans laquelle $R_6$ représente un $C_{1-6}$ alcoxy éventuellement substitué par un phényle, et par exemple le méthylate ou l'éthylate de sodium à 0°C selon une adaptation du procédé décrit par Takeda (Bull. Chem. Soc. JP, 1970, vol. 43, p. 2997). Le mélange de produits (X) et (XI) obtenu est traité par la thiourée en présence par exemple de méthanol ou d'éthanol à reflux pendant 4 ou 8 heures pour donner le composé de formule (III).

Le composé de formule (III) dans laquelle $R_4$=-C(O)-$R_6$, $R_6$ représentant un hydroxy, peut être obtenu par hydrolyse des composés ci-dessus pour lesquels $R_6$ représente un groupe $C_{1-6}$ alcoxy éventuellement substitué par un phényle, selon les conditions connues de l'homme du métier.

Le composé de formule (III) dans laquelle $R_5$=-C(O)-$R_6$, $R_6$ représentant un groupe $C_{1-6}$ alcoxy, peut être obtenu selon le schéma 4.

## Schéma 4

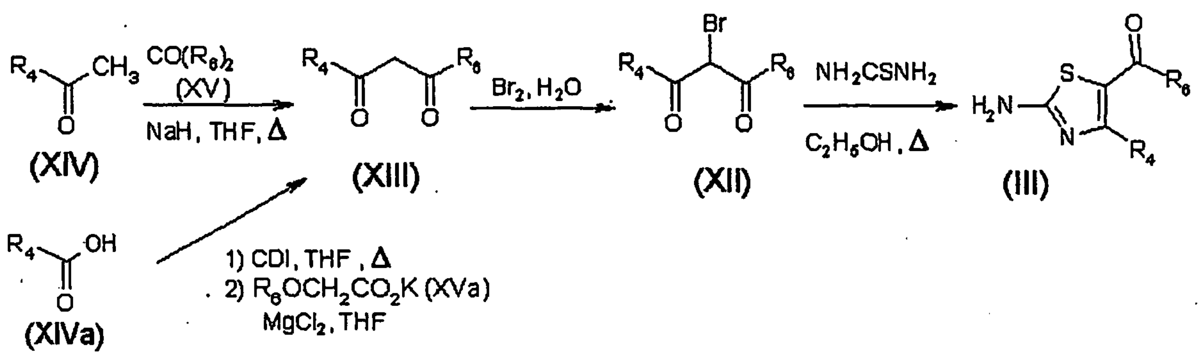


(XIV) (XIII) (XII) (III) (XIVa)

Selon le schéma 4, le composé de formule (III), peut être obtenu par bromation d'un β-céto-ester de formule (XIII), dans laquelle $R_6$ représente un $C_{1-6}$ alcoxy éventuellement substitué par un phényle, suivie d'une réaction avec la thiourée selon une adaptation du procédé décrit par A. Barton, Breukelman, Kaye (J.C.S Perkin I, 1982, p.159), pour obtenir le composé de formule (XII).

Le β-céto-ester de formule (XIII) peut être obtenu par réaction d'une cétone de formule (XIV) dans laquelle $R_4$ est tel que défini précédemment avec un dialkylcarbonate de formule (XV) dans laquelle $R_6$ représente un $C_{1-6}$ alcoxy éventuellement substitué par un phényle, selon une adaptation du procédé décrit par L. Crombie, R.C.F. Jones and C.J. Palmer (J.C.S Perkin Trans I, 1987, p.323). Le β-céto-ester de formule (XIII) peut également être obtenu par réaction d'un acide de formule (XIVa) activé par le carbonyldiimidazole (CDI) avec un malonate de formule (XVa) dans laquelle $R_6$ représente un $C_{1-6}$ alcoxy éventuellement substitué par un phényle, selon une adaptation du procédé décrit par exemple par D.W. Brooks, L.D.-L. Lu et S. Masamune (Angew. Chem. Int. Ed. Engl., 18, 1979, p.72).

Dans le cas où $R_4$ représente un atome d'hydrogène, la préparation du composé de formule (XIII) se fait selon une adaptation du procédé décrit par exemple par Tetrahedron Letters, 42, 2001, p.2101. Le composé de formule (III) dans laquelle $R_5$=-C(O)-$R_6$, $R_6$ représentant un hydroxy, peut être obtenu par hydrolyse des composés ci-dessus pour lesquels $R_6$ représente un groupe $C_{1-6}$ alcoxy, selon les conditions connues de l'homme du métier.

Le composé de formule (III) dans laquelle $R_4$ ou $R_5$ représente un groupe -C(O)-$NR_7R_8$ peut être obtenu selon le schéma 5.

## Schéma 5

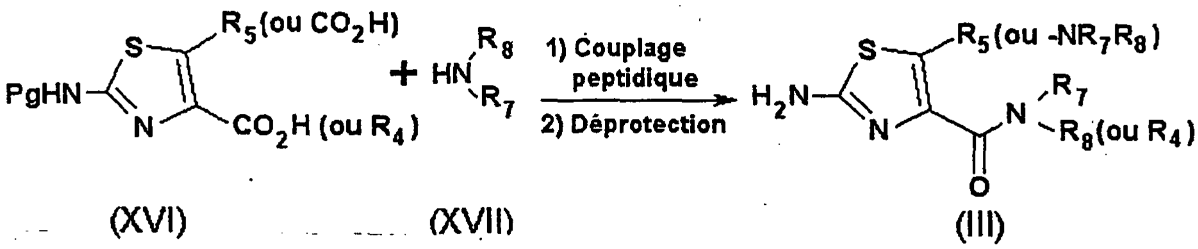


(XVI) (XVII) (III)

Selon ce schéma, le composé de formule (III) est obtenu par couplage peptidique du composé de formule (XVI), dans laquelle $R_5$ ou $R_4$ représente un groupe carboxylique et Pg un groupe protecteur tel qu'un Boc, avec un composé de formule (XVII) dans laquelle $R_7$ et $R_8$ sont tels que définis précédemment en présence par exemple d'HOBt et (EDAC, HCl). Le composé ainsi obtenu est ensuite déprotégé selon les conditions connues de l'homme du métier. Le composé de formule (XVI), dans laquelle Pg représente un Boc, peut être obtenu par protection d'un composé de formule (III) dans laquelle $R_4$ ou $R_5$ représente un groupe -C(O)$R_6$ et $R_6$ est un $C_{1-6}$ alcoxy éventuellement substitué par un phényle, par action de di-ter-butyldicarbonate dans du tétrahydrofurane anhydre en présence de diméthylaminopyridine à température ambiante, suivi d'une hydrolyse du carboxylate selon les conditions connues par l'homme de métier, par exemple avec de l'hydroxyde de lithium dans un mélange tétrahydrofurane/eau 7:3 (v/v) à une température de 60°C.

Les composés de départ, notamment les composés de formule (IV), (V), (VII), (VIII), (IX), (XIV), (XIVa), (XV), (XVa), et (XVII) sont disponibles dans le commerce ou décrits dans la littérature, ou peuvent être préparés par des méthodes qui y sont décrites ou qui sont connues de l'homme du métier. Les composés de formule (IV) où $R_2$ ou $R'_2$ représente un hydroxy peuvent être préparés par addition de cyanure de triméthylsilyle sur un aldéhyde selon une adaptation du procédé décrit par D.A. Evans et coll. (J. C. S., Chem. Comm. 1973, p.55) ou par action du nitrite de sodium sur un alpha aminoacide selon une adaptation du procédé décrit par I. Shinn et coll. (J. Org. Chem., 200, 65, p.7667).

Lorsqu'une fonction d'un composé est réactive, par exemple lorsque $R_1$ comporte un hydroxy, elle peut nécessiter une protection préalable avant réaction. L'homme du métier pourra déterminer aisément la nécessité d'une protection préalable.

Les significations de $R_1$, $R_2$, $R'_2$, $R_3$, $R_4$, $R_5$, X, $R_6$, $R_7$ et $R_8$ dans les composés de formule (II) à (XVII) sont telles que définies pour les composés de formule (I).

Les composés de formule (II), (III), et (VI) sont nouveaux et font également partie de l'invention. Ils sont utiles en tant qu'intermédiaires de synthèse pour la préparation des composés de formule générale (I).

Les exemples suivants décrivent la préparation de certains composés conformes à l'invention. Ces exemples ne sont pas limitatifs et ne font qu'illustrer l'invention.
Les numéros des composés exemplifiés renvoient à ceux donnés dans le tableau ci-après. Les micro-analyses élémentaires et les spectres RMN, IR ou de masse confirment les structures des composés obtenus.

**Exemple 1 : 2-amino-5-(1-méthyléthyl)thiazole-4-carboxylate de méthyle**

A 14,4 g d'isobutyraldéhyde en solution dans. 400 ml de diéthylester, on additionne, à 0°C, 24,6 g de méthyldichloroacétate, puis goutte à goutte 400 ml d'une solution de méthylate de sodium (0,5M) dans du méthanol. Après 1 h à 0°C, on additionne 100 ml solution aqueuse saturée en chlorure de sodium et on extrait à l'éther. On sèche la phase organique sur sulfate de sodium anhydre. On évapore uniquement l'éther en conservant le méthanol, on additionne 8 g de thiourée et on chauffe à reflux pendant 6 h. On évapore le milieu réactionnel à sec, on le reprend à l'acétate d'éthyle et on le lave avec une solution aqueuse à 10 % d'hydroxyde d'ammonium, puis avec une solution aqueuse saturée en chlorure de sodium. On sèche la phase organique sur sulfate de sodium anhydre, puis on la concentre. On reprend le résidu par 100 ml d'éther et on le filtre sur fritté. On obtient 18,6 g d'un solide blanc.
RMN 300MHz (CDCl3) δppm : 1,25 (d,6H) ; 3,35 (s,3H) ; 4,10 (m,1H) ; 5,50 (s,2H).

**Exemple 2 : 2-amino-4-(2,2-diméthylpropyl)thiazole-5-carboxylate d'éthyle**

A 4 g de 4,4-diméthylpentanone en solution dans 100 ml de tétrahydrofurane anhydre, on additionne 3 g d'hydrure de sodium à 60 %, suivi de 8,2 g de diéthylcarbonate goutte à goutte. On chauffe le milieu réactionnel à reflux

pendant 5 h. On laisse refroidir et on hydrolyse avec 50 ml d'eau distillée. On évapore le tétrahydrofurane. On extrait avec de l'acétate d'éthyle. On sèche la phase organique sur sulfate de sodium puis on l'évapore. On obtient 4,9 g d'une huile colorée.
RMN 300MHz δ en ppm ($CDCl_3$) : 1,05 (s,9H) ; 1,28 (t,3H) ; 2,42 (s,2H) ; 3,40 (s,2H) ; 4,18 (q,4H).
A 4,9 g de β céto-ester obtenu ci-dessus en suspension dans 75 ml d'eau distillée à 0˚C, on additionne goutte à goutte 1,4 ml de brome. On agite le milieu réactionnel 1 h à 0˚C puis on l'extrait à l'acétate d'éthyle. On lave la phase organique avec une solution saturée en chlorure de sodium puis on la sèche sur sulfate de sodium.
Après évaporation, on obtient 4,1 g d'une huile orangée.
RMN 300MHz ($CDCl_3$) δppm : 1,00 (s,9H) ; 1,30 (t,3H) ; 2,60 (ms,2H) ; 4,25 (q,2H) ; 4,75 (s, 1H).
A 4,10 g de bromo-β-céto-ester en solution dans 60 ml d'éthanol, on additionne 1,1 g de thiourée. On chauffe le milieu réactionnel à reflux pendant 6 h, puis on l'évapore à sec.
On reprend le résidu à l'acétate d'éthyle et on le lave avec une solution aqueuse à 10 % d'hydroxyde d'ammonium, puis avec une solution aqueuse saturée en chlorure de sodium et on le sèche sur sulfate de sodium.
Après évaporation, on recristallise le résidu dans un mélange acétate d'éthyle/pentane. On obtient 2,1 g d'un solide blanc.
RMN 300MHz ($CDCl_3$) δppm : 0,97 . (s,9H) ; 1,21 (t,3H) ; 2,92 (s,2H) ; 4,25 (q,2H) ; 5,65 (s,2H).

**Exemple 3 : 2-[2-[2-(3,5-difluorophényl)acétylamino]propanoylamino]-5-propylthiazole-4-carboxylate de méthyle (composé n˚ 2).**

**Exemple 3.1 : N-3,5-difluorophénylacétyl-(S)-alanine**

A 1,72 g d'acide 3,5-difluorophénylacétique en solution dans 50 ml de diméthylformamide à 0˚C, on additionne 1,01 g de N-méthylmorpholine, 5,72 g de PyBOP, 2,15 g de chlorhydrate de (S)-alaninebenzylester et 1,01 g de N-méthylmorpholine. On laisse revenir à température ambiante et on agite pendant 18 h. On évapore le milieu réactionnel, on le reprend à l'acétate d'éthyle et on le lave 2 fois avec une solution aqueuse saturée en bicarbonate de sodium, une solution de potassium hydrogénosulfate (1 M) puis avec une solution aqueuse saturée en chlorure de sodium. On sèche la phase organique sur sulfate de sodium anhydre.
On chromatographie le résidu sur une colonne de gel de silice, en éluant avec un mélange éther de pétrole/acétate d'éthyle 7:3 (v/v) pour obtenir 1,9 g d'un solide blanc.
RMN 300 MHz ($CDCl_3$) δppm : 1,40 (d,3H) ; 3,54 (s,2H) ; 4,62 (m,1H) ; 5,18 (m,2H) ; 6,10 (d,1H) ; 6,73 (t,2H) ; 6,80 (d, 1H) ; 7,32 (m,5H).
A 1,9 g de N-3,5-difluorophénylacétyl-(S)-alaninebenzylester en solution dans 80 ml d'éthanol absolu, on additionne 300 mg de palladium sur charbon à 10 %.
On hydrogène le milieu réactionnel à pression atmosphérique et à température ambiante durant 8 h. On filtre le milieu réactionnel sur papier lavé à l'éthanol absolu, puis on l'évapore. On obtient 1,37 g d'un solide blanc.
RMN 300 MHz ($CDCl_3$) δppm : 1,36 (d,3H) ; 3,48 (s,2H) ; 3,70 (s,1H) ; 4,40 (m,1 H) ; 6,65 (t,2H) ; 6,70 (d,1 H) ; 6,95 (d,1 H).

**Exemple 3.2 : 2-[2-[2-(3,5-difluorophényl)acétylamino]propanoyl-amino]-5-propylthiazole-4-carboxylate de méthyle**

A 0,24 g de 2-amino-5-propyl-thiazole-4-carboxylate de méthyle, préparé selon le procédé décrit dans l'exemple 1, en solution dans 25 ml de diméthylformamide à 0˚C, on additionne 0,12 g de N-méthylmorpholine, 0,69 g de PyBOP puis 0,275 g du composé obtenu à l'étape 3.1 de l'exemple 3. On laisse revenir la réaction à température ambiante et on l'agite pendant 18 h.
On évapore le solvant, on reprend le résidu à l'acétate d'éthyle, et on le lave 2 fois avec une solution aqueuse saturée en bicarbonate de sodium, 1 fois à l'eau, et 1 fois avec une solution aqueuse 1 N d'hydrogénosulfate de potassium puis avec une solution aqueuse saturée en chlorure de sodium.
On sèche la phase organique sur sulfate de sodium anhydre et on la concentre. On chromatographie le résidu sur une colonne de silice en éluant avec un mélange éther de pétrole et acétate d'éthyle 1:1 (v/v) pour obtenir 0,22 g d'une poudre blanche.
LC/MS : $MH^+$ = 426.
RMN 500 MHz ($CDCl_3$) δppm : 0,91 (q, 3H) ; 1,30 (d,3H) ; 1,61 (m, 2H) ; 3,06 (t,2H) ; 3,53 (s,2H) ; 3,77 (s,3H) ; 4,38 (m,1H) ; 6,98 (m,2H) ; 7,08 (m,1H) ; 8,57 (d, 1 H) ; 12,49 (s, 1 H).

**Exemple 4 : 2-[2-[2-(3,5-difluorophényl)acétylamino]propanoylamino]-4-propylthiazole-5-carboxylate de mé-**

**thyle (composé n° 144).**

A-0,3g de 2-amino-4-propyl-thiazole-5-carboxylate de méthyle, préparé selon le procédé décrit dans l'exemple 2, en solution dans 30 ml de diméthylformamide à 0°C, on additionne 0,101 g de N-méthylmorpholine, 0,86 g de PyBOP et 0,34 g de N-3,5-difluorophenylacetyl-(S)-alanine, obtenu à l'étape 3.1 de l'exemple 3. On laisse remonter le milieu réactionnel à température ambiante puis on l'agite pendant 18h.
Après évaporation du solvant, on reprend le résidu par de l'acétate d'éthyle et on le lave 2 fois avec une solution aqueuse saturée en bicarbonate de sodium, 2 fois à l'eau, 1 fois avec une solution aqueuse 1M hydrogénosulfate de potassium, puis avec une solution aqueuse saturée en chlorure de sodium.
On sèche la phase organique sur sulfate de sodium anhydre puis on la concentre. On chromatographie le résidu sur une colonne de silice, en éluant avec un mélange d'acétate d'éthyle et d'éther de pétrole 3:7 (v/v) pour obtenir 0,45 g d'une poudre blanche.
LC/MS : $MH^+$ = 426.
RMN 500 MHz (DMSO) δppm : 0,88 (t, 3H) ; 1,32 (d, 3H) ; 1,66 (m, 2H) ; 2,95 (m, 2H) ; 3,54 (s, 2H) ; 3,76 (s, 3H) ; 4,47 (m, 2H) ; 6,97 et 7,10 (2m, 3H) ; 8,63. (d, 1 H) ; 12,66 (s, 1 H).

**Exemple 5 : 2-[2-[2-(3,5-difluorophényl)acétylamino]-(2S)-pentanoylamino]-5-(1-méthyléthyl)thiazole-4-carboxylate de méthyle (composé n° 18).**

**Exemple 5.1 : 2-(2-amino-(2S)-pentanoylamino)-5-(1-méthyléthyl)-thiazole-4-carboxylate de méthyle**

A 2,3 g de 2-amino-5-(1-méthyléthyl)thiazole-4-carboxylate de méthyle, obtenu à l'exemple 1, en solution dans 100 ml de diméthylformamide à 0°C, on additionne 1,22 g de N-méthylmorpholine, 6,34 g de PyBOP puis 2,65 g de (S)-Bocnorvaline. On laisse revenir le milieu réactionnel à température ambiante puis on l'agite pendant 16 h.
Après évaporation, on reprend le résidu par de l'acétate d'éthyle et on le lave 2 fois avec une solution aqueuse saturée en bicarbonate de sodium, 2 fois à l'eau, 1 fois avec une solution aqueuse 1H d'hydrogénosulfate de potassium, puis avec une solution aqueuse saturée en chlorure de sodium.
On sèche la phase organique sur sulfate de sodium anhydre, puis on la concentre.
On chromatographie le résidu sur une colonne de gel de silice, en éluant avec un mélange d'acétate d'éthyle et d'éther de pétrole 3:7 (v/v). On obtient 3,5 g d'un solide blanc.
RMN 300 MHz ($CDCl_3$) δppm : 0,97 (t,3H) ; 1,37 (d,6H) ; 1,45 (s,9H) ; 1,67 (m,2H) ; 1,90 (m,2H) ; 3,90 (s,3H) ; 4,10 (m, 1H); 4,38 (massif, 1H); 4,90 (massif, 1 H).
On agite 3,3 g de produit obtenu ci-dessus en solution dans 60 ml d'acide trifluoroacétique à température ambiante pendant 30 min, puis on l'évapore.
On reprend le résidu à l'acétate d'éthyle, et on le lave 2 fois avec une solution aqueuse saturée en carbonate de sodium, puis avec une solution aqueuse saturée en chlorure de sodium. On sèche la phase organique sur sulfate de sodium anhydre, puis on l'évapore pour donner 2 g d'un solide blanc.
RMN 300 MHz ($CDCl_3$) δppm : 0,97 (t,3H) ; 1,35 (d,6H) ; 1,40 à 1,60 (m,2H) ; 1,80 (m,2H) ; 3,60 (m,1H) ; 3,97 (s,3H) ; 4,12 (m,1H).

**Exemple 5.2 : 2-[2-[2-(3,5-difluorophényl)acétylamino]-(2S)-pentanoyl-amino]-5-(1-méthyléthyl)thiazole-4-carboxylate de méthyle**

A 0,7 g de 2-(2-amino-(2S)-pentanoylamino)-5-(1-méthyléthyl)-thiazole-4-carboxylate de méthyle, obtenu à l'étape 5.1 de l'exemple 5, en solution dans 30 ml de diméthylformamide à 0°C, on additionne 0,255 g de N-méthylmorpholine, 1,30 g de PyBOP puis 0,43 g d'acide 3,5-difluorophénylacétique.
On laisse revenir la réaction à température ambiante et on l'agite pendant 18 h. On évapore le milieu réactionnel. On reprend le résidu à l'acétate d'éthyle et on le lave 2 fois avec une solution aqueuse saturée en bicarbonate de sodium, 2 fois à l'eau, 1 fois avec une solution aqueuse 1M d'hydrogénosulfate de potassium, puis avec une solution aqueuse saturée en chlorure de sodium. On sèche la phase organique sur sulfate de sodium anhydre puis on la concentre. On chromatographie le résidu sur une colonne de silice en éluant avec un mélange éther de pétrole/acétate d'éthyle 1:1 (v/v) pour obtenir 0,7 g d'un solide blanc.
LC/MS : $MH^+$ = 454.
RMN 500MHz (DMSO) δppm : 0,85 (t, 3H) ; 1,26 (d, 6H) ; 1,28-1,65 (m, 4H) ; 3,53 (m, 2H) ; 3,78 (s, 3H) ; 3,97 (m, 1H) ; 4,36 (m, 1H) ; 6,96 (d, 2H) ; 7,08 (m, 1H) ; 8,49 (d, 1 H) ; 12,52 (s, 1H).

**Exemple 6 : 2-[2-[2-(3,5-difluorophényl)acétylamino]-(2S)-pentanoylamino]--4-(1-méthyléthyl)-thiazole-5-car-**

**boxylate de méthyle (composé n° 146).**

On procède de la même manière que dans l'exemple 5 en remplaçant le 2-amino-5-(1-méthyléthyl)thiazole-4-carboxylate de méthyle par le 2-amino-4-(1-méthyléthyl)thiazole-5-carboxylate de méthyle, préparé selon le procédé décrit dans l'exemple 2.
LC/MS : $MH^+$ = 454.
RMN 500MHz (DMSO) δppm : 0,87 (t,3H) ; 1,27 (d,6H) ; 1,35 (m,2H) ; 1,64 (m,2H); 3,54 (s,2H) ; 3,77 (s,3H) ; 3,90 (m, 1H) ; 4,45 (m, 1H) ; 6,96-7,09 (m,3H); 8,53 (d,1 H) ; 12,69 (s,1 H).

**Exemple 7 : 2-[2-[2-(3,5-difluorophényl)-2-hydroxy-acétylamino]-(2S)-pentanoylamino]-5-(1-méthyléthyl)-thiazole-4-carboxylate de méthyle (composés n° 19 et 20).**

A 1 g de 2-(2-amino-(2S)-pentanoylamino)-5-(1-méthyléthyl)thiazole-4-carboxylate de méthyle, obtenu à l'étape 5.1 de l'exemple 5, en solution dans 80 ml de diméthylformamide à 0°C, on additionne 0,366 g de N-méthylmorpholine, 1,87 g de PyBOP puis 0,677 g d'acide 3,5-difluoromandélique. On laisse revenir le milieu réactionnel à température ambiante, on l'agite 16 h, puis on le concentre. On reprend le résidu par de l'acétate d'éthyle, on le lave 2 fois avec une solution aqueuse saturée en bicarbonate de sodium, 2 fois à l'eau, 1 fois avec une solution aqueuse 1M d'hydrogénosulfate de potassium puis avec une solution aqueuse saturée en chlorure de sodium. On sèche la phase organique sur sulfate de sodium anhydre puis on la concentre. On chromatographie le résidu sur une colonne de gel de silice, en éluant avec un mélange d'éther de pétrole et d'acétate d'éthyle allant de 7:3 (1 :1) (v/v). Les deux isomères (S,S) et (R, S) peuvent être ainsi séparés. On obtient 0,45 g d'une poudre blanche (isomère (S,S)) et 0,50 g (mélange des 2 diastéréoisomères).
On rechromatographie le mélange des 2 diastéréoisomères (0,50 g) sur une colonne de gel silice, en éluant avec un mélange éther de pétrole et acétate d'éthyle 7:3 (v/v) à (1:1) pour obtenir 0,25 g d'une poudre blanche (isomère R,S).
Isomère (S,S) :
LC/MS : $MH^+$ = 470.
RMN 500MHz (DMSO) δppm : 0,80 (t,3H) ; 1,18 (m,2H); 1,22 (d,6H); 1,68
(m,2H) ; 3,78 (s,3H) ; 3,97 (m,1H) ; 4,43 (m,1H) ; 5,05 (d,1H) ; 6,55 (d,1H) ; 7,13 (m,3H) ; 8,24 (d, 1H) ; 12,46 (s, 1H).

$\alpha_D^{20} = -53°$ (c = 1/MeOH)

isomère (R,S) :
LC/MS : $MH^+$ = 470.
RMN 500MHz (DMSO) δppm : 0,82 (t,3H) ; 1,26 (d,6H) ; 1,26 (m,2H) ; 1,68 (m,2H) ; 3,78 (s,3H) ; 3,97 (m,1H) ; 4,40 (m,1 H) ; 5,07 (d,1H) ; 6,42 (d,1H) ; 7,12 (m,3H) ; 8,27 ,(d,1H) ; 12,51 (s,1H).

$\alpha_D^{20} = -83°$ (c = 1/MeOH)

**Exemple 8 : 2-[2-[2-(3,5-difluorophényl)-2-hydroxy-acétylamino]-(2S)-pentanoylamino]-4-(1-méthyléthyl)-thiazole-5-carboxylate de méthyle (composés n° 150 et 151).**

On procède de la même manière que dans l'exemple 7, en remplaçant le 2-(2-(S)-pentanoylamino)-5-(1-méthyléthyl)-thiazole-4-carboxylate de méthyle par le 2-(2-(S)-pentanoylamino)-4-(1-méthyléthyl)-thiazole-5-carboxylate de méthyle obtenu selon le procédé décrit à l'étape 5.1 de l'exemple 5.
Isomère (S,S) :
LC/MS : $MH^+$ = 471.
RMN 500MHz (DMSO) δppm : 0,81 (t,3H) ; 1,18 (d,6H) ; 1,22 (m,2H) ; 1,71 (m,2H) ; 3,77 (s,3H) ; 3,90 (m,1H) ; 4,50 (m,1H) ; 5,06 (s,1H) ; 6,54 (s,1H) ; 7,12 (m,3H) ; 8,30 (d,1H) ; 12,66 (s,1 H).

$\alpha_D^{20} = -73°$ (c =1/MeOH)

Isomère (R,S) :
LC/MS : $MH^+$ = 471 (pureté 95 %)
RMN 500MHz (DMSO) δppm : 0,83 (t,3H); 1,19 (d,6H); 1,22 (m,2H); 1,70 (m,2H) ; 3,77 (s,3H) ; 3,91 (m,1H) ; 4,48 (m, 1H) ; 5,07 (s,1H) ; 6,42 (s,1H) ; 7,12 (m,3H) ; 8,34 (d,1 H) ; 12,67 (s,1H).

$\alpha_D^{20} = -104°$ (c = 1/MeOH)

**Exemple 9 : 2-[2-[2-(3,5-difluorophényl)acétylamino]-(2S)-pentanoylamino]-5-(1-méthyléthyl)-thiazole-4-N,N-diméthylcarboxamide (composé n° 26).**

**Exemple 9.1 : acide 2-*ter*-butoxycarbonylamino-5-(1-méthyléthyl)-thiazole-4-carboxylique.**

A 4 ,4 g de 2-amino-5-(1-méthyléthyl)thiazole-4-carboxylate de méthyle, obtenu à l'exemple 1, en solution dans 150 ml de tétrahydrofurane, on additionne 5,27 g de di-*ter*-butyldicarbonate et 0,13 g de diméthylaminopyridine. On agite le mélange à température ambiante pendant 16 h. On évapore le milieu réactionnel. On reprend le résidu à l'acétate d'éthyle, et on le lave 2 fois avec une solution aqueuse 0,5N d'acide chlorhydrique, 1 fois à l'eau, puis avec une solution aqueuse saturée en chlorure de sodium. On sèche la phase organique sur sulfate de sodium anhydre et on la concentre. On obtient 6,1 g du dérivé aminothiazole protégé sous forme d'un solide qu'on utilise tel quel sans purification.
RMN 300 MHz ($CDCl_3$) δppm : 1,25 (d,6H) ; 1,50 (s,9H); 3,85 (s,3H); 3,97 (m,1 H).
LC/MS : $MH^+$ = 301 (M - Boc)$^+$ = 201
A 6,10 g de produit obtenu ci-dessus en solution dans 150 ml de tétrahydrofurane, on additionne à température ambiante une solution de 1,68 g d'hydroxyde de lithium dans 80 ml d'eau distillée. On chauffe à reflux le mélange réactionnel du tétrahydrofurane 16 h, puis on le concentre. On reprend le résidu par de l'eau et on le lave 2 fois avec de l'acétate d'éthyle. On acidifie la phase aqueuse avec une solution 1N d'acide chlorhydrique jusqu'à environ pH ~ 4, on la sature en chlorure de sodium et on l'extrait 2 fois avec de l'acétate d'éthyle.
On sèche la phase organique sur sulfate de sodium anhydre, puis on la concentre. On obtient 5,10 g d'un solide blanc.
LC/MS : $MH^+$ = 287.
RMN 300 MHz ($CDCl_3$) δppm : 1,19 (d,6H) ; 1,45 (s,9H) ; 3,80 (s,3H) ; 3,90 (massif s,1 H) ; 4,15 (m,1H).

**Exemple 9.2 : 2-*ter*-butoxycarbonylamino-5-(1-méthyléthyl)thiazole-4-N,N-diméthylcarboxamide**

A 2 g d'acide 2-*ter*-butoxycarbonylamino-5-(1-méthyléthyl)thiazole-4-carboxylique, obtenu à l'étape 9.1, en solution dans 80 ml de diméthylformamide, on additionne 1,07 g d'hydrate d'hydroxybenzotriazole, 1,33 g de (EDAC, HCl) puis 0,57 g de chlorhydrate de N,N diméthylamine et 0,77 g de N-méthylmorpholine. On agite le milieu réactionnel à température ambiante 16 h, puis on le concentre. On reprend le résidu à l'acétate d'éthyle, et on le lave 2 fois avec une solution aqueuse saturée en bicarbonate de sodium, 2 fois avec une solution aqueuse 1M d'hydrogénosulfate de potassium, 1 fois à l'eau, puis avec une solution aqueuse saturée en chlorure de sodium. On sèche la phase organique sur sulfate anhydre et on la concentre. On obtient 1,45 g d'un solide blanc.
LC/MS : $MH^+$ = 314
RMN 300 MHz ($CDCl_3$) δppm : 1,30 (d,6H) ; 1,53 (s,9H) ; 2,98 (s,3H) ; 3,09 (s,3H) ; 3,40 (m,1 H) ; 8,00 (s,1 H).

**Exemple 9.3 : 2-amino-5-(1-méthyléthyl)-thiazole-4-N,N-diméthyl-carboxamide**

A 1,4 g de 2-*ter*-butoxycarbonylamino-5-(1-méthyléthyl)thiazole-4-N,N-dimethyl-carboxamide, obtenu à l'étape 9.2, on additionne 30 ml d'acide trifluoroacétique. On agite le mélange à température ambiante 30 min, puis on le concentre. On reprend le résidu à l'acétate d'éthyle, et on le lave 2 fois avec une solution aqueuse saturée en carbonate de sodium, puis avec une solution aqueuse saturée en chlorure de sodium. On sèche la phase organique sur sulfate de sodium anhydre, puis on la concentre. On obtient 0,90 g d'un solide blanc.
LC/MS : $MH^+$ = 214.
RMN 300 MHz ($CDCl_3$) δppm : 1,22 (d,6H) ; 3,00 (s,3H) ; 3,00 (s,3H); 3,30 (m, 1H) ; 4,90 (s, 1H).

**Exemple 9.4 : 2-((2S)-pentanoylamino)-5-(1-méthyléthyl)-thiazole-4-N,N-diméthylcarboxamide**

On réalise le couplage de 0,9 g de 2-amino-5-(1-méthyléthyl)-thiazole-4-N,N-dimethylcarboxamide, obtenu à l'étape 9.3, avec 1 g de (S)-BocNorvaline selon le procédé décrit à l'étape 5.1 de l'exemple 5. On obtient après chromatographie sur silice éluée avec un mélange 1:1 (v/v) d'acétate d'éthyle et d'éther de pétrole 1,1 g d'une huile visqueuse qui cristallise à température ambiante.
LC/MS : $MH^+$ = 413.
RMN 300 MHz ($CDCl_3$) δppm : 0,97 (t,3H); 1,30 (d,6H); 1,40 (s,9H); 1,65 (m,2H) ; 1,90 (m,2H) ; 2,95 (s,3H) ; 3,08 (s, 3H) ; 3,48 (m,1 H) ; 4,30 (m,1 H) ; 5,25 (massif 1H).
On déprotège 1,1 g du composé obtenu ci-dessus dans 20 ml d'acide trifluoroacétique selon le procédé décrit à l'étape 9.3. On obtient 0,77 g d'un solide blanc.
LC/MS : $MH^+$ = 313.
RMN 300 MHz ($CDCl_3$) δppm : 0,98 (t,3H) ; 1,60 (d,6H) ; 1,30-1,95 (massif 4H) ; 2,98 (s,3H) ; 3,08 (s,3H) ; 3,40 (m,1

H) ; 3,58 (m,1 H).

**Exemple 9.5 : 2-[2-[2-(3,5-difluorophényl)acétylamino]-(2S)-pentanoyl-amino]-5-(1-méthyléthyl)-thiazole-4-N,N-diméthylcarboxamide**

A 0,23 g de 2-((2S)-pentanoylamino)-5-(1-méthyléthyl)-thiazole-4-N,N-diméthyl-carboxamide, obtenu à l'étape 9.4, dans 15 ml de diméthylformamide à 0°C, on additionne 0,088 g de N-méthyle morpholine puis 0,416 g de PyBOP et 0,138 g d'acide 3,5-difluorophénylacétique. On laisse le milieu réactionnel revenir à température ambiante pendant 16 h, puis on le concentre. On reprend le résidu à l'acétate d'éthyle, et on le lave 2 fois avec une solution aqueuse 0,5N d'acide chlorhydrique, 1 fois à l'eau, puis avec une solution aqueuse saturée en chlorure de sodium. On sèche la phase organique sur sulfate de sodium anhydre et on la concentre. Après chromatographie sur colonne de silice éluée avec un mélange d'acétate d'éthyle et d'éther de pétrole 1:1 (v/v) ; on obtient 0,26 g d'un solide blanc.

LC/MS : $MH^+$ = 467.

RMN 500MHz (DMSO) δppm: 0,87 (t,3H) ; 1,24 (d,6H) ; 1,29-1,67 (m,4H) ; 2,86 (s,3H) ; 2,95 (s,3H) ; 3,22 (m,1H) ; 3,54 (m,2H) ; 4,41 (m,1H) ; 6,98 (d,2H) ; 7,08 (m, 1H) ; 8,49 (d,1H) ; 12,21 (s,1 H).

$\alpha_D^{20} = -74°$ (c=1.0, MeOH).

**Exemple 10 : 2-[2-[2-(3,5-difluorophényl)-2-hydroxy-(2S)-acétylamino]-(2S)-pentanoylamino]-5-(1-méthyléthyl)-thiazole-4-N,N-diméthylcarboxamide (composé n° 88) et 2-[2-[2-(3,5-difluorophényl)-2-hydroxy-(2R)-acétylamino]-(2S)-pentanoylamino]-5-(1-méthyléthyl)-thiazole-4-N,N-diméthylcarboxamide (composé n° 89).**

Selon le procédé décrit précédemment à l'étape 9.5 de l'exemple 9, le couplage de 0,77 g de 2-((2S)-pentanoylamino)-5-(1-méthyléthyl)-thiazole-4-N,N-diméthyl-carboxamide, obtenu à l'étape 9.4 de l'exemple 9, avec 0,51 g d'acide 3,5-difluoromandélique en présence de 0,28 g N-méthylmorpholine et 1,4 g de PyBOP à 0°C, donne après chromatographie sur une colonne de silice éluée avec un mélange 75:25 (v/v) d'acétate d'éthyle et d'éther de pétrole 0,80 g d'un solide blanc.

LC/MS : $MH^+$ = 483 (2 pics : 2 diastéréoisomères (R,S) et (S,S)).

RMN 500MHz (DMSO) δppm : 0,72 (t,3H); 1,06 (m,1H) ; 1,16(m,1H) ; 1,27 (d,6H) ; 1,58 (m,2H) ; 2,10 (s,3H) ; 3,78 (s, 3H) ; 3,96 (m,1H) ; 4,37 (m,1 H) ; 5,97 (s,1H) ; 7,34 et 7,47 (2m,5H) ; 8,62 (s,1H) ; 12,49 (s,1 H).

$\alpha_D^{20} = -67°$ (c=1.0, MeOH).

On peut séparer les deux diastéréoisomères par HPLC préparative sur colonne $C_{18}$ avec un gradient acétonitrile/$H_2O$ de 95:5 (v/v) à 5:95 en 23 mn.

On obtient 0,1 g (isomère (S,S)) d'un solide blanc,

LC/MS : $MH^+$ = 483.

RMN 500MHz (DMSO) δppm : 0,80 (t,3H) ; 1,25 (d, 6H) ; 1,28 (m, 2H) ; 1,75 (m,2H) ; 2,80 (s,3H) ; 3,00 (s,3H) ; 3,25 (m, 1H) ; 4,49 (m, 1H) ; 5,08 (s, 1H) ; 6,55 (s large, 1H) ; 7,13 (d,3H) ; 8,25 (d,1 H) ; 12,15 (s, 1H).

et 0,17 g (isomère (R,S)) d'un solide blanc.

LC/MS : $MH^+$ = 483.

RMN 500MHz (DMSO) δppm : 0,88 (t,3H) ; 1,22 (d,6H) ; 1,30 (m, 2H) ; 1,68 (m,2H) ; 2,87 (s,3H) ; 2,98 (s,3H) ; 3,25 (m,1H) ; 4,45 (m,1H) ; 5,10 (s,1H) ; 6,42 (s,1H) ; 7,18 (m,5H); 8,30 (d,1H) ; 12,20 (s,1H).

**Exemple 11 : 2-[2-[2-hydroxy-2-phényl-(2S)-acétylamino]-(2S)-pentanoyl-amino]-5-(1-méthyléthyl)-thiazole-4-carboxylate de méthyle (composé n° 30) et 2-[2-[2-hydroxy-2-phényl-(2R)-acétylamino]-(2S)-pentanoylamino]-5-(1-méthyléthyl)-thiazole-4-carboxylate de méthyle (composé n° 31)**

A 0,35 g de 2-(2-amino-(2S)-pentanoylamino)-5-(1-méthyléthyl)thiazole-4-carboxylate de méthyle, obtenu à l'étape 5.1 de l'exemple 5, en solution dans 20 ml de diméthylformamide à 0°C, on additionne 0,13 g de N-méthylmorpholine, 0,67 g de PyBOP puis 0,20 g d'acide (S)-mandélique ou d'acide (R)-mandélique. On laisse le milieu réactionnel revenir à température ambiante puis on l'agite 16 h et on le concentre. On reprend le résidu à l'acétate et on le lave selon le procédé décrit dans l'exemple 7. Après évaporation de la phase organique, on chromatographie le résidu sur une colonne de silice en éluant avec un mélange d'acétate d'éthyle et d'éther de pétrole 1:1 (v/v). On obtient 0,36 g du diastéréoisomère (S,S) et 0,37 g du diastéréoisomère (R,S), sous forme d'une poudre blanche.

Isomère (S,S):

LC/MS : $MH^+$ 434.

RMN 500MHz (DMSO) δppm : 0,80 (t,3H); 1,16 (m,2H); 1,26 (s,9H); 1,68 (m,2H) ; 3,78 (s,3H) ; 3,97 (m,1 H) ; 4,43 (m,1 H) ; 4,98 (d,1 H) ; 6,31 (d,1H); 7,32 (m,5H) ; 8,14 (d,1 H) ; 12,46 (s,1 H).

$\alpha_D^{20} = -51°$ (c=1.0, MeOH).

Isomère (R,S) :
LC/MS : $MH^+$ = 434.
RMN 500MHz (DMSO) δppm : 0,85 (t,3H); 1,28 (s,6H); 1,28 (m,2H); 1,68 (m,2H) ; 3,78 (s,3H) ; 3,97 (m,1H) ; 4,44 (m, 1H) ; 5,00 (d,1H) ; 6,17 (d,1H) ; 7,30 (m,5H) ; 8,17 (d,1 H) ; 12,51 (s,1H).

$\alpha_D^{20} = -110°$ (c=1.0, MeOH).

**Exemple 12 : 2-[2-[2-(3,5-difluorophényl)-2-oxoacétylamino]-(2S)-pentanoylamino]-5-(1-méthyléthyl)-thiazole-4-carboxylate de méthyle (composé n° 75).**

A 0,3 g 2-[2-[2-(3,5-difluorophényl)-2-hydroxy-acétylamino]-(2S)-pentanoyl-amino]-5-(1-méthyléthyl)-thiazole-4-carboxylate de méthyle, obtenu à l'exemple 7, en solution dans 40 ml de dichlorométhane absolu stabilisé à l'amylène, on additionne 0,55 g de réactif de Dess-Martin periodinane et 0,09 g de *tert*-butanol. On agite le mélange réactionnel à température ambiante pendant 20 h puis on l'évapore. On chromatographie le résidu (sans traitement préalable) sur une colonne de silice en éluant avec un mélange d'éther de pétrole et d'acétate d'éthyle 8 :2 (v/v). On obtient 0,20 g d'une poudre blanche.
LC/MS : $MH^+$ = 468 (pureté 100 %)
RMN 500MHz (DMSO) δppm : 0,97 (t,3H); 1,20 (d,6H); 1,40 (m,2H); 1,80 (m,2H) ; 3,87 (s,3H) ; 4,05 (m,1H) ; 4,60 (m, 1H); 7,60 (m,2H) ; 7,80 (m, 1H) ; 7,99 (m,2H) ; 9,30 (d,1 H) ; 12,70 (s,1 H).

**Exemple 13 : 2-[2-[2-(3,5)-difluorophényl-2-hydroxy-(2S)-acétylamino]-(2S)-pentanoylamino]-4-méthylthiazole-5-carboxylate de méthyle (composé n° 165).**

Le composé du titre peut être obtenu selon les procédés décrits dans les exemples 7 et 8.
A 0,90 g de 2-(2-amino-(2S)-pentanoylamino)-4-(méthyl)-thiazole-5-carboxylate de méthyle obtenu selon le procédé décrit dans l'exemple 5.1, en solution dans 75 ml de diméthylformamide à 0°C, on additionne 0,366 g de N-méthylmorpholine, 1,87g de PyBOP puis 0,677 g d'acide 3,5-difluoromandélique. On laisse revenir le milieu réactionnel à température ambiante, on l'agite 16 h, puis on le concentre. On reprend le résidu par de l'acétate d'éthyle, on le lave 2 fois avec une solution aqueuse saturée en bicarbonate de sodium, 2 fois à l'eau, 1 fois avec une solution aqueuse (1M) d'hydrogénosulfate de potassium puis une solution aqueuse saturée en chlorure de sodium. On sèche la phase organique sur sulfate de sodium anhydre puis on la concentre. On chromatographie le résidu sur une colonne de gel de silice, en éluant avec un mélange d'éther de pétrole et d'acétate d'éthyle allant de 8:2 à 7:3 (v/v). On obtient 0,1 g d'une poudre blanche (isomère S,S) et 0,7 g (mélange des 2 diastéréoisomères).
Isomère (S,S) :
LC/MS $MH^+$ = 442.
RMN 500MHz (DMSO) δppm : 0,82 (t,3H) ; 1,26 (m,2H) ; 1,69 (m,2H) ; 2,63 (s,3H) ; 3,77 (s,3H) ; 4,50 (m,1H) ; 5,07 (d, 1H) ; 6,55 (d,1H) ; 7,16 (m,3H) ; 8,31 (d, 1H) ; 12,65 (s,1H).

**Exemple 14 : 2-[2-[2-(3,5)-difluorophényl)-2-oxo-acétylamino]-(2S)-pentanoyl-amino]-4-méthylthiazole-5-carboxylate de méthyle (composé n° 167).**

A 0,7 g de 2-[2-[2-(3,5)-difluorophényl-2-hydroxy-(2S)-acétylamino]-(2S)-pentanoylamino]-4-méthylthiazole-5-carboxylate de méthyle, obtenu à l'exemple 13 en solution dans 80 ml de dichlorométhane absolu stabilisé à l'amylène, on additionne 1,35 g de réactif de Dess-Martin periodinane et 0,24 g de *ter*-butanol.
On agite le mélange réactionnel à température ambiante pendant 20 h puis on l'évapore. On chromatographie le résidu (sans traitement préalable) sur une colonne de silice en éluant avec un mélange d'éther de pétrole et d'acétate d'éthyle 8:2 (v/v). On obtient 0,68 g d'une poudre blanche.
LC/MS : $MH^+$ = 440
$\alpha_D$ = - 87° (c = 1, MeOH)
RMN 500MHz (DMSO) δppm : 0,90 (t,3H) ; 1,38 (m,2H); 1,75 (m,2H) ; 2,63 (s,3H); 3,78 (s,3H) ; 4,61 (m,1H) ; 7,69 (m, 3H) ; 9,43 (d,1H).

**Exemple 15 : 2-[2-[2-hydroxy-3,3-diméthyl-(2S)-butyrylamino]-(2S)-pentanoylamino]-5-(1-méthyléthyl)-thiazole-4-carboxylate de méthyle (composé n° 97).**

A 0,24 g de 2-(2-amino-(2S)-pentanoylamino)-5-(1-méthyléthyl)thiazole-4-carboxylate de méthyle, obtenu à l'étape 5.1 de l'exemple 5, en solution dans 10 ml de diméthylformamide à 0°C, on additionne 97μl de N-méthylmorpholine, 0.116 g d'acide (S)-(-)-2-hydroxy-3,3-diméthylbutyrique puis 0,46 g de PyBOP. On agite le milieu réactionnel pendant 18 heures après retour à température ambiante puis on le concentre sous pression réduite. On reprend le résidu dans 50 ml d'acétate d'éthyle et on le lave successivement avec 50 ml d'une solution saturée de $KHSO_4$ dans $H_2O$, 50 ml d'une solution saturée de $K_2CO_3$ dans $H_2O$, 50 ml d'une solution saturée de NaCl puis 50 ml de $H_2O$. Après séchage et évaporation de la phase organique, on chromatographie le résidu sur une colonne de silice (40 g) éluée avec un gradient allant de 100% d'éther de pétrole à un mélange 30/70 d' éther de pétrole/acétate d'éthyle (v/v). On obtient 0,269 g de cristaux blancs.
LC/MS : $MH^+$ = 414.
RMN 500MHz (DMSO) δppm : 0,90 (t,3H) ; 0,93 (s,9H) ; 1,30 (d,6H) ; 1,32 (m,2H) ; 1,71 (m,2H) ; 3,58 (d,1H); 3,82 (s, 3H) ; 4,02 (m,1H); 4,52 (m,1 H) ; 5,60 (d,1 H) ; 7,82 (d,1 H) ; 12,45 (s,1H).

$\alpha_D^{20}$ = - 74° (c=1.0, MeOH).

**Exemple 16 : 2-[2-(2-fluoro-2-phényl-(2S)-acétylamino)-(2S)-pentanoyl-amino]-5 (1-méthyléthyl)-thiazole-4-carboxylate de méthyle (composé n° 98)** et **2-[2-(2-fluoro-2-phényl-(2R)-acétylamino)-(2S)-pentanoylamino]-5 (1-méthyléthyl)-thiazole-4-carboxylate de méthyle (composé n° 99).**

A 0,60 g de 2-(2-amino-(2S)-pentanoylamino)-5-(1-méthyléthyl)thiazole-4-carboxylate de méthyle, obtenu à l'étape 5.1 de l'exemple 5, en solution dans 15 ml de diméthylformamide à 0°C, on additionne 242μl de N-méthylmorpholine, 0.34 g d'acide α-fluorophénylacétique puis 1,144 g de PyBOP. On agite le milieu réactionnel pendant 18 heures après retour à température ambiante puis on le concentre sous pression réduite. On reprend le résidu dans 50 ml d'acétate d'éthyle et on le lave successivement avec 50 ml d'une solution saturée de $KHSO_4$ dans $H_2O$, 50 ml d'une solution saturée de $K_2CO_3$ dans $H_2O$, 50 ml d'une solution saturée de NaCl puis 50 ml de $H_2O$. Après séchage et évaporation de la phase organique, on chromatographie le résidu sur une colonne de silice (40 g) éluée avec un gradient allant de 100% d'éther de pétrole à un mélange 30/70 d'éther de pétrole/acétate d'éthyle (v/v). On obtient 123 mg d'isomère (S, S) et 177 mg d'isomère (R, S).
Isomère (S, S) :
LC/MS : $MH^+$ = 436.
RMN 500MHz (DMSO) δppm : 0,87 (t,3H) ; 1,22 (d,6H) ; 1,32 (m,2H) ; 1,70 (m,2H) ; 3,78 (s,3H) ; 3,98 (m,1 H) ; 4,52 (m,1 H) ; 5,90 et 6,00 (2s,1H) ; 7,44 (m,5H) ; 8,70 (d,1H) ; 12,55 (s,1 H).

$\alpha_D^{20}$ = - 55° (c=1.0, MeOH)
Isomère (R, S) :
LC/MS : $MH^+$ = 436.
RMN 500MHz (DMSO) δppm : 0,90 (t,3H) ; 1,24 (m,2H) ; 1,30 (s,6H) ; 1,77 (m,2H) ; 3,85 (s,3H) ; 4,05 (m,1 H) ; 4,48 (m,1H); 5,97 et 6,05 (2s,1H) ; 7,50 (m,5H) ; 8,80 (d,1 H) ; 12,62 (s,1H).

$\alpha_D^{20}$ = - 71.3° (c=1.0, MeOH)

**Exemple 17 : 2-[2-[2-(3-chloro)-2-hydroxy-(2S)-acétylamino]-(2S)-pentanoyl-amino]-5-(1-méthyléthyl)-thiazole-4-carboxylate de méthyle (composé n° 107)**

**Exemple 17.1 : acide 2-(3-chlorophényl)-2-hydroxy-(2S)-acétique**

A une solution de 2,85 ml de 3-chlorobenzaldéhyde dans 20 ml de dichlorométhane, on additionne avec précaution 3,66 ml de cyanure de triméthylsilyle puis une quantité catalytique d'iodure de zinc ($ZnI_2$). On agite le milieu réactionnel pendant 3 heures à température ambiante puis à 60°C durant 2 heures. On refroidit le milieu réactionnel à 0°C et on additionne 9 ml d'HCl concentré. On agite le milieu réactionnel pendant 18 heures à température ambiante puis 1 heure à reflux. Après refroidissement, on verse le mélange réactionnel dans de l'eau et on extrait deux fois avec 50 ml AcOEt. On extrait les phases organiques combinées avec 100 ml de NaOH 7.5N à 4°C. Après séparation, on lave la phase aqueuse avec 3X 50 ml d'AcOEt. On acidifie la phase aqueuse avec 70 ml de HCl 12N et on l'extrait avec 3X 50 ml d'AcOEt. On sèche les phases organiques rassemblées et on évapore le solvant.

L' acide (R)-3-chloromandélique est séparé par cristallisation sous forme de sel de (R)-(+)-phénéthylamine. L' acide (S)-3-chloromandélique est obtenu à partir des eaux mères par cristallisation sous forme de sel de (S)-(-)-phénéthylamine. On obtient 78 mg de cristaux blancs.

On utilise l'acide obtenu sans autre purification dans l'étape suivante.

**Exemple 17.2 : 2-[2-[2-(3-chloro)-2-hydroxy-(2S)-acétylamino]-(2S)-pentanoylamino]-5-(1-méthyléthyl)-thiazole-4-carboxylate de méthyle**

A 0,072 g de 2-(2-amino-(2S)-pentanoylamino)-5-(1-méthyléthyl)thiazole-4-carboxylate de méthyle, obtenu à l'étape 5.1 de l'exemple 5, en solution dans 5 ml de diméthylformamide à 0°C, on additionne 29µl de N-méthylmorpholine, 0.081 g d'acide (S)-3-chloromandélique puis 0,137 g de PyBOP. On agite le milieu réactionnel pendant 40 heures après retour à température ambiante puis on le concentre sous pression réduite. On reprend le résidu dans 50 ml d'acétate d'éthyle et on le lave successivement avec 50 ml d'une solution saturée de $KHSO_4$ dans $H_2O$, 50 ml d'une solution saturée de $K_2CO_3$ dans $H_2O$, 50 ml d'une solution saturée de NaCl puis 50 ml de $H_2O$. Après séchage et évaporation de la phase organique, on chromatographie le résidu sur une colonne de silice (40 g) éluée avec un gradient allant de 100% d'éther de pétrole à 100% d'acétate d'éthyle. On obtient 0,059 g de cristaux blancs.

LC/MS : $MH^+$ = 468.

RMN 500MHz (DMSO) δppm : 0,87 (t,3H) ; 1,20 (m,2H) ; 1,22 (d,6H) ; 1,70 (m,2H) ; 3,80 (s,3H) ; 4,00 (m,1H) ; 4,43 (m,1H) ; 5,02 (s,1H) ; 6,44 (s,1H) ; 7,30 (m,3H) ; 7,45 (s,1 H) ; 8,20 (d,1 H) ; 12,45 (s,1 H).

**Exemple 18 : 2-[2-(3-éthyl-2-hydroxy-(2S)-pentanoylamino)-(2S)-pentanoyl-amino]-5-(1-méthyléthyl)-thiazole-4-carboxylate de méthyle (composé n° 108) et 2-[2-(3-éthyl-2-hydroxy-(2R)-pentanoylamino)-(2S)-pentanoyl-amino]-5-(1-méthyléthyl)-thiazole-4-carboxylate de méthyle (composé n° 109)**

**Exemple 18.1 : acide 3-éthyl-2-hydroxypentanoïque**

A une solution de 1,24 ml de 2-éthylbutyraldéhyde dans 18 ml de dichlorométhane anhydre, on additionne avec précaution 1,5 ml de cyanure de triméthylsilyle puis une quantité catalytique de $ZnI_2$. On agite le milieu réactionnel pendant 2 heures à température ambiante puis à 60°C durant 3,5 heures. On refroidit le milieu réactionnel à 0°C et on additionne 3,5 ml d'HCl concentré. On agite le milieu réactionnel pendant 18 heures à température ambiante puis 1 heure à reflux. Après refroidissement, on verse le mélange réactionnel dans de l'eau et on extrait deux fois avec 50 ml d'AcOEt. On extrait les phases organiques combinées avec 100 ml de NaOH 7.5N à 4°C. Après séparation, on lave la phase aqueuse avec 3X 50 ml d'AcOEt. On acidifie la phase aqueuse avec 70 ml de HCl 12N et on extrait avec 3X 50 ml d'AcOEt. On sèche les phases organiques rassemblées et on évapore le solvant.

On utilise l'acide obtenu sans autre purification dans l'étape suivante.

**Exemple 18.2 : 2-[2-(3-éthyl-2-hydroxy-(2S)-pentanoylamino)-(2S)-pentanoylamino]-5-(1-méthyléthyl)-thiazole-4-carboxylate de méthyle et 2-[2-(3-éthyl-2-hydroxy-(2R)-pentanoylamino)-(2S)-pentanoylamino]-5-(1-méthyléthyl)-thiazole-4-carboxylate de méthyle**

A 0,457 g de 2-(2-amino-(2S)-pentanoylamino)-5-(1-méthyléthyl)-thiazole-4-carboxylate de méthyle, obtenu à l'étape 5.1 de l'exemple 5, en solution dans 15 ml de diméthylformamide à 0°C, on additionne 181 µl de N-méthylmorpholine, 0,45 g d'acide 3-éthyl-2-(S)-hydroxypentanoïque puis 0,858 g de PyBOP. On agite le milieu réactionnel pendant 18 heures après retour à température ambiante puis on le concentre sous pression réduite. On reprend le résidu dans 50 ml d'acétate d'éthyle et on le lave successivement avec 50 ml d'une solution saturée de $KHSO_4$ dans $H_2O$, 50 ml d'une solution saturée de $K_2CO_3$ dans $H_2O$, 50 ml d'une solution saturée de NaCl puis 50 ml de $H_2O$. Après séchage et évaporation de la phase organique, on chromatographie le résidu sur une colonne de silice (40 g) éluée avec un gradient allant de 100% d'éther de pétrole à un mélange 20/80 d'éther de pétrole/acétate d'éthyle (v/v). On obtient 78 mg d'isomère (S, S) et 131 mg d'isomère (R, S).

Isomère (S,S):

LC/MS : $MH^+$ = 428.

RMN 500MHz (DMSO) δppm: 0,86 (t,3H) ; 0,93 (m,6H) ; 1,24 (m,2H); 1,32 (d,6H); 1,38 (m,4H); 1,55 (m, 1H) ; 1,72 (m, 2H); 3,78 (s,3H) ; 3,98 (m, 1H) ; 4,05 (m, 1H) ; 4,55 (m, 1H) ; 5,55 (d, 1H) ; 7,92 (d, 1H) ; 12,50 (s, 1H).

$\alpha_D^{20} = -53.9°$ (c=1.0, MeOH).

Isomère (R, S) :

LC/MS : $MH^+$ = 436.

RMN 500MHz (DMSO) δppm : 0,80 (t,3H) ; 0,90 (m,6H) ; 1,20 (m,2H) ; 1,30 (d,6H); 1,34 (m,4H) ; 1,55 (m, 1H) ; 1,74 (m,2H) ; 3,86 (s,3H) ; 4,00 (m,2H) ; 4,53 (m,1H) ; 5,42 (d,1H) ; 8,00 (d,1H) ; 12,54 (s,1 H).

$\alpha_D^{20}$ = - 26.9° (c=1.0, MeOH).

**Exemple 19 : 2-[2-[3-(3,5-difluorophényl)-2-hydroxy-(2S)-propionylamino]-(2S)-pentanoylamino]-5-(1-méthyléthyl)-thiazole-4-carboxylate de méthyle (composé n° 110)**

**Exemple 19.1 : acide 3-(3,5-difluorophény)-2-hydroxy-(2S)-propionique**

A une suspension de 1,6 g de (S)-3,5-difluorophénylalanine dans 5,3 ml de $H_2SO_4$ (2,5N), on additionne goutte à goutte à 0°C une solution de 0,829 g de nitrite de sodium dans 4,2 ml d'$H_2O$. On agite le mélange réactionnel pendant 2 heures à 0°C puis 17 heures à température ambiante. On extrait le mélange réactionnel avec 2X100 ml d'AcOEt. On lave les phases organiques rassemblées avec 100 ml de solution saturée de NaCl dans $H_2O$. On obtient après séchage 1,197 g de cristaux jaunes.
On utilise l'acide obtenu sans autre purification dans l'étape suivante.

**Exemple19.2 :2-[2-[3-(3,5-difluorophényl)-2-hydroxy-(2S)-propionyl-amino]-(2S)-pentanoylamino]-5-(1-méthyléthyl)-thiazole-4-carboxylate de méthyle**

A 0,897 g de 2-(2-amino-(2S)-pentanoylamino)-5-(1-méthyléthyl)thiazole-4-carboxylate de méthyle, obtenu à l'étape 5.1 de l'exemple 5, en solution dans 10 ml de diméthylformamide à 0°C, on additionne 363µl de N-méthylmorpholine, 0,666 g d'acide 3-(3,5-difluorophényl)-2-hydroxy-propionique puis 1,72 g de PyBOP. On agite le milieu réactionnel pendant 17 heures après retour à température ambiante puis on le concentre sous pression réduite. On reprend le résidu dans 50 ml d'acétate d'éthyle et on le lave successivement avec 50 ml d'une solution saturée de $KHSO_4$ dans $H_2O$, 50 ml d'une solution saturée de $K_2CO_3$ dans $H_2O$, 50 ml d'une solution saturée de NaCl puis 50 ml de $H_2O$. Après séchage et évaporation de la phase organique, On chromatographie le résidu sur une colonne de silice (90 g) éluée avec un gradient allant de 100% d'éther de pétrole à un mélange 10/90 d'éther de pétrole/acétate d'éthyle (v/v). On obtient 0,43 g de cristaux blancs.
LC/MS : $MH^+$ = 484.
RMN 500MHz (DMSO) δppm : 0,82 (m,3H) ; 1,10 (m,2H) ; 1,27 (m,6H) ; 1,61 (m,2H) ; 2,83 et 2,95 (2m,1H) ; 3,77 (s, 3H) ; 3,96 (m,1H) ; 4,21 (m,1H) ; 4,43 (m, 1H) ; 5,68 et 5,81 (2d,1H) ; 6,93-7,01 (m,3H) ; 7,84 et 7,97 (2d,1H) ; 12,46 (s, 1H).

$\alpha_D^{20}$ = - 32.8° (c=1.0, MeOH).

**Exemple20 :2-[2-[2-(2-benzyloxyphényl)-2-hydroxy-acétylamino]-(2S)-pentanoylamino]-5-(1-méthyléthyl)-thiazole-4-carboxylate de méthyle (composé n° 122).**

**Exemple 20.1 : acide (2-benzyloxyphényl)hydroxyacétique**

A une solution de 1,24 ml de 2-benzyloxybenzaldéhyde dans 10 ml de dichlorométhane anhydre, on additionne avec précaution 4,4 ml de cyanure de triméthylsilyle puis une quantité catalytique de $ZnI_2$. On agite le milieu réactionnel pendant 2,5 heures à température ambiante puis à 60°C durant 4 heures. On refroidit le milieu réactionnel à 0°C et on additionne 10.5 ml d'HCl concentré. On agite le milieu réactionnel est pendant 18 heures à température ambiante puis 1 heure à reflux. Après refroidissement, on verse le mélange réactionnel dans de l'eau et on extrait deux fois avec 50 ml d'AcOEt. On extrait les phases organiques combinées avec 100 ml de NaOH 7,5N à 4°C. Après séparation, on lave la phase aqueuse est lavée avec 3X 50 ml d'AcOEt. On acidifie la phase aqueuse avec 70 ml de HCl 12N et on l'extrait avec 3X 50 ml AcOEt. On sèche les phases organiques rassemblées et on évapore le solvant.
On utilise l'acide obtenu sans autre purification dans l'étape suivante.

**Exemple 20.2 : 2-[2-[2-(2-benzyloxyphényl)-2-hydroxy-acétylamino]-(2S)-pentanoylamino]-5-(1-méthyléthyl)-thiazole-4-carboxylate de méthyle**

A 1,88 g de 2-(2-amino-(2S)-pentanoylamino)-5-(1-méthyléthyl)thiazole-4-carboxylate de méthyle, obtenu à l'étape 5.1 de l'exemple 5, en solution dans 50 ml de diméthylformamide à 0°C, on additionne 760 µl de N-méthylmorpholine, 13g d'acide (2-benzyloxyphenyl)hydroxyacétique puis 3,6 g de PyBOP. On agite le milieu réactionnel 17 heures

après retour à température ambiante puis on le concentre sous pression réduite. On reprend le résidu dans 100 ml d'acétate d'éthyle et on le lave successivement avec 100 ml d'une solution saturée de $KHSO_4$ dans $H_2O$, 100 ml d'une solution saturée de $K_2CO_3$ dans $H_2O$, 100 ml d'une solution saturée de NaCl puis 100 ml de $H_2O$. Après séchage et évaporation de la phase organique, on chromatographie le résidu sur une colonne de silice (90 g) éluée avec un gradient allant de 100 % d'éther de pétrole à un mélange 30/70 d'éther de pétrole/acétate d'éthyle (v/v). On obtient 0,43 g d'une mousse blanche.
LC/MS : $MH^+$ = 540.

$\alpha_D^{20}$ = - 87.0° (c=1.0, MeOH).
RMN 500MHz (DMSO) δppm : 0,85 (m,3H) ; 1,26 (m,2H) ; 1,28 (d,6H) ; 1,69 (m,2H); 3,78 (s,3H); 3,98 (m,1H); 4,47 (m, 1H); 5,22 (s,2H); 5,33 (2d,1H); 6,07 et 6,11 (2d,1H); 6,90-7,50 (m,8H) 8,00 (m,1 H) ; 12,48 (s,1 H).

**Exemple21 :2-[2-[2-hydroxy-2-(2-hydroxyphényl)acétylamino]-(2S)-pentanoylamino]-5-(1-méthyléthyl)-thiazole-4-carboxylate de méthyle (composé n° 128).**

A une solution de 1,187 g de 2-[2-[2-(2-benzyloxyphényl)-2-hydroxy-acétylamino]-(2S)-pentanoylamino]-5-(1-méthyléthyl)-thiazole-4-carboxylate de méthyle, obtenu à l'exemple 20 dans 10 ml d'éthanol sous atmosphère d'azote on ajoute 144 mg de Pd/C (10%). On agite le mélange réactionnel 24 h sous atmosphère de $H_2$. On élimine le catalyseur par filtration et on évapore le solvant sous pression réduite. On chromatographie le résidu sur une colonne de silice (90g) éluée avec un gradient allant de 100 % d'éther de pétrole à un mélange 40/60 d'éther de pétrole/acétate d'éthyle (v/v). On obtient 0,68 g de cristaux blancs.
LC/MS : $MH^+$ = 450.

$\alpha_D^{20}$ = - 89.5° (c=1.0, MeOH).
RMN 500MHz (DMSO) δppm : 0,91 (m,3H) ; 1,28 (m,2H) ; 1,30 (d,6H) ; 1,80 (m,2H) ; 3,85 (s,3H) ; 4,07 (m,1H) ; 4,53 (m,1H) ; 5,31 et 5,35 (2s,1H) ; 6,87 (m,2H) ; 7,13 (m,1H); 7,27 (m,1H); 8,17 (m,1H); 9,66 (s,large 1 H) ; 12,49 , (s,1 H).

**Exemple 22 : 2-[2-[2-(3,5-difluorophényl)-2-hydroxy-(2S)-acétylamino]-(2S)-(3-méthoxypropionyl)amino]-5-(1-méthyléthyl)-thiazole-4-carboxylate de méthyle (composé n° 60) et 2-[2-[2-(3,5-difluorophényl)-2-hydroxy-(2R)-acétylamino]-(2S)-(3-méthoxypropionyl)amino]-5-(1-méthyléthyl)-thiazole-4-carboxylate de méthyle (composé n° 61).**

**Exemple 22.1 : 2-[(2S)-2-amino-3-méthoxypropionylamino]-5-(1-méthyléthyl)-thiazole-4-carboxylate de méthyle**

A 2,00 g de 2-amino-5-(1-méthyléthyl)thiazole-4-carboxylate de méthyle, obtenu à l'exemple 1, en solution dans 100 ml de diméthylformamide à 0°C, on additionne 1,01 g de N-méthylmorpholine, 5,72 g de PyBOP puis 2,40 g de (S)-BOC-O-méthylsérine, dicyclohexylamine. On laisse revenir la réaction à température ambiante et on l'agite pendant 18 h.

On évapore le solvant, on reprend le résidu à l'acétate d'éthyle, et on le lave 2 fois avec une solution aqueuse saturée en bicarbonate de sodium, 1 fois à l'eau, et 1 fois avec une solution aqueuse 1 N d'hydrogénosulfate de potassium puis avec une solution aqueuse saturée en chlorure de sodium.
On sèche la phase organique sur sulfate de sodium anhydre et on la concentre. On chromatographie le résidu sur une colonne de silice en éluant avec un mélange éther de pétrole et acétate d'éthyle 8:2 (v/v) pour obtenir 2,70 g d'une poudre blanche.
LC/MS : $MH^+$ = 402.
RMN 300 MHz ($CDCl_3$) : 1,33 (d,6H) ; 1,48 (s,9H); 3,32 (s,3H) ; 3,33 et 3,99
(2m,2H) ; 3,55 (m,1H); 3,92 (s,3H) ; 4,13 (m,1H); 4,5 (s, large 1H); 5,40 (d,1 H).
On agite 4,30 g de produit obtenu de la manière décrite ci-dessus en solution dans 60 ml d'acide trifluoroacétique à température ambiante pendant 30 min, puis on l'évapore.
On reprend le résidu à l'acétate d'éthyle, et on le lave 2 fois avec une solution aqueuse saturée en carbonate de sodium, puis avec une solution aqueuse saturée en chlorure de sodium. On sèche la phase organique sur sulfate de sodium anhydre, puis on l'évapore pour donner 0,50 g d'un solide blanc, qui est utilisé sans purification à l'étape suivante.

**Exemple 22.2 : 2-[2-[2-(3,5-difluorophényl)-2-hydroxy-(2S)-acétyl-amino]-(2S)-(3-méthoxypropionyl)amino]-5-(1-méthyléthyl)-thiazole-4-carboxylate de méthyle et 2-[2-[2-(3,5-difluorophényl)-2-hydroxy-(2R)-acétylamino]-(2S)-(3-méthoxypropionyl)amino]-5-(1-méthyléthyl)-thiazole-4-carboxylate de méthyle**

A 0,29 g de 2-[(2S)-2-amino-3-méthoxypropionylamino]-5-(1-méthyléthyl)-thiazole-4-carboxylate de méthyle, obtenu à l'étape 22.2, en solution dans 80 ml de diméthylformamide à 0°C, on additionne 0,106 g de N-méthylmorpholine, 0,55 g de PyBOP puis 0,20 g d'acide 3,5-difluoromandélique. On laisse revenir le milieu réactionnel à température ambiante, on l'agite 16 h, puis on le concentre. On reprend le résidu par de l'acétate d'éthyle, on le lave 2 fois avec une solution aqueuse saturée en bicarbonate de sodium, 2 fois à l'eau, 1 fois avec une solution aqueuse 1 M d'hydrogénosulfate de potassium puis avec une solution aqueuse saturée en chlorure de sodium. On sèche la phase organique sur sulfate de sodium anhydre puis on la concentre. On chromatographie le résidu sur une colonne de gel de silice, en éluant avec un mélange d'éther de pétrole et d'acétate d'éthyle (1:1) (v/v). Les deux isomères (S,S) et (R,S) peuvent être ainsi séparés. On obtient 0,10 g d'une poudre blanche [isomère (S,S)] et 0,50 g [isomère (R,S)].
Isomère (S,S) :
LC/MS : $MH^+$ = 472.
RMN 500MHz (DMSO) δppm : 1,25 (d,6H) ; 3,23 (s,2H) ; 3,59 (m,1H) ; 3,61 (m,1H) ; 3,78 (s,3H) ; 3,96 (m,1H) ; 4,64 (m,1H) ; 5,09 (s,1H) ; 6,62 (s,1H) ; 7,13 (m,3H) ; 8,23 (d,1 H) ; 12,53 (s,1 H).
Isomère (R,S):
LC/MS: $MH^+$=472.
RMN 500MHz (DMSO) δppm: 1,26 (t,3H) ; 3,23 (s,2H) ; 3,62 (m,1H) ; 3,71 (m,1 H) ; 3,78 (s,3H); 3,97 (m, 1H); 4,00 (m, 1 H); 5,14 (s,1 H); 6,56 (s,1 H) ; 7,13 (m,3H) ; 8,26 (d,1H); 12,59 (s,1 H).

**Exemple23:2-[2-[2-(3,5-difluorophényl)acétylamino]-(2S)-(3-méthoxy-propionyl)amino]-5-(1-méthyléthyl)-thiazole-4-carboxylate de méthyle (composé n° 59).**

A 0,21 g de 2-[2-[2-(3,5-difluorophényl)-2-hydroxy-acétylamino]-(2S)-(3-méthoxy-propionyl)amino]-5-(1-méthyléthyl)-thiazole-4-carboxylate de méthyle, obtenu à l'exemple 22, en solution dans 30 ml de diméthylformamide à 0°C, on additionne 0,09 g de N-méthylmorpholine, 0,42 g de PyBOP puis 0,14 g d'acide 3,5-difluorophénylacétique. On laisse revenir la réaction à température ambiante et on l'agite pendant 18 h. On évapore le milieu réactionnel. On reprend le résidu à l'acétate d'éthyle et on le lave 2 fois avec une solution aqueuse saturée en bicarbonate de sodium, 2 fois à l'eau, 1 fois avec une solution aqueuse 1M d'hydrogénosulfate de potassium, puis avec une solution aqueuse saturée en chlorure de sodium. On sèche la phase organique sur sulfate de sodium anhydre puis on la concentre. On chromatographie le résidu sur une colonne de silice en éluant avec un mélange éther de pétrole/acétate d'éthyle 1:1 (v/v) pour obtenir 0,12 g d'un solide blanc.
LC/MS : $MH^+$ = 456.
RMN 500MHz (DMSO) δppm : 1,26 (t,3H) ; 3,28 (d,3H) ; 3,55 (s,2H) ; 3,57 (m,2H); 3,78 (s,3H); 3,97 (m, 1H); 4,64 (m, 1H) ; 6,98 (d,2H); 7,08 (m, 1H) ; 8,57 (d,1 H) ; 12,54 (s, 1H).

**Exemple 24 : 2-[2-[2-(3,5-difluorophényl)acétylamino]-(2S)-butyrylamino]-5-(1-méthyléthyl)-thiazole-4-carboxylate de méthyle (composé n° 51).**

**Exemple 24.1 : 2-[(2S)-2-amino-butyrylamino]-5-(1-méthyléthyl)-thiazole-4-carboxylate de méthyle**

Le couplage de 2,03 g d'acide 2-(2S)-(*ter*-butoxycarbonyl)aminobutyrique dans 50 ml de N,N-diméthylformamide avec 1,92 g de 2-amino-5-(1-méthyléthyl)-thiazole-4-carboxylate de méthyle, obtenu à l'exemple 1 avec 5,72 g de PyBOP et 1,07 g de N-méthylmorpholine, donne après chromatographie 2,50 g d'une poudre blanche. La déprotection du groupement BOC dans le TFA donne après lavage basique 1,60 g d'un solide blanc qu'on utilise sans purification dans l'étape suivante.
LC/MS : $MH^+$ = 286.

**Exemple 24.2 : 2-[2-[2-(3,5-difluorophényl)acétylamino]-(2S)-butyrylamino]-5-(1-méthyléthyl)-thiazole-4-carboxylate de méthyle.**

Le couplage du 2-[(2S)-2-amino-butyrylamino]-5-(1-méthyléthyl)-thiazole-4-carboxylate de méthyle, obtenu à l'étape 24.1, dans du N,N-diméthylformamide, selon le procédé décrit dans l'exemple 5.2, avec 0,38 g d'acide 3,5-difluorophénylacétique, de 1,14 g de PyBOP et 0,22 g de N-méthylmorpholine donne après chromatographie 0,66 g d'une poudre blanche.
LC/MS : $MH^+$ = 440.

RMN 500MHz (DMSO) δppm : 0,79 (t,3H) ; 1,26 (d,6H) ; 1,73 (m,2H); 3,78 (s,3H) ; 3,96 (m,1H) ; 4,36 (m,1H) ; 5,06 (s, 1H) ; 6,56 (s,1H) 7,13 (m,3H) ; 8,22 (d,1H) ; 12,46 (s,1H).
$\alpha_D$ = - 73° (c=1, MeOH).

**Exemple 25 : 2-[2-[2-(3,5-difluorophényl)acétylamino]-(2S)-pentanoyl-amino]-4-méthyl-thiazole-5-N-méthyl-N-phényléthylcarboxamide (composé n° 176).**

Le composé de formule ci-dessus est obtenu en utilisant le procédé décrit dans l'exemple 9.

**Exemple 25.1 : 2-amino-4-méthyl-thiazole-5-N-méthyl-N-phényléthyl-carboxamide**

La réaction avec 5,58 g de 2-amino-4-méthyl-thiazole-5-carboxylate de méthyle obtenu selon le procédé décrit dans l'exemple 2, dans du tétrahydrofurane avec 7,19 g de di-*ter*-butyldicarbonate et 0,18 g de diméthylaminopyridine donne 6,90 g d'une poudre blanche utilisée telle quelle sans purification.
LC/MS : $MH^+$ =187.
RMN 300 MHz ($CDCl_3$) : 1,45 (s,9H) ; 2,48 (s,3H) ; 9,80 (s, large 1 H).
On obtient le 2-amino-4-méthyl-thiazole-5-N-méthyl-N-phényléthylcarboxamide, protégé sur l'amine par un BOC, par couplage selon le procédé décrit dans l'exemple 9.2. Pour cela on emploie 1,50 g de 2-*ter*-butoxycarbonylamino-4-méthyl-thiazole-5-carboxylate d'éthyle en solution dans 100 ml de N,N-diméthylformamide avec 0,88 g d'hydroxybenzotriazole, hydrate, 0,95 g EDAC, HCl et 0,78 g de N-méthyl-N-phénéthylamine. Après chromatographie, on obtient 1,10 g d'une poudre blanche.
LC/MS : $MH^+$ = 376.
$(M-BOC)^+$ = 276.
La déprotection du groupement protecteur BOC se fait selon le procédé décrit dans l'exemple 9.3. On obtient 0,90 g de 2-amino-4-méthyl-thiazole-5-N-méthyl-N-phényléthylcarboxamide sous forme d'une poudre blanche.
LC/MS : $MH^+$ = 276.

**Exemple 25.2 : 2-(2-amino-(2S)-pentanoylamino)-4-méthyl-thiazole-5-N-méthyl-N-phényléthylcarboxamide**

On réalise le couplage de 0,90 g de 2-amino-4-méthyl-thiazole-5-N-méthyl-N-phényléthylcarboxamide, obtenu à l'étape 25.1, avec 0,78 g de (S)-BOC norvaline selon le procédé décrit à l'étape 5.1 de l'exemple 5. On obtient après chromatographie sur silice éluée avec un mélange 1:1 (v/v) d'acétate d'éthyle et d'éther de pétrole 1,05 g d'une mousse blanche. LC/MS : $MH^+$ = 475.
On déprotège 1,05 g du composé obtenu ci-dessus dans 25 ml d'acide trifluoroacétique selon le procédé décrit à l'étape 9.3. On obtient 0,80 g d'un solide blanc.
LC/MS : $MH^+$ = 375.

**Exemple 25.3 : 2-[2-[2-(3,5-difluorophényl)acétylamino]-(2S)-pentanoyl-amino]-4-méthyl-thiazole-5-N-méthyl-N-phényléthyl-carboxamide**

On procède selon le même procédé que dans l'exemple 5.2.
Le couplage de 0,80 g de 2-(2-amino-(2S)-pentanoylamino)-4-méthyl-thiazole-5-N-méthyl-N-phényléthylcarboxamide dans 50 ml de N,N-diméthylformamide, avec 1,19 g PyBOP, 0,23 g N-méthylmorpholine et 0,40 g d'acide 3,5-difluorophénylacetique, donne après purification sur colonne de gel de silice éluée avec un mélange 1:1 (v/v) d'acétate d'éthyle et d'éther de pétrole 0,67 g d'une poudre blanche. LC/MS : $MH^+$ = 259.
RMN 500MHz (DMSO) δppm: 0,92 (t,3H) ; 1,38 (m,2H) ; 1,65 (m,2H); 2,14 (s,3H) ; 2,88 (m,2H) ; 2,97 (s,3H) ; 3,63 (s, 2H) ; 3,67 (m,2H) ; 4,51 (m,1 H) ; 7,04 (d,2H) ; 7,12 (t,1H) ; 7,24-7,30 (m,5H) ; 8,55 (d,1H) ; 12,46 (s,1H).
$\alpha_D$ = - 103° (c =1, MeOH).
Le tableau qui suit illustre les structures chimiques et les propriétés physiques de quelques exemples de composés selon l'invention.

**Tableau**

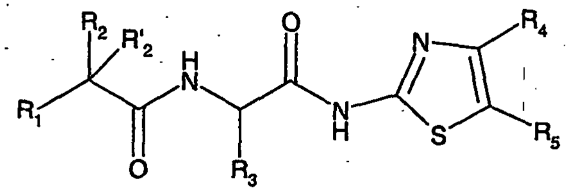

(I)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 1. | 3,5-difluorophényle | H, H | $-CH_3$ | $-COR_6$ | $-CH(CH_3)_2$ | $OCH_3$ | - | - | 1,16 (d,6H), 1,25 (d,3H), 3,53 (s,2H), 3,77 (s,3H), 3,97 (m, 1H), 4,36 (m, 1H), 6,97 (d,9H), 7,10 (m, 1H), 8,55 (d, 1H), 12,47 (s,1H)** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 2. | 3,5-difluorophényle | H, H | $-CH_3$ | $-COR_6$ | $-(CH_2)_2CH_3$ | $OCH_3$ | - | - | 0,91 (q,3H), 1,30 (d,3H), 1,61 (m,2H), 3,06 (t,2H), 3,53 (s,2H), 3,77 (s,3H), 4,38 (m, 1H), 6,98 (m,2H), 7,08 (m,1H), 8,57 (d,1H), 12,49 (s, H)*** |
| 3. | 3,5-difluorophényle | H,H | $-CH_3$ | $-COR_6$ | $-CH_2CH(CH_3)_2$ | $OCH_3$ | - | - | 0,89 (d,6H), 1,30 (d,3H), 1,85 (m,1H), 2,98 (d,2H), 3,53 (s,2H), 3,77 (s,3H), 4,38 (m,1H), 6,98 (d,2H), 7,09 (m,1H), 8,57 (d,1H), 12,50 (s,1H)** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 4. | 3,5-difluorophényle | H, H | $-CH_3$ | $-COR_6$ | $-CH_2C(CH_3)_3$ | $OCH_3$ | - | - | 0,91 (s,9H), 1,31 (d,3H), 3,07 (s,2H), 3,53 (s,2H), 3,76 (s,3H), 4,38 (m, 1 H), 6,97 (d,2H), 7,08 (m,1 H), 8,57 (d,1H), 12,51 (s,1 H)** |
| 5. | 3,5-difluorophényle | H, H | $-CH_3$ | $-COR_6$ | 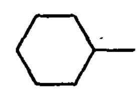 | $OCH_3$ | - | - | 1,30 (d,3H), 1,31 (m,5H), 1,67-1,97 (m, 5H), 3,53 (s, 2H), 3,65 (m, 1H), 3,77 (s, 3H), 4,38 (m,1 H), 6,98 (d,2H), 7,08 (m,1 H), 9,67 (d,1H), 12,47 (s,1H)** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 6. | 3,5-difluorophényle | H, H | $-CH_3$ | $-COR_6$ | $-(CH_2)_5CH_3$ | $OCH_3$ | - | - | 0,85 (t,3H), 1,29 (9H), 1,56 (m,2H), 3,10 (t, 2H), 3,69 (t, 2H), 3,77 (s, 3H), 4,41 (m, 1H), 6,97 (m, 2H), 7,07 (m, 1H), 8,56 (d, 1H), 12,48 (s, 1H)** |
| 7. | 3,4-difluorophényle | H, H | $-CH_3$ | $-COR_6$ | phényle | $OCH_3$ | - | - | 1,33 (d,3H), 3,50 (s,2H), 3,67 (s,3H), 4,42 (m,1H), 7,28-7,48 (m, 3H), 8,57 (d, 1H), 12,70 (s, 1H)** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 8. | 3,4-difluorophényle | H, H | $-CH_3$ | $-COR_6$ | 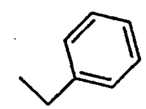 | $OCH_3$ | - | - | 1,25 (d,3H), 3,48 (s,2H), 3,85 (s,3H), 4,35 (m,1H), 4,47 (s,2H), 7,20-7,40 (m, 3H), 8,50 (d, 1H), 12,50 (s, 1H)** |
| 9. | 3,5-difluorophényle | H, H | $-CH_3$ | $-COR_6$ | H | $OCH_2CH_3$ | - | - | 1,25 (t+d,6H), 3,65 (s,2H), 4,25 (m,2H), 4,45 (m,1 H), 6,95 (d,2H), 7,10 (m,1 H), 8,05 (s,1H), 8,57 (d,1H), 12,65 (s,1H)** |
| 10. | 3,5-difluorophényle | H, H | $-CH_3$ | $-COR_6$ | $-CH_3$ | $OCH_3$ | - | - | 1,27 (d,3H), 2,57 (s,3H), 3,50 (s,2H), 3,74 (s,3H), 4,36 (m,1H), 6,96 (d,2H), 7,05 (m,1 H), 8,53 (d,1H), 12,43 (s,1H)** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 1 1. | 3,5-difluorophényle | H, H | $-CH_3$ | $-COR_6$ | 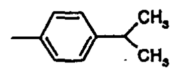 | $OCH_3$ | - | - | 1,22 (d,6H), 1,33 (d,3H), 2,93 (m,1 H), 3,54 (s,2H), 3,68 (s,3H), 4,42 (m,1 H), 6,98 (d,2H), 7,08 (m,1H), 7,29 (d,2H), 7,39 (d,2H), 8,60 (d,1H), 12,68 (s,1H)** |
| 12. | 3,5-difluorophényle | H, H | $-CH_3$ | $-COR_6$ | $-(CH_2)_2CH_3$ | $NR_7R_8$ | 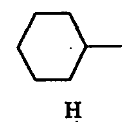 | - | 0,89 (t,3H), 1,29 (m,3H), 1,31 (3H), 1,22-1,87 (m, 10H), 3,10 (t, 2H), 3,53 (s, 2H), 3,67 (m,1 H), 4,43 (m,1 H), 6,98 (m, 2H), 7,07 (m, 1H), 7,41 (d, 1H), 8,54 (d, 1H), 12,18 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 13. | 3,5-difluorophényle | H, H | $-CH_3$ | $-COR_6$ | 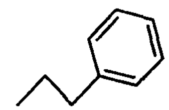 | OH | - | - | 1,30 (d,3H), 2,98 (t,2H), 3,38 (t,2H), 3,50 (s,2H), 4,41 (m, 1 H), 6,98 (d,2H), 7,07 (m,1 H), 7,16-7,28 (m, 5H), 8,53 (d, 1H)*** |
| 14. | 3,5-difluorophényle | H, H | $-CH_3$ | $-COR_6$ | $CH_3(CH_2)_2-$ | $NR_7R_8$ | 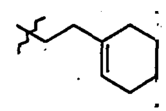 | - | 0,89 (t,3H), 1,30 (d,3H), 1,49 (m,4H), 1,95 (m,4H), 2,10 (m,2H), 3,11 (m,2H), 3,30 (m,2H), 3,53 (s,2H), 4,49 (m,1H), 5,39 (s,1H), 6,98 (d,2H), 7,09 (m,1 H), 7,61 (t,1H), 8,54 (d,1H), 12,13 (s,1H)** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 15. | 3,5-difluorophényle | H, H | $CH_3$ (S) | $-COR_6$ | $(CH_3)_2CH-$ | OH | - | - | 1,24 (d,6H), 1,27 (d,3H), 3,51 (s,2H), 4,02 (m,1H), 4,38 (m,1H), 6,98 (d,2H), 7,09 (m,1H), 8,56 (d,1H), 12,41 (s,1H)*** |
| 16. | 3,5-difluorophényle | H, H | $CH_3$ (S) | $-COR_6$ | $(CH_3)_2CH-$ | $OCH_3$ | - | - | 1,25 (d,6H), 1,30 (d,3H), 3,53 (s,2H), 3,78 (s,3H), 3,97 (m,1H), 4,36 (m,1H), 6,96-7,11 (m, 3H), 8,56 (d,1 H), 12,47 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 17. | 3,5-difluorophényle | OH, H (S) | $CH_3$ (S) | $-COR_6$ | $(CH_3)_2CH-$ | $OCH_3$ | - | - | 1,25 (d,6H), 1,30 (d,3H), 3,78 (s,3H), 3,97 (m, 1H), 4,40 (m, 1H), 5,04 (d, 1H), 6,55 (s,1 H), 7,13 (m, 3H), 8,34 (d,1 H), 12,43 (s, 1H)*** |
| 18. | 3,5-difluorophényle | H, H | $-(CH_2)_2CH_3$ (S) | $-COR_6$ | $(CH_3)_2CH-$ | $OCH_3$ | - | - | 0,85 (t,3H), 1,26 (d,6H), 1,28-1,65 (m, 4H), 3,53 (m, 2H), 3,78 (s, 3H), 3,97 (m, 1H), 4,36 (m, 1H), 6,96 (d, 2H), 7,08 (m, 1H), 8,49 (d,1 H), 12,52 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 19. | 3,5-difluorophényle | OH, H (S) | $-(CH_2)2CH_3$ (S) | $-COR_6$ | $(CH_3)_2CH-$ | $OCH_3$ | - | - 53° | 0,80 (t,3H), 1,18 (m, 2H), 1,22 (d,6H), 1,68 (m,2H), 3,78 (s,3H), 3,97 (m, 1H), 4,43 (m,1H). 5,05 (d,1 H), 6,55 (d,1 H), 7,13 (m,3H), 8,24 (d,1 H), 12,46 (s,1 H)*** |
| 20. | 3,5-difluorophényle | OH, H (R) | $-(CH_2)_2CH_3$ (S) | $-COR_6$ | $(CH_3)_2CH-$ | $OCH_3$ | - | - 83° | 0,82 (t,3H), 1,26 (d,6H), 1,26 (m,2H), 1,68 (m,2H), 3,78 (s,3H), 3,97 (m,1H), 4,40 (m,1 H), 5,07 (d,1H), 6,42 (d,1H), 7,12 (m,3H), 8,27 (d,1H), 12,51 (s,1H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 21. | 3,5-difluorophényle | H, H | $CH_3(CH_2)_2$-(S) | $-COR_6$ | $(CH_3)_2CH$- | $NR_7R_8$ | 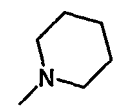 | | 0,85 (t,3H), 1,22 (d,6H), 1,27-1,65 (m, 11H), 3,19-3,30 (m, 3H), 3,51-3,73 (m,4H), 4,40 (m,1H), 7,09-6,90 (m, 3H), 8,49 (d, 1H), 12,22 (s, 1H)*** |
| 22. | 3,5-difluorophényle | H, H | $CH_3(CH_2)_2$-(S) | $-COR_6$ | $(CH_3)_3C$- | $OCH_3$ | - | | 0,85 (m,3H), 1,42 (s,9H), 1,30 (m,2H), 1,64 (m,2H), 3,29 (m,2H), 3,79 (s,3H), 4,37 (m,1 H), 6,98-7,09 (m, 3H), 8,46 (d,1 H), 12,39 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 23. | 3,5-difluorophényle | H, H | $CH_3(CH_2)_2$-(S) | $-COR_6$ | 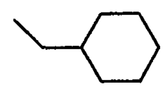 | $OCH_3$ | - | - | 0,85 (m,3H), 0,96 (t,2H), 0,95-1,69 (m, 14H), 2,99 (t, 2H), 3,54 (m, 2H), 3,77 (s, 3H), 4,37 (m,1 H), 6,97 (d,2H), 7,08 (m,1 H), 8,48 (d,1 H), 12,51 (s,1 H)*** |
| 24. | 3,5-difluorophényle | H, H | $CH_3$ (S) | $-COR_6$ | $(CH_3)_2CH$- | $OC(CH_3)_3$ | - | - | 1,26 (d,6H), 1,34 (d,3H), 1,51 (s,9H), 3,53 (s,2H), 3,90 (m,1 H), 4,39 (m,1 H), 6,93 (d,2H), 7,07 (m,1H), 8,51 (s,1H), 12,40 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 25. | 3,5-difluorophényle | H, H | $CH_3(CH_2)_3$-(S) | $-COR_6$ | $(CH_3)_2CH-$ | $OCH_3$ | - | - | 0,83 (t,3H), 1,25 (d,6H), 1,23-1,72 (m, 6H), 3,55 (m, 2H), 3,78 (s, 3H), 3,97 (m, 1H), 4,35 (m,1 H), 7,09-6,97 (m,3H), 8,47 (d,1H), 12,49 (s,1H)*** |
| 26. | 3,5-difluorophényle | H, H | $CH_3(CH_2)_2$-(S) | $-COR_6$ | $(CH_3)_2CH-$ | $NR_7R_8$ | $CH_3$, $CH_3$ | - 74° | 0,87 (t,3H), 1,24 (d,6H), 1,29-1,67 (m, 4H), 2,86 (s, 3H), 2,95 (s, 3H), 3,22 (m, 1H), 3,54 (m, 2H), 4,41 (m,1 H), 6,98 (d,2H), 7,08 (m,1H), 8,49 (d,1H), 12,21 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 27. | 3,5-difluorophényle | OH, H (S) | $CH_3(CH_2)_2$-(S) | $-COR_6$ | $(CH_3)_3C$- | $OCH_3$ | - | -54° | 0,81 (t,3H), 1,22 (m,2H), 1,43 (s,9H), 1,67 (m,2H), 3,78 (s,3H), 4,42 (m,1H), 5,05. (s,1 H), 6,55 (s,1 H), 7,12 (m,3H), 8,22 (d,1 H), 12,37 (s,1 H)*** |
| 28. | 3,5-difluorophényle | OH, H(R) | $CH_3(CH_2)_2$-(S) | $-COR_6$ | $(CH_3)_3C$- | $OCH_3$ | - | - 78° | 0,82 (t,3H), 1,25 (m,2H), 1,42 (s,9H), 1,69 (m,2H), 3,79 (s,3H), 4,40 (m,1 H), 5,07 (d,1H), 6,42 (d,1H), 7,13 (m,3H), 8,26 (d,1 H), 12,41 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 29. | 3,5-difluorophényle | H, H | $CH_3(CH_2)_2$-(S) | $-COR_6$ | $CH_3CH_2$- | $OCH_3$ | - | -70° | 0,86 (t,3H), 1,27 (t,3H), 1,29-1,35 (m, 2H), 1,63 (m, 2H), 3,10 (q, 2H), 3,54 (m, 2H), 3,78 (s, 3H), 4,39 (m,1 H), 6,98 (d,2H), 7,07 (m,1 H), 8,47 (d,1 H), 12,50 (s,1H)*** |
| 30. | phényle | OH, H (S) | $CH_3(CH_2)_2$-(S) | $-COR_6$ | $(CH_3)_2CH$- | $OCH_3$ | - | -51° | 0,80 (t,3H), 1,16 (m,2H), 1,26 (s,9H), 1,68 (m,2H), 3,78 (s,3H), 3,97 (m,1H), 4,43 (m,1 H), 4,98 (d,1 H), 6,31 (d,1 H), 7,32 (m,5H), 8,14 (d,1H), 12,46 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 31. | phényle | OH, H (R) | $CH_3$ $(CH_2)_2$-(S) | -$COR_6$ | $(CH_3)_2CH$- | $OCH_3$ | - | -110° | 0,85 (t,3H), 1,28 (s,6H), 1,28 (m,2H), 1,68 (m,2H), 3,78 (s,3H), 3,97 (m, 1H), 4,44 (m,1H), 5,00 (d,1H), 6,17 (d,1H), 7,30 (m,5H), 8,17 (d,1H), 12,51 (s,1H)*** |
| 32. | 3,5-difluorophényle | OH, H | $CH_3$ $(CH_2)_2$-(S) | -$COR_6$ | $(CH_3)_2CH$- | $OCH_3$ | - | -66° | 0,85 (m,3H), 1,28 (d,6H), 1,30 (m,2H), 3,78 (s,3H), 3,96 (m,1 H), 4,45 (m,1 H), 5,20 et 5,22 (2s,1H), 6,44-6,50 (2s, 1H), 7,05 (m, 1H), 7,19 (m,1 H), 7,44 (m,1 H), 8,19 et 8,23 (2d,1H), 12,50 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 33. | 3,4-difluorophényle | OH, H | $CH_3(CH_2)_2$-(S) | -$COR_6$ | $(CH_3)_2CH$- | $OCH_3$ | - | - 67° | 0,82 (m,3H), 1,26 (d,6H), 3,97 (m, 1H), 4,41 (m,1H), 5,03 (m,1H), 6,35 et 6,48 (2s,1H), 7,27 (m,1H), 7,42 (m,2H), 8,23 (m,1H), 12,48 (d,1H)*** |
| 34. | 2-chlorophényle | OH,H(R) | $CH_3(CH_2)_2$-(S) | -$COR_6$ | $(CH_3)_2CH$- | $OCH_3$ | - | -127° | 0,86 (t,3H), 1,26 (d,6H), 1,29 (m,2H), 1,78 (m,2H), 3,78 (s,3H), 3,97 (m,1 H), 4,46 (m,1H), 5,33 (s,1H), 6,44 (s,1H), 7,29 (d,2H), 7,39 (d,1 H), 7,45 (d,1 H), 8,24 (d,1 H), 12,51 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 35. | 3-chlorophényle | OH, H(R) | $CH_3(CH_2)_2$-(S) | $-COR_6$ | $(CH_3)_2CH-$ | $OCH_3$ | - | - 77° | 0,83 (t,3H), 1,23 (d,6H), 1,29 (m,2H), 1,69 (m,2H), 3,78 (s,3H), 3,96 (m,1 H), 4,39 (m,1H), 5,04 (s,1H), 6,31 (s,1H), 7,30-7,46 (m, 4H), 8,24 (d, 1H), 12,51 (s, 1H)*** |
| 36. | 2-chlorophényle | OH, H | $CH_3(CH_2)_2$-(S) | $-COR_6$ | $(CH_3)_2CH-$ | $OCH_3$ | - | - 65° | 0,86 (t,3H), 1,26 (d,6H), 1,34 (m,2H), 1,73 (m,2H), 3,78 (s,3H), 3,96 (m,1H), 4,46 (m,1 H), 5,34 (d,1H), 6,44 et 6,49 (2s,1H), 7,30 et 7,42 (2m,4H), 8,18 et 8,23 (2d,1 H), 12,50 (d.1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 37. | 2,6-difluorophényle | OH, H | $CH_3(CH_2)_2$-(S) | -$COR_6$ | $(CH_3)_2CH$- | $OCH_3$ | - | - 66° | 0,87 (t,3H), 1,26 (s,6H), 1,28 (m,2H), 3,79 (s,3H), 3,98 (m,1H), 4,54 (m,1H), 5,28 (d,1 H), 6,69 et 6,73 (2d,1 H), 7,06 (m,2H), 7,39 (m,1H), 8,11 et 8,21 (2d,1H), 12,54 (s,1H)*** |
| 38. | 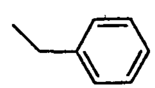 | OH, H(S) | $CH_3(CH_2)_2$-(S) | -$COR_6$ | $(CH_3)_2CH$- | $OCH_3$ | - | -58° | 0,81 (t,3H), 1,10 (m,2H), 1,26 (d,6H), 1,60 (m,2H), 2,74 et 2,95 (dd,2H), 3,78 (s,3H), 3,95 (m, 1H), 4,15 (m, 1H), 4,45 (m,1 H), 5,69 (d, 1H), 7,18 (m, 5H), 7,80 (d,1 H), 12,44 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 39. | 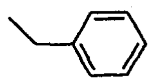 | OH, H (R) | $CH_3(CH_2)_2$-(S) | $-COR_6$ | $(CH_3)_2CH-$ | $OCH_3$ | - | -6° | 0,84 (t,3H), 1,18 (m,2H), 1,26 (m,6H), 1,64 (m,2H), 2,70 et 2,93 (dd,2H), 3,79 (s,3H), 3,98 (m, 1H), 4,14 (m, 1H), 4,45 (m, 1H), 5,57 (d, 1H), 7,09-7,21 (m,5H), 7,95 (d,1 H), 12,44 (s, 1H)*** |
| 40. | cyclohexyl | OH, H (S) | CH3 $(CH_2)_2$-(S) | $-COR_6$ | $(CH_3)_2CH-$ | $OCH_3$ | - | - 59° | 0,85 (t,3H), 1,11-1,18 (m, 7H), 1,24 (d, 6H), 1,28 (m, 1H), 1,65-1,69 (m,7H), 3,69 (d,1H), 3,78 (s, 3H), 3,97 (m, 1H), 4,50 (m, 1H), 5,46 (d, 1H), 7,80 (d, 1H), 12,44 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 41. | cyclohexyl | OH, H (R) | $CH_3(CH_2)_2$-(S) | $-COR_6$ | $(CH_3)_2CH-$ | $OCH_3$ | - | insolub le MeOH | 0,85 (t,3H), 1,25 (d,6H), 1,05-1,67 (m, 15H), 3,70 (d, 1H), 3,78 (s, 3H), 3,96 (m, 1H), 4,45 (m, 1H), 5,34 (d, 1H), 7,82 (d, 1H), 12,50 (s, 1H)*** |
| 42. | 3,5-difluorophényle | OH, H (S) | $CH_3(CH2)_3$-(S) | $-COR_6$ | $(CH_3)_2CH-$ | $OCH_3$ | - | -49° | 0,78 (t,3H), 1,18 (m,4H), 1,27 (d,6H), 1,71 (m,1 H), 3,78 (s,3H), 3,97 (m,1 H), 4,42 (m,1H), 5,05 (m,1H), 6,56 (s,1H), 7,13 (m,3H), 8,23 (d,1 H), 12,45 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 43. | 3,5-difluorophényle | OH, H (S) | $CH_3(CH_2)_2$-(S) | $-COR_6$ | 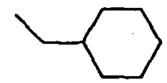 | $OCH_3$ | - | - | 0,80 (t,3H), 0,95 (m,2H), 1,13-1,69 (m, 13H), 2,99 (d, 2H), 3,77 (s, 3H), 4,42 (m, 1H), 5,05 (s, 1H), 6,55 (s,1 H), 7,11 (m, 3H), 8,24 (d, 1H), 12,51 (s, 1H)*** |
| 44. | 3,5-difluorophényle | OH, H (S) | $CH_3(CH_2)_2$-(S) | $-COR_6$ | $CH_3CH_2$- | $OCH_3$ | - | - 55° | 0,80 (t,3H), 1,19 (d,3H), 1,20 (m, 1H), 1,69 (m,2H), 3,09 (m,2H), 3,78 (s,3H), 4,41 (m, 1H), 5,05 (s,1H), 6,55 (s,1H), 7,12 (m,3H), 8,26 (d, 1H), 12,45 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 45. | 3,5-difluorophényle | OH, H (R) | $CH_3(CH_2)_2$-(S) | -COR6 | $CH_3CH_2$- | $OCH_3$ | - | - | 0,83 (t,3H), 1,22 (t,3H), 1,28 (m, 1H), 1,69 (m,2H), 3,09 (m,2H), 3,78 (s,3H), 4,40 (m,1H), 5,07 (s,1H), 6,42 (s,1H), 7,12 (m,3H), 8,29 (d,1H), 12,51 (s,1H)*** |
| 46. | 3,5-difluorophényle | H, H | $CH_3(CH_2)_2$-(S) | $-COR_6$ | $(CH_3)_2CH$- | $NR_7R_8$ | $CH_3CH_2$, $CH_3CH_2$ | - | 0,85 (t,3H), 1,03-1,11 (m, 6H), 1,20 (d, 6H), 1,20-1,32 (m,2H), 1,60 (m,2H), 3,18 (m,3H), 3,39 (m,2H), 3,55 (s, 2H), 4,42 (m, 1H), 6,98 (d, 2H), 7,07 (m,1 H), 8,49 (d,1 H), 12,21 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 47. | 3,5-difluorophényle | H, H | $CH_3(CH_2)_2$-(S) | $-COR_6$ | $(CH_3)_2CH-$ | $NR_7R_8$ | 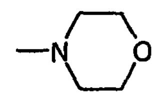 | - 66° | 0,85 (t,3H), 1,21 (d,6H), 1,34-1,22 (m, 2H), 1,64 (m, 2H), 3,26 (m, 1H), 3,27 (s, 2H), 3,50 (s, 2H), 3,51 (s, 2H), 3,57 (s, 4H), 4,41 (m, 1H), 6,97 (d, 2H), 7,08 (m, 1H), 8,49 (d, 1H), 12,25 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 48. | 3,5-difluorophényle | H, H | $CH_3(CH_2)_2$-(S) | $-COR_6$ | $(CH_3)_2CH-$ | $NR_7R_8$ | $CH_3$, H | - | 0,86 (t,3H), 1,22 (d,6H), 1,35 (m,2H), 1,63 (m,2H), 2,75 (s,3H), 3,58 (d,2H), 4,18 (m,1H), 4,47 (m,1H), 6,98 (d,2H), 7,07 (m,1H), 7,69 (s,1H), 8,50 (s,1H), 12,13 (s, 1H)*** |
| 49. | 3,5-difluorophényle | H, H | $CH_3(CH_2)_2$-(S) | $-COR_6$ | $(CH_3)_2CH-$ | $NR_7R_8$ | 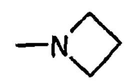 | - | 0,86 (t,3H), 1,21 (d,6H), 1,34 (m,2H), 1,62 (m,2H), 2,20 (m,2H), 3,54 (s,2H), 3,97 (m,2H), 4,38 (m,2H), 4,47 (m,1H), 6,97 (d,2H), 7,08 (m,1H), 8,52 (d,1H), 12,08 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 50. | 3,5-difluorophényle | H, H | $CH_3(CH_2)_2$-(S) | $-COR_6$ | phényle | $OCH_3$ | - | -112° | 0,87 (t,3H), 1,30 (m,2H), 1,67 (m,2H), 3,56 (s,2H), 3,67 (s,3H), 4,43 (m,1H), 6,98 (d,2H), 7,06 (m,1H), 7,44 (m,5H), 8,56 (d,1H), 12,71 (s,1H)*** |
| 51. | 3,5-difluorophényle | H, H | $CH_3CH_2$- (S) | $-COR_6$ | $(CH_3)_2CH$- | $OCH_3$ | - | -73° | 0,79 (t,3H), 1,26 (d,6H), 1,73 (m,2H), 3,78 (s,3H), 3,96 (m,1 H), 4,36 (m,1 H), 5,06 (s,1 H), 6,56 (s,1 H), 7,13 (m,3H), 8,22 (d, 1H), 12,46 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 52. | phényle | $-OCH_3$, H(S) | $CH_3(CH_2)_2$-(S) | $-COR_6$ | $(CH3)_2CH-$ | $OCH_3$ | - | - 37° | 0,86 (t,3H), 1,25 (m,2H), 1,30 (d,6H), 1,75 (m,2H), 3,38 (s,3H), 3,79 (s, 3H), 4,00 (m,1 H), 4,47 (m, 1H), 4,75 (s, 1H), 7,33 (m,5H), 8,22 (d, 1H), 12,50 (s, 1H)*** |
| 53. | phényle | $-OCH_3$, H(R) | $CH_3(CH_2)_2$-(S) | $-COR_6$ | $(CH_3)_2CH-$ | $OCH_3$ | - | -113° | 0,85 (t,3H), 1,27 (d, 6H), 1,26 (m,2H), 1,70 (m,2H), 3,30 (s,3H), 3,80 (s,3H), 3,98 (m,1 H), 4,41 (m,1H), 4,77 (s,1 H), 7,30 (m,5H), 8,30 (d,1H), 12,50 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 54. | phényle | OH, $CH_3$ (S) | $CH_3$ $(CH_2)_2$-(S) | $-COR_6$ | $(CH_3)_2CH-$ | $OCH_3$ | - | - 66° | 0,77 (t,3H), 1,10-1,17 (m, 2H), 1,35 (d, 6H), 1,68 (s, 3H), 3,85 (s, 3H), 4,05 (m,1 H), 4,45 (m, 1H), 6,30 (s, 1H), 7,25-7,50 (m,5H), 8,00 (d,1H), 12,47 (s,1H)*** |
| 55. | phényle | OH, $CH_3$ (R) | $CH_3$ $(CH_2)_2$-(S) | $-COR_6$ | $(CH_3)_2CH-$ | $OCH_3$ | - | - 61° | 0,90 (t,3H), 1,28 (d,6H), 1,30 (m,2H), 1,65 (s,3H), 1,71 (m,2H), 3,83 (s,3H), 4,00 (m,1 H), 6,28 (s,1 H), 7,22-7,50 (m, 5H), 8,00 (d, 1H), 12,50 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 56. | 3,5-difluorophényle | H, H | $CH_3(CH_2)_2$-(S) | $-COR_6$ | $(CH_3)_2CH-$ | $NR_7R_8$ | $(CH_3)_2CH-$. $CH_3$ | - | 0,86 (t,3H), 1,07 (m,6H), 1,11 (m,6H), 1,21 (m,2H), 1,62 (m,2H), 2,66 (S,3H), 2,81 (s,3H), 3,27 (m, 1 H), 3,54 (s,2H), 3,77 (m,1 H), 4,41 (m,1 H), 6,97 (d,2H), 7,07 (m,1H), 8,48 (d,1 H), 12,20 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 57. | 3,5-difluorophényle | OH, H | $CH_3(CH_2)_2$-(S) | $-COR_6$ | $(CH_3)_2CH$- | $NR_7R_8$ | $CH_3$, $CH_3$ | -119° | 0,72 (t,3H), 1,06 (m,1H), 1,16(m,1H), 1,27 (d,6H), 1,58 (m,2H), 2,10 (s,3H), 3,78 (s,3H), 3,96 (m,1 H), 4,37 (m,1 H), 5,97 (s,1 H), 7,34 et 7,47 (2m,5H), 8,62 (s,1 H), 12,49 (s,1 H)*** |
| 58. | 3,5-difluorophényle | H, H | $CH_3(CH_2)_2$-(S) | $-COR_6$ | $(CH_3)_2CH$- | $NR_7R_8$ | $CH_3$, $OCH_3$ | - | 0,86 (t,3H), 1,23 (s,6H), 1,28-1,35 (m, 2H), 1,64 (m, 2H), 3,20 (s, 3H), 3,30 (m, 1H), 3,52 (s, 3H), 4,42 (m, 1H), 6,98 (d, 2H), 7,08 (m, 1H), 8,49 (d, 1H), 12,23 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 59. | 3,5-difluorophényle | H, H | $CH_3OCH_2$-(S) | $-COR_6$ | $(CH_3)_2CH-$ | $OCH_3$ | - | - | 1,26 (t,3H), 3,28 (d,3H), 3,55 (s,2H), 3,57 (m,2H), 3,78 (s,3H), 3,97 (m,1 H), 4,64 (m,1H), 6,98 (d,2H), 7,08 (m,1H), 8,57 (d,1H), 12,54 (s,1 H)*** |
| 60. | 3,5-difluorophényle | OH, H (S) | $CH_3OCH_2$-(S) | $-COR_6$ | $(CH_3)_2CH-$ | $OCH_3$ | - | - | 1,25 (d,6H), 3,23 (s,2H), 3,59 (m,1 H), 3,61 (m,1H), 3,78 (s,3H), 3,96 (m,1H), 4,64 (m,1H), 5,09 (s,1H), 6,62 (s,1 H), 7,13 (m,3H), 8,23 (d,1H), 12,53 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 61. | 3,5-difluorophényle | OH, H (R) | $CH_3OCH_2$-(S) | $-COR_6$ | $(CH_3)_2CH-$ | $OCH_3$ | - | - | 1,26 (t,3H), 3,23 (s,2H), 3,62 (m,1 H), 3,71 (m, 1H), 3,78 (s,3H), 3,97 (m,1H), 4,00 (m,1H), 5,10 (s,1H), 6,56 (s,1H), 7,13 (m,3H), 8,26 (d,1 H), 12,59 (s,1H)*** |
| 62. | 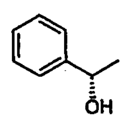 | H, H | $CH_3(CH_2)_2$-(S) | $-COR_6$ | $(CH_3)_2CH-$ | $OCH_3$ | - | - | 0,86 (t,3H), 1,27 (d,6H), 1,28 (m,2H), 1,62 (m,2H), 3,78 (s,3H), 3,97 (m, 1H), 4,41 (m, 1H), 4,94 (m, 1H), 5,33 (s, 1H), 7,21 (m, 5H), 8,13 (d, 1H), 12,41 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 63. | 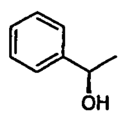 | H, H | $CH_3(CH_2)_2$-(S) | $-COR_6$ | $(CH_3)_2CH-$ | $OCH_3$ | - | - | 0,79 (t,3H), 1,12 (m,2H), 1,27 (d,6H), 1,51 (m,2H), 3,78 (s,3H), 3,96 (m,1 H), 4,37 (m,1 H), 4,91 (m,1 H), 5,34 (s,1 H), 7,30 (m,5H), 8,09 (d,1 H), 12,38 (s,1 H)*** |
| 64. | 3,5-difluorophényle | H, H | $CH_3(CH_2)_2$-(S) | $-COR_6$ | $(CH_3)_2CH-$ | $NR_7R_8$ | $CH_3CH_2$-, $CH_3$ | - | 0,86 (t,3H), 1,05 (m,3H), 1,07 (d,6H), 1,08 (m,2H), 1,21 (m,2H), 2,82 et 2,92 (2s,3H), 3,20 (m,2H), 3,30 (m, 1H), 3,55 (s,2H), 4,41 (m, 1H), 6,98 (d, 2H), 7,09 (m, 1H), 8,49 (m, 1H), 12,19 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 65. | phényle | H, H | $CH_3(CH_2)_2$-(S) | $-COR_6$ | $(CH_3)_2CH-$ | $OCH_3$ | - | - | 0,85 (t,3H), 1,26 (d,6H), 1,34 (m,2H), 3,46 (q,2H), 3,78 (s,3H), 3,97 (m,1H), 4,36 (m,1 H), 7,24 (m,5H), 8,38 (d,1 H), 12,47 (s,1H)*** |
| 66. | 2,3-difluorophényle | OH, H | $CH_3(CH_2)_2$-(S) | $-COR_6$ | $(CH_3)_2CH-$ | $OCH_3$ | - | -71° | 0,86 (m,3H), 1,26 (d,6H), 1,28 (m,2H), 1,72 (m,2H), 3,78 (s,3H), 3,96 (m,1 H), 4,47 (m,1H), 5,24 et 5,28 (2d,1 H), 6,55 et 6,62 (2d,1H), 7,17-7,36 (m, 3H), 8,19 et 8,23 (dd,1H), 12,49 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 67. | 2,5-difluorophényle | OH, H | $CH_3(CH_2)_2$-(S) | $-COR_6$ | $(CH_3)_2CH-$ | $OCH_3$ | - | -68° | 0,85 (m,3H), 1,26 (d,6H), 1,30 (m,2H), 1,72 (m,2H), 3,78 (s,3H), 3,97 (m,1 H), 4,45 (m,1H), 5,20 et 5,23 (2s,1H), 6,50 et 6,57 (2s,1H), 7,18 (m,3H), 8,19 et 8,25 (dd,1H), 12,48 (s,1H)*** |
| 68. | phényle | 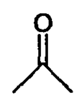 | $CH_3(CH_2)_2$-(S) | $-COR_6$ | $(CH_3)_2CH-$ | $OCH_3$ | - | -40° | 0,97 (t,3H), 1,20 (d,6H), 1,40 (m,2H), 1,80 (m,2H), 3,87 (s,3H), 4,05 (m,1 H), 4,60 (m,1 H), 7,60 (m,2H), 7,80 (m,1 H), 7,99 (m,2H), 9,30 (d,1 H), 12,70 (s,1 H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 69. | 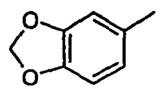 | OH, H | CH3 $(CH_2)_2$-(S) | $-COR_6$ | $(CH_3)_2CH-$ | $OCH_3$ | - | -66° | 0,86 (t,3H), 1,30 (d,6H), 1,25 (m,2H), 1,72 (m,2H), 3,81 (s,3H), 4,00 (m,1 H), 4,45 (m, 1H), 4,90 et 4,94 (dd,1H), 5,99 (m,2H), 6,13 et 6,26 (dd,1H), 6,86 (m,3H), 8,14 (m, 1H), 12,47 et 12,52 (d,1 H)*** |
| 70. | phényle | $-OC(O)CH_3$, H (S) | $CH_3$ $(CH_2)_2$-(S) | $-COR_6$ | $(CH_3)_2CH-$ | $OCH_3$ | - | - | 0,86 (t,3H), 1,24 (d,6H), 1,26 (m,2H), 1,63 (m,2H), 2,11 (s,3H), 3,77 (s,3H), 3,95 (m, 1H), 4,45 (m, 1H), 5,94 (s, 1H), 7,38 (m,5H), 8,55 (d,1 H), 12,48 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 71. | phényle | $-OC(O)CH_3$, H (R) | $CH_3(CH_2)_2$-(*S*) | $-COR_6$ | $(CH_3)_2CH-$ | $OCH_3$ | - | - | 0,81 (t,3H), 1,21 (d,6H), 1,22 (m,2H), 1,67 (m,2H), 2,86 (s, 3H), 2,95 (s,3H), 3,22 (m, 1H), 4,44 (m, 1H), 5,06 (m, 1H), 6,54 et 6,41 (2s, 1H), 7,13 (m,3H), 8,26 (d,1 H), 12,19 (s, 1H)*** |
| 72. | 3,5-difluorophényle | OH, H (*S*) | $CH_3CH_2$- (*S*) | $-COR_6$ | $(CH_3)_2CH-$ | $OCH_3$ | - | - | 0,79 (t,3H), 1,25 (d,6H), 3,78 (s,3H), 3,96 (m, 1H), 4,36 (m, 1H), 5,06 (s, 1H), 6,56 (s, 1H), 7,13 (m,3H), 8,22 (d, 1H), 12,46 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 73. | 3,5-difluorophényle | OH, H(R) | $CH_3CH_2$-(*S*) | -$COR_6$ | $(CH_3)_2CH$- | $OCH_3$ | - | - | 0,83 (t,3H), 1,26 (d,6H), 1,73 (m,2H), 3,78 (s,3H), 3,95 (m, 1H), 4,32 (m,1H), 5,08 (s,1H), 6,44 (s,1H), 7,12 (m,3H), 8,26 (d,1H), 12,50 (s,1H)*** |
| 74. | 3,5-difluorophényle | H, H | $CH_3(CH_2)_2$-(S) | -$COR_6$ | $(CH_3CH_2)_2CH$- | $OCH_3$ | - | - | 0,77 (m,6H), 0,83 (t,3H), 1,43-1,75 (m, 8H), 3,53 (m, 2H), 3,70 (m, 1H), 3,77 (s, 3H), 4,36 (m, 1H), 6,98 (d, 2H), 7,08 (m, 1H), 8,46 (d, 1H), 12,51 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 75. | 3,5-difluorophényle | 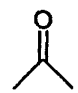 | $CH_3(CH_2)_2$-(S) | $-COR_6$ | $(CH_3)_2CH$- | $OCH_3$ | - | - | 0,89 (t,3H), 1,28 (d,6H), 1,33 (m,2H), 1,77 (m,2H), 3,78 (s,3H), 3,98 (m,1H), 4,56 (m,1 H), 7,70 (m,3H), 9,36 (d,1H), 12,62 (s,1H)*** |
| 76. | CH3S(CH2)2- | OH, H | $CH_3$ $(CH_2)_2$-(S) | $-COR_6$ | $(CH_3)_2CH$- | $OCH_3$ | - | - | 0,93 (t,3H), 1,38 (d,6H), 1,39 (m,2H), 1,74-1,90 (m, 2H), 2,10 (s, 3H), 2,56 (s, 2H), 3,85 (s, 3H), 4,04 (m, 1H), 4,08 (m, 1H), 4,51 (m,1 H), 5,73 (d,1H), 7,97 (d,1 H), 12,50 (S,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 77. | (CH3)2CHCH2- | OH, H (S) | $CH_3(CH_2)_2$-(S) | $-COR_6$ | $(CH_3)_2CH-$ | $OCH_3$ | - | - | 0,87 (m,9H), 1,27 (d,6H), 1,26-1,39 (m, 4H), 1,67 (m, 3H), 3,78 (s, 3H), 3,90 (m, 2H), 4,45 (m,1 H), 5,49 (d,1 H), 7,85 (d,1 H), 12,43 (s, 1H)*** |
| 78. | 3,5-difluorophényle | OH, H (S) | $CH_3(CH_2)_2$-(S) | $-COR_6$ | $(CH_3CH_2)_2CH-$ | $OCH_3$ | - | -61° | 0,80 (m,9H), 1,25 (m,2H), 1,50 (m,2H), 1,70 (m,4H), 3,73 (m,1 H), 3,80 (s,3H), 4,45 (m,1H), 5,05 (s,1H), 6,55 (s,1H), 7,15 (m,3H), 8,25 (d,1 H), 12,45 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 79. | 3,5-difluorophényle | OH,H(R) | $CH_3(CH_2)_2$-(S) | $-COR_6$ | $(CH_3CH_2)_2CH-$ | $OCH_3$ | - | - | 0,80 (m,6H), 0,85 (m,3H), 1,30 (m,2H), 1,50 (m,2H), 1,75 (m,4H), 3,75 (m, 1H), 3,85 (s,3H), 4,45 (m, 1H), 5,11 (s,1H), 6,45 (s,1H), 7,18 (m,3H), 8,31 (d,1H), 12,60 (s,1H)*** |
| 80. | 3,5-difluorophényle | H, H | $CH_3(CH_2)_2$-(S) | $-COR_6$ | 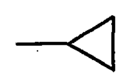 | $OCH_3$ | - | - 96° | 0,78 (d,2H), 0,88 (t,3H), 1,20 (d,2H), 1,25-1,40 (m, 2H), 1,68 (m, 2H), 2,90 (m, 1H), 3;55 (m, 2H), 3,82 (s; 3H), 4,38 (m, 1H), 7,00-7,15 (m,3H), 8,50 (d,1 H), 12,48 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 81. | 3,5-difluorophényle | OH, H (S) | $CH_3(CH_2)_2$-(S) | $-COR_6$ | 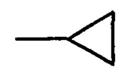 | $OCH_3$ | - | - 56° | 0,75 (m,2H), 0,82 (t,3H), 1,22 (m,2H), 1,70 (m,2H), 2,90 (m,1 H), 3,80 (s,3H), 4,42 (m,1 H), 5,08 (s,1H), 6,48 (s,1H), 7,13 (m,3H), 8,25 (d,1H), 12,48 (s,1H)*** |
| 82. | 3,5-difluorophényle | OH, H (R) | $CH_3(CH_2)$-(S) | $-COR_6$ | 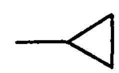 | $OCH_3$ | - | -84° | 0,78 (m,2H), 0,90 (t,3H), 1,20 (m,2H), 1,30 (m,2H), 1,70 (m,2H), 2,90 (m,1H), 3,85 (s,3H), 4,40 (m,1H), 5,10 (s,1H), 6,42 (s, 1H), 7,18 (m,3H), 8,30 (d,1H), 12,55 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 83. | phényle | $CH_3CH_2$-, H (S) | $CH_3(CH_2)_2$-(S) | -$COR_6$ | $(CH_3)_2CH$- | $OCH_3$ | - | -25° | 0,80 (t,3H), 0,86 (t,3H), 1,26 (d,6H), 1,30-1,40 (m, 2H), 1,65 (m, 2H), 3,50 (m,1 H), 3,80 (s,3H), 3,95 (m,1 H), 4,45 (m,1H), 7,20-7,30 (m, 5H), 8,30 (d, 1H), 12,45 (s, 1H)*** |
| 84. | phényle | $CH_3CH_2$-, H (R) | $CH_3(CH_2)_2$-(S) | -$COR_6$ | $(CH_3)_2CH$- | $OCH_3$ | - | | 0,80 (t,3H), 0,85 (t,3H), 1,18-1,30 (m, 2H), 1,30 (d, 6H), 1,60 (m, 2H) 1,95 (m, 2H), 3,50 (m,1 H), 3,80 (s,3H), 4,00 (m,1 H), 4,30 (m,2H), 7,20-7,30 (m, 5H), 8,32 (d, 1H), 12,45 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 85. | phényle | $CH_3$, H (S) | $CH_3$ $(CH_2)_2$-(S) | $-COR_6$ | $(CH_3)_2CH-$ | $OCH_3$ | - | -17° | 0,91 (t,3H), 1,30 (d,6H), 1,38 (d,3H), 1,30-1,45 (m, 2H), 1,68 (m, 2H), 3,80 (m, 1H), 3,85 (s, 3H), 4,00 (m, 1H), 4,48 (m, 1H), 7,21-7,30 (m,5H), 8,30 (d,2H), 12,48 (s,1 H)*** |
| 86. | phényle | $CH_3$, H(R) | $CH_3$ $(CH_2)_2$-(S) | $-COR_6$ | $(CH_3)_2CH-$ | $OCH_3$ | - | - | 0,78 (t,3H), 1,14-1,25 (m, 2H), 1,30 (m, 6H), 1,57 (m, 2H), 3,72 (m, 1H), 3,75 (s, 3H), 3,98 (m,1 H), 4,31 (m, 1H), 7,24-7,40 (m,5H), 8,26 (d,1H), 12,45 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 87. | phényle | 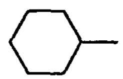 , H | $CH_3(CH_2)_2$-(S) | $-COR_6$ | $(CH_3)_2CH-$ | $OCH_3$ | - | - | 0,78 et 0,90 (2t, 3H), 0,60-2,00 (m,15H), 1,25 et 1,35 (2d,6H), 3,30 (m,1 H), 3,80 (2s,3H), 4,00 (m,1 H), 4,25 et 4,45 (2m,1 H), 7,20-7,35 (m, 5H), 8,30 (m,1 H), 12,45 (m,1 H)*** |
| 88. | 3,5-difluorophényle | OH, H (S) | $CH_3(CH_2)_2$-(S) | $-COR_6$ | $(CH_3)_2CH-$ | $NR_7R_8$, | $CH_3$, $CH_3$ | - | 0,80 (t,3H), 1,25 (d,6H), 1,28 (m,2H), 1,75 (m,2H), 2,80 (s,3H), 3,00 (s,3H), 3,25 (m,1H). 4,49 (m,1 H), 5,08 (s,1H), 6,55 (s large, 1H), 7,13 (d, 3H), 8,25 (d, 1H), 12,15 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 89. | 3,5-difluorophényle | OH, H (R) | $CH_3(CH_2)_2$-(S) | -$COR_6$ | $(CH_3)_2CH$- | $NR_7R_8$ | $CH_3$, $CH_3$ | -64° | 0,88 (t,3H), 1,22 (d,6H), 1,30 (m,2H), 1,68 (m,2H), 2,87 (s,3H), 2,98 (s,3H), 3,25 (m,1H), 4,45 (m,1H), 5,10 (s,1H), 6,42 (s,1H), 7,18 (m,5H), 8,30 (d,1H), 12,20 (s,1H)*** |
| 90. | 3,5-difluorophényle | H, H | $CH_3(CH_2)_2$-(S) | -$COR_6$ | H | $OCH_2CH_3$ | - | - | 0,89 (t,3H), 1,28 (t,3H), 1,35 (m,2H), 1,65 (m,2H), 3,58 (m,2H), 4,28 (m,2H), 4,40 (m,1H), 6,98 (d,2H), 7,08 (t,1H), 8,05 (s,1H), 8,50 (d,1 H), 12,67 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 91. | cyclohexyl | H, H | $CH_3(CH_2)_2$-(S) | $-COR_6$ | $(CH_3)_2CH-$ | $OCH_3$ | - | - | 0,85 (t,3H), 0,90 (m,3H), 1,30 (m,2H), 1,30 (d,6H), 1,60 (m,9H), 2,00 (m,2H), 3,80 (s,3H), 3,98 (m,1H), 4,36 (m,1H), 8,08 (d,1H), 12,40 (s,1H)*** |
| 92. | 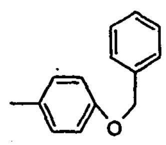 | H, H | $CH_3(CH_2)_2$-(S) | $-COR_6$ | $(CH_3)_2CH-$ | $OCH_3$ | - | - | 0,85 (t,3H), 1,28 (d,6H), 1,30 (m,2H), 1,60 (m,1H), 3,60 (m,2H), 3,80 (s,1H), 4,00 (m,1H), 5,10 (s,2H), 6,86-7,46 (m, 9H), 8,18 (d, 1H), 12,42 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 93. | 2-hydroxyphényle | H, H | $CH_3(CH_2)_2$-(S) | $-COR_6$ | $(CH_3)_2CH-$ | $OCH_3$ | - | - | 0,88 (t,3H), 1,28 (d,6H), 1,26 (m,2H), 1,75 (m,2H), 3,50 (m,2H), 3,80 (s,3H), 4,00 (m,1H), 4,40 (m,1H), 6,70-7,10 (m, 4H), 8,25 (s,1 H), 9,55 (s,1 H), 12,45 (s,1 H)*** |
| 94. | 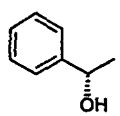 | OH, H (S) | $CH_3(CH_2)_2$-(S) | $-COR_6$ | $(CH_3)_2CH-$ | $OCH_3$ | - | - 74° | 0,89 (t,3H), 1,29 (d,6H), 1,32 (m,2H), 1,67 (m,2H), 3,80 (s,3H), 4,00 (m,2H), 4,55 (m,1 H), 4,92 (d,1 H), 5,51 (d,1 H), 7,20-7,40 (m, 5H), 7,80 (d,1 H), 12,50 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 95. | 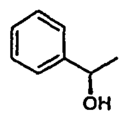 | OH, H (R) | $CH_3(CH_2)_2$-(S) | $-COR_6$ | $(CH_3)_2CH$- | $OCH_3$ | - | +38° | 0,90 (t,3H), 1,26 (m,2H), 1,38 (d,6H), 1,69 (m,2H), 3,86 (s,3H), 4,05 (m,1 H), 4,08 (m,1 H), 4,53 (m,1 H), 4,92 (m,1 H), 5,33 (m,1 H), 5,41 (d,1H), 7,25-7,45 (m, 5H), 8,05 (d,1 H), 12,50 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 96. | $Ph(CH_2)_2$- | OH, H(R) | $CH_3(CH_2)_2$-(S) | -$COR_6$ | $(CH_3)_2CH$- | $OCH_3$ | - | -8° | 0,86 (t,3H), 1,25 (d,6H), 1,28 (m,2H), 1,68 (m,2H), 1,74 (m,2H), 2,51 (m,2H), 3,78 (s,3H), 3,91 (m,1 H), 3,97 (m,1 H), 4,43 (m,1H), 5,65 (s,1H), 7,13-7,26 (m, 5H), 7,91 (d, 1H), 12,47 (s, 1H)*** |
| 97. | $(CH_3)_3C$- | OH,H(R) | $CH_3(CH_2)_2$-(S) | -$COR_6$ | $(CH_3)_2CH$- | $OCH_3$ | - | - | 0,90 (t,3H), 0,93 (s,9H), 1,30 (d,6H), 1,32 (m,2H), 1,71 (m,2H), 3,58 (d,1H), 3,82 (s,3H), 4,02 (m,1 H), 4,52 (m,1 H), 5,60 (d,1 H), 7,82 (d,1H), 12,45 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 98. | phényle | F, H (S) | $CH_3(CH_2)_2$-(S) | $-COR_6$ | $(CH_3)_2CH-$ | $OCH_3$ | - | - | 0,87 (t,3H), 1,22 (d,6H), 1,32 (m,2H), 1,70 (m,2H), 3,78 (s,3H), 3,98 (m,1 H), 4,52 (m,1H), 5,90 et 6,00 (2s,1H), 7,44 (m,5H), 8,70 (d,1 H), 12,55 (s,1H)*** |
| 99. | phényle | F, H (R) | $CH_3(CH_2)_2$-(S) | $-COR_6$ | $(CH_3)_2CH-$ | $OCH_3$ | - | - | 0,90 (t,3H), 1,24 (m,2H), 1,30 (s,6H), 1,77 (m,2H), 3,85 (s,3H), 4,05 (m,1 H), 4,48 (m,1 H), 5,97 et 6,05 (2s, 1H), 7,50 (m, 5H), 8,80 (d, 1H), 12,62 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 100. | $(CH_3)_2CH-$ | OH, H (S) | $CH_3(CH_2)_2$-(S) | $-COR_6$ | $(CH_3)_2CH-$ | $OCH_3$ | - | - | 0,77 (d,3H), 0,85 (t,3H), 0,95 (d,3H), 1,26 (d,6H), 1,32 (m,2H), 1,70 (m,2H), 1,95 (m,1H), 3,72 (m,1H), 3,78 (s,3H), 3,98 (m,1H). 4,50 (m,1 H), 5,45 (s,1 H), 7,80 (d,1H), 12,45 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 101. | 3,5-difluorophényle | H, H | $CH_3(CH_2)_2$-(S) | $-COR_6$ | 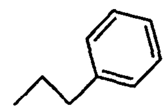 | $OCH_3$ | - | - | 0,92 (t,3H), 1,36 (m,2H), 1,71 (m,2H), 2,96 (m,2H), 3,44 (m,2H), 3,61 (s,1H), 3,84 (s,3H), 4,47 (m,1H), 7,12 (d,2H), 7,16 (t,1H), 7,23-7,36 (m, 5H), 8,53 (d, 1H), 12,55 (s,1 H)*** |
| 102. | 3,5-difluorophényle | 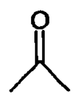 | $CH_3(CH_2)_2$-(S) | $-COR_6$ | 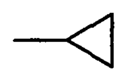 | $OCH_3$ | - | - | 0,81 (m,2H), 0,97 (t,3H), 1,25 (m,2H), 1,39 (m,2H), 1,83 (m,2H), 2,95 (m,1H). 3,86 (s,3H), 4,61 (m,1H), 7,78 (m,3H), 9,40 (d,1H), 12,68 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 103. | 3,5-difluorophényle | H, H | $CH_3(CH_2)_2$-(S) | $-COR_6$ | 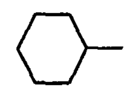 | $OCH_3$ | - | - | 0,92 (t,3H), 1,34-1,43 (m, 7H), 1,69-1,84 (m,5H), 2,00 (m,2H), 3,61 (d,2H), 3,69 (m,1H), 3,84 (s, 3H), 4,47 (m, 1H), 7,06 (d, 2H), 7,25 (t, 1H), 8,55 (d, 1H), 12,55 (s,1 H)*** |
| 104. | 3,5-difluorophényle | H, H | $CH_3(CH_2)_2$-(S) | $-COR_6$ | $(CH_3)_3CCH_2$- | $OCH_3$ | - | - | 0,86 (t,3H), 0,91 (s,9H), 1,34 (m,2H), 1,66 (m,2H), 3,12 (m,2H), 3,58 (m,2H), 3,76 (s,3H), 4,40 (m,1H), 7,02 (d,2H), 7,09 (t,1H), 8,49 (d,1H), 12,50 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 105. | 3,5-difluorophényle | 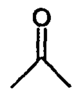 | $CH_3(CH_2)_2$-(S) | $-COR_6$ | $(CH_3)_2CH-$ | $NR_7R_8$ | $CH_3$, $CH_3$ | - | 0,89 (t,3H), 1,23 (d,6H), 1,37 (m,2H), 1,73 (m,2H), 2,88 (s,3H), 2,95 (s,3H), 3,24 (m,1H), 4,58 (m,1H), 7,72 (m,3H), 9,36 (d,1H), 12,36 (s,1H)*** |
| 106. | $(CH_3)_3C-$ | OH, H | $CH_3(CH_2)_2$-(S) | $-COR_6$ | $(CH_3)_2CH-$ | $NR_7R_8$ | $CH_3$, $CH_3$ | - | 0,87 (t,3H), 0,90 (s,9H), 1,25 (d,6H), 1,33 (m,2H), 1,68 (m,2H), 2,89 (s,3H), 2,97 (s,3H), 3,57 (s,1 H), 4,54 (m,1 H), 5,56 (s,1 H), 7,84 (d,1 H), 12,15 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 107. | 3-chlorophényle | OH, H (S) | $CH_3(CH_2)_2$-(S) | $-COR_6$ | $(CH_3)_2CH$- | $OCH_3$ | - | - | 0,87 (t,3H), 1,20 (m,2H), 1,22 (d,6H), 1,70 (m,2H), 3,80 (s,3H), 4,00 (m, 1H), 4,43 (m,1 H), 5,02 (s,1 H), 6,44 (s,1H), 7,30 (m, 3H), 7,45 (s, 1H), 8,20 (d, 1H), 12,45 (s,1H)*** |
| 108. | $(CH_3CH_2)_2CH$- | OH, H (S) | $CH_3(CH_2)_2$-(S) | $-COR_6$ | $(CH_3)_2CH$- | $OCH_3$ | - | - | - 0,86 (t,3H), 0,93 (m,6H), 1,24 (m,2H), 1,32 (d,6H), 1,38 (m,4H), 1,55 (m,1H), 1,72 (m,2H), 3,78 (s,3H), 3,98 (m,1 H), 4,05 (m, 1H), 4,55 (m, 1H), 5,55 (d, 1H), 7,92 (d,1H), 12,50 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 109. | $(CH_3CH_2)_2CH-$ | OH, H(R) | $CH_3(CH_2)_2$-(S) | $-COR_6$ | $(CH_3)_2CH-$ | $OCH_3$ | - | - | 0,80 (t,3H), 0,90 (m,6H), 1,20 (m,2H), 1,30 (d,6H), 1,34 (m,4H), 1,55 (m,1H), 1,74 (m,2H), 3,86 (s,3H), 4,00 (m,2H), 4,53 (m,1H), 5,42 (d,1H), 8,00 (d,1 H), 12,54 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 110. | 3,5-difluorophényle | OH, H | $CH_3(CH_2)_2$-(S) | $-COR_6$ | $(CH_3)_2CH-$ | $OCH_3$ | - | - | 0,82 (m,3H), 1,10 (m,2H), 1,27 (m,6H), 1,61 (m,2H), 2,83 et 2,95 (2m,1 H), 3,77 (s,3H), 3,96 (m, 1 H), 4,21 (m, 1H), 4,43 (m,1 H), 5,68 et 5,81 (2d, 1H), 6,93-7,01 (m, 3H), 7,84 et 7,97 (2d,1H), 12,46 (s,1H)*** |
| 111. | 3,5-difluorophényle | H, H | $CH_3(CH_2)_2$-(S) | $-COR_6$ | 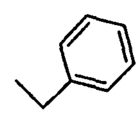 | $OCH_3$ | - | - | 0,87 (t,3H), 1,32 (m,2H), 1,61 (m,2H), 3,55 (m,2H), 3,83 (s,3H), 4,39 (m,1H), 4,61 (s,2H), 6,98 (d,2H), 7,10 (t,1H), 7,30 (m,5H), 8,49 (d,1H), 12,55 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 112. | 3,5-difluorophényle | OH, H (S) | $CH_3(CH_2)_2$-(S) | $-COR_6$ | 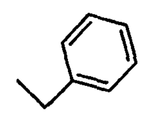 | $OCH_3$ | - | - | 0,81 (t,3H), 1,22 (m,2H), 1,68 (m,2H), 3,83 (s,3H), 4,40 (m,1 H), 4,44 (s,2H), 5,06 (s,1 H), 6,56 (s,1H), 7,15-7,24 (m, 3H), 7,32 (m, 5H), 8,24 (d, 1H), 12,52 (s, 1H)*** |
| 113. | 3,5-difluorophényle | OH, H (R) | $CH_3(CH_2)_2$-(S) | $-COR_6$ | 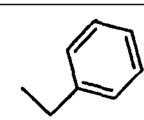 | $OCH_3$ | - | - | 0,83 (t,3H), 1,24 (m,2H), 1,69 (m,2H), 3,82 (s,3H), 4,37 (m,1 H), 4,48 (s,2H), 5,07 (s,1H), 6,55 (s,1H), 7,13 (m,3H), 7,33 (m,5H), 8,26 (d,1 H), 12,59 (s,1 1H) *** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 114. | 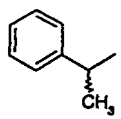 | oH, H | $CH_3$ $(CH_2)_2$-(S) | $-COR_6$ | $(CH_3)_2CH-$ | $OCH_3$ | - | - | 0,70-0,80 (m, 3H), 1,12-1,20 (m,3H), 1,20-1,35 (m, 2H), 1,30 (m, 6H), 1,60 (m, 2H), 3,15 (m, 1H), 3,80 (s, 3H), 4,05 (m, 2H), 4,40 (m, 1H), 5,43, 5,55 et 5,80 (3d,1H), 6,95-7,30 (m, 5H), 7,60 et 7,95 (2d,1H), 12,30-12,50 (m,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 115. | 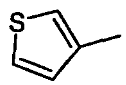 | H, H | $CH_3$ $(CH_2)_2$-(S) | -$COR_6$ | $(CH_3)_2CH$- | $OCH_3$ | - | - | 0,88 (t,3H), 1,27 (d,6H), 1,35 (m,2H), 1,67 (m,2H), 3,50 (m,2H), 3,80 (s,3H), 4,00 (m,1H), 4,38 (m,1H), 7,00 (d,1H), 7,25 (d,1 H), 7,50 (d,1H), 8,35 (d,1H), 12,50 (s,1 H)*** |
| 116. | 3,5-difluorophényle | OH, H(S) | $CH_3$ $(CH_2)_2$-(S) | -$COR_6$ | phényle | $OCH_3$ | - | - | 0,84 (t,3H), 1,19-1,31 (m, 2H), 1,74 (m, 2H), 3,69 (s, 3H), 4,50 (m,1 H), 5,09 (m,1 H), 6,58 (s,1H), 7,50 (s,large, 3H), 7,45 et 7,51 (2s,5H), 8,31 (d,1H), 12,75 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 117. | 3,5-difluorophényle | OH, H (R) | $CH_3(CH_2)_2$-(S) | $-COR_6$ | phényle | $OCH_3$ | - | - | 0,86 (t,3H), 1,26-1,36 (m, 2H), 1,75 (m, 2H), 3,69 (s, 3H), 4,48 (m,1 H), 5,11 (s,1 H), 6,59 (s,1H), 7,14 (s,large, 3H), 7,34 et 7,49 (2s,5H), 8,35 (d,1 H), 12,84 (s,1 H)*** |
| 118. | 3,5-difluorophényle | H, H | $CH_3(CH_2)_2$-(S) | $-COR_6$ | $(CH_3)_2CH$- | $OCH_2CH_3$ | - | - | 0,87 (t,3H), 1,32 (d,6H), 1,35 (m,2H), 1,61 (m,2H), 3,54 (m,2H), 3,96 (m,1 H), 4,24 (m,2H), 4,37 (m,1 H), 6,96 (d,2H), 7,07 (t,1 H), 8,46 (d,1H), 12,50 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 119. | 3,5-difluorophényle | OH, H(S) | $CH_3$ $(CH_2)_2$-(S) | -$COR_6$ | $(CH_3)_3CCH_2$- | $OCH_3$ | - | - | 0,81 (t,3H), 0,90 (s,9H), 1,21 (m,2H), 1,69 (m,2H), 3,08 (m,2H), 3,77 (s,3H), 4,44 (m,1H), 5,07 (s,1H), 7,17 (m,3H), 8,28 (d,1H)*** |
| 120. | 3,5-difluorophényle | OH, H (R) | $CH_3$ $(CH_2)_2$-(S) | -$COR_6$ | $(CH_3)_3CCH_2$- | $OCH_3$ | - | - | 0,87 (t,3H), 0,97 (s,9H), 1,24 (m,2H), 1,72 (m,2H), 3,12 (m,2H), 3,80 (s,3H), 4,41 (m,1H), 5,10 (d,1 H), 6,45 (d,1 H), 7,10 (m,3H), 8,30 (d,1H), 12,50 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 121. | 3-chlorophényle | 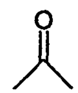 | CH3 $(CH_2)_2$-(S) | $-COR_6$ | $(CH_3)_2CH-$ | $OCH_3$ | - | - | 0,91 (t,3H), 1,32 (d,6H), 1,37 (m,2H), 1,78 (m,2H), 3,79 (s,3H), 4,02 (m,1 H), 4,56 (m,1H), 7,63 (t,1H), 7,80 (d,1H), 7,94 (d,1 H), 7,99 (s,1 H), 9,33 (d,1 H), 12,66 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 122. | 2-benzyloxyphényle | OH, H | $CH_3(CH_2)_2$-(S) | $-COR_6$ | $(CH_3)_2CH-$ | $OCH_3$ | - | - | 0,85 (m,3H), 1,26 (m,2H), 1,28 (d,6H), 1,69 (m,2H), 3,78 (s,3H), 3,98 (m,1H), 4,47 (m,1H), 5,22 (s,2H), 5,33 (2d, 1H), 6,07 et 6,11 (2d,1 H), 6,90-7,50 (m, 8H) 8,00 (m,1 H), 12,48 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 123. | 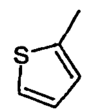 | H, H | $CH_3$ $(CH_2)_2$-(S) | $-COR_6$ | $(CH_3)_2CH-$ | $OCH_3$ | - | - | 0,87 (t,3H), 1,28 (d,6H), 1,36 (m,2H), 1,67 (m,2H), 3,70 (m,2H), 3,78 (s,3H), 3,98 (m,1H), 4,36 (m,1H), 6,90 et 6,92 (d+t, 2H), 7,33 (d,1 H), 8,41 (d, 1 H), 12,58 (s, 1H)*** |
| 124. | 2,3-difluorophényle | OH, H (S) | $CH_3$ $(CH_2)_2$-(S) | $-COR_6$ | $(CH_3)_2CH-$ | $OCH_3$ | - | - | 0,85 (t,3H), 1,27 (d,6H), 1,31 (m,2H), 1,72 (m,2H), 3,79 (s,3H), 3,97 (m,1H), 4,49 (m,1 H), 5,25 (s, 1 H), 6,62 (s,large 1 H), 7,22 (m, 2H), 7,36 (m, 1H), 8,21 (d, 1H), 12,53 (s, 1H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 125. | 2,3-difluorophényle | OH, H (R) | $CH_3(CH_2)_2$-(S) | -$COR_6$ | $(CH_3)_2CH$- | $OCH_3$ | - | - | 0,89 (t,3H), 1,29 (d,6H), 1,33 (m,2H), 1,71 (m,2H), 3,80 (s,3H), 3,96 (m,1 H), 4,47 (m,1H), 5,30 (d,1 H), 6,58 (d,1H), 7,19 (m,2H), 7,26 (m,1H), 8,26 (d,1H), 12,57 (s,1 H)*** |
| 126. | 2,5-difluorophényle | OH, H (S) | $CH_3(CH_2)_2$-(S) | -$COR_6$ | $(CH_3)_2CH$- | $OCH_3$ | - | - | 0,86 (t,3H), 1,28 (d,6H), 1,31 (m,2H), 1,72 (m,2H), 3,79 (s,3H), 3,98 (m,1H), 4,48 (m,1H), 5,21 (s,1H), 6,59 (s,large 1H), 7,21 (m, 3H), 8,22 (d,1 H), 12,51 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 127. | 2,5-difluorophényle | OH, H (R) | $CH_3(CH_2)_2$-(S) | $-COR_6$ | $(CH_3)_2CH-$ | $OCH_3$ | - | - | 0,90 (t,3H), 1,34 (d,6H), 1,39 (m,2H), 1,77 (m,2H), 3,83 (s,3H), 4,06 (m,1 H), 4,50 (m,1H). 5,36 (d,1H), 6,56 (d,1H), 7,25 (m,3H), 8,32 (d,1H), 12,58 (s, 1H)*** |
| 128. | 2-hydroxyphényle | OH, H | $CH_3(CH_2)_2$-(*S*) | $-COR_6$ | $(CH_3)_2CH-$ | $OCH_3$ | - | - | 0,91 (m,3H), 1,28 (m,2H), 1,30 (d,6H), 1,80 (m,2H), 3,85 (s,3H), 4,07 (m,1H), 4,53 (m,1 H), 5,31 et 5,35 (2s,1 H), 6,87 (m,2H), 7,13 (m,1H), 7,27 (m,1H). 8,17 (m.1H), 9,66 (s, large 1 H), 12,49 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 129, | 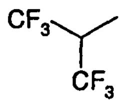 | OH, H (S) | $CH_3(CH_2)_2$-(*S*) | $-COR_6$ | $(CH_3)_2CH$- | $OCH_3$ | - | - | 0,92 (t,3H), 1,27 (m,2H), 1,32 (d,6H), 1,77 (m,2H), 3,84 (s,3H), 4,05 (m,1H), 4,34 (m,1H), 4,59 (m,1 H), 4,64 (m,1 H), 7,08 (d,1 H), 8,25 (d,1 H), 12,50 (s,1 H)*** |
| 130. | 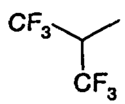 | OH, H (R) | $CH_3(CH_2)_2$-(*S*) | $-COR_6$ | $(CH_3)_2CH$- | $OCH_3$ | - | - | 0,94 (t,3H), 1,37 (d,6H), 1,42 (m,2H), 1,78 (m,2H), 3,85 (s,3H), 4,06 (m,1 H), 4,28 (m,1 H), 4,56 (m,1H), 4,66 (m,1 H), 7,10 (d,1 H), 8,40 (d,1 H), 12,58 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 131. | 3,5-difluorophényle | H, H | $CH_3(CH_2)_2$-(*S*) | $-COR_6$ | $(CH_3)_2CH-$ | NR7R8 | 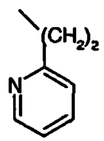 H, | - | 0,87 (t,3H), 1,28 (d,6H), 1,29 (m,2H), 1,63 (m,2H), 2,98 (m,2H), 3,52 (m,2H), 3,62 (m,2H), 4,20 (m, 1H), 4,46 (m, 1H), 6,98 (d,2H), 7,10 (t, 1H), 7,28 (m,2H), 7,70 (t, 1H), 7,90 (m,2H), 8,53 (m,2H), 12,20 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 132. | 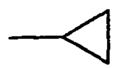 | OH, H (S) | $CH_3(CH_2)_2$-(*S*) | $-COR_6$ | $(CH_3)_2CH-$ | $OCH_3$ | - | - | 0,33 (m,2H), 0,37 (m,2H), 0,90 (t,3H), 1,09 (m,1H), 1,30 (s,6H), 1,34 (m,2H), 1,71 (m,2H), 3,66 (m,1 H), 3,83 (s,3H), 4,03 (m,1H), 4,59 (m,1H), 5,55 (d,1H), 7,88 (d,1H), 12,52 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 133. | 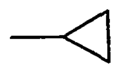 | OH, H | $CH_3(CH_2)_2$-(*S*) | $-COR_6$ | $(CH_3)_2CH-$ | $OCH_3$ | - | - | 0,30 (m,2H), 0,38 (m,2H), 0,86 (t,3H), 1,03 (m,1H), 1,27 (d,6H), 1,30 (m,2H), 1,67 (m,2H), 3,55 (m,1 H), 3,78 (s,3H), 3,99 (m,1 H), 4,50 (m,1 H), 5,37 et 5,50 (2d,1 H), 7,76 et 7,84 (2d,1 H), 12,50 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 134. | 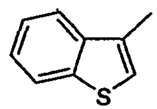 | H, H | $CH_3(CH_2)_2$-(*S*) | -$COR_6$ | $(CH3)_2CH$- | $OCH_3$ | - | - | 0,87 (t,3H), 1,28 (d,6H), 1,36 (m,2H), 1,67 (m,2H), 3,75 (m,2H), 3,77 (s,3H), 3,98 (m,1 H), 4,42 (m,1 H), 7,33 (m,2H), 7,50 (s,1 H), 7,83 (d,1 H), 7,94 (d,1H), 8,52 (d,1H), 12,58 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 135. | 3-chlorophényle | OH, H | $CH_3(CH_2)_2$-(*S*) | $-COR_6$ | $(CH_3)_2CH-$ | $OCH_3$ | - | - | 0,87 (m,3H), 1,25 (m,2H), 1,28 (s,6H), 1,75 (m,2H), 3,83 (s,3H), 4,01 (m,1H), 4,48 (m,1H), 4,94 (2d,1H), 6,14 et 6,29 (2d,1H), 6,67 (m,1H), 6,87 (m,2H), 7,14 (m,1H), 8,11 (m,1H), 8,41 (2s,1H), 12,52 (d,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 136. | 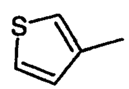 | OH, H | $CH_3(CH_2)_2$-(*S*) | $-COR_6$ | $(CH_3)_2CH-$ | $OCH_3$ | - | - | 0,87 (m,3H), 1,24 (s,6H), 1,26 (m,2H), 1,73 (m,2H), 3,80 (s,3H), 3,98 (m,1 H), 4,45 (m,1 H), 5,08 (m,1H), 6,17 et 6,27 (2d,1 H), 7,11 (d, 1H), 7,41-7,48 (m, 2H), 8,14 (m, 1H), 12,50 (d, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 137. | 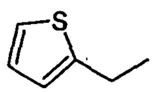 | H, H | $CH_3(CH_2)_2$-(*S*) | $-COR_6$ | $(CH_3)_2CH-$ | $OCH_3$ | - | - | 0,86 (t,3H), 1,28 (d,6H), 1,29 (m,2H), 1,61 (m,2H), 2,47 (m,2H), 2,81 (m,2H), 3,88 (s,3H), 4,00 (m,1H), 4,46 (m,1 H), 6,98 (s,1H), 7,23 (s, 1 H), 7,43 (s,1H), 8,16 (d,1H), 12,44 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 138. | 3,5-difluorophényle | H, H | $CH_3(CH_2)_2$-(*S*) | $-COR_6$ | $(CH_3)_2CH-$ | NR7R8 | H, 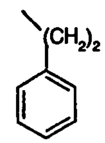 | - | 0;92 (t,3H), 1,31 (d,6H), 1,40 (m,2H), 1,71. (m,2H), 2,86 (m,2H), 3,54 (m,2H), 3,60 (m,2H), 4,24 (m,1H), 4,50 (m,1H), 7,13 (d,2H), 7,18 (t,1H), 7,25-7,36 (m, 5H), 7,80 (d,1 H), 8,56 (d,1 H), 12,25 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 139. | 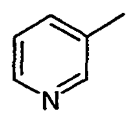 | OH, H | $CH_3(CH_2)_2$-(*S*) | $-COR_6$ | $(CH_3)_2CH-$ | $OCH_3$ | - | - | 0,84 (t,1H), 1,20 (m,2H), 1,22 (d,6H), 1,70 (m,2H), 3,80 (s,3H), 3,99 (m,1H), 4,45 (m,1 H), 5,07 (s,1H), 6,49 (d,1H), 7,37 (d,1H), 7,80 (d,1H), 8,27 (d,1H), 8,48 (d,1 H), 8,61 (s,1 H), 12,42 (s large, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 140. | 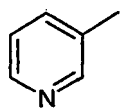 | 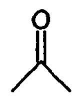 | $CH_3(CH_2)_2$-(*S*) | $-COR_6$ | $(CH_3)_2CH-$ | $OCH_3$ | - | - | 0,95 (t,3H), 1,35 (d,6H), 1,42 (m,2H), 1,85 (m,2H), 3,82 (s,3H), 4,05 (m,1H), 4,62 (m, 1H), 7,70 (d,1 H), 8,40 (d, 1H), 8,90 (d,1H), 9,19 (s, 1H), 9,35 (d, 1H), 12,65 (s,1H)*** |
| 141. | 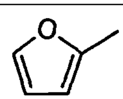 | 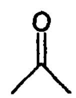 | $CH_3(CH_2)_2$-(*S*) | $-COR_6$ | $(CH_3)_2CH-$ | $OCH_3$ | - | - | 0,95 (t,3H), 1,38 (d,6H), 1,41 (m,2H), 1,85 (m,2H), 3,84 (s,1H), 4,04 (m,1H), 4,58 (m,1 H), 6,86 (s,1H), 7,86 (s, 1H), 8,23 (s,1H), 9,20 (d,1 H), 12,60 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 142. | 3,5-difluorophényle | H, H | $CH_3(CH_2)_2$(S) | $-COR_6$ | 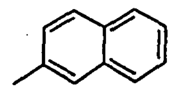 | $OCH_3$ | - | - | 0,88 (t,3H), 1,35 (m,2H), 1,69 (m,2H), 3,61 (m,2H), 3,68 (s,3H), 4,46 (m,1H), 7,08-(d,2H), 7,09 (t,1H), 7,60 (m,3H), 7,96 (m,3H), 7,99 (s,1H), 8,52 (d,1H), 12,78 (s,1H)*** |
| 143. | 3,5-difluorophényle | H, H | $CH_3$ $(CH_2)_2$-(S) | $-COR_6$ | 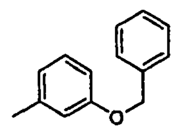 | $OCH_3$ | - | - | 0,89 (t,3H), 1,30 (m,2H), 1,68 (m,2H), 3,56 (m,2H), 3,67 (s,3H), 4,43 (m,1 H), 5,13 (s,2H), 6,98 (d,2H), 7,08-7,47 (m, 10H), 8,58 (d, 1H), 12,65 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 144. | 3,5-difluorophényle | H, H | $CH_3$ | $-(CH_2)_2CH_3$ | $-COR_6$ | $OCH_3$ | - | - | 0,88 (t,3H), 1,32 (d,3H), 1,66 (m,2H), 2,95 (m,2H), 3,54 (s,2H), 3,76 (s,3H), 4,47 (m,2H), 6,97 et 7,10 (2m,3H), 8,63 (d, 1H), 12,66 (s,1H)*** |
| 145. | 3,5-difluorophényle | H, H | $CH_3$ $(CH_2)_2$-(S) | 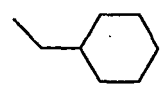 | $-COR_6$ | $OCH_3$ | - | - | 0,87 (t,3H), 0,98 (m,2H), 1,13 (m, 3H), 1,27 et 1,36 (2m,2H), 1,59-1,70 (m, 8H), 2,83 et 2,93 (2m,2H), 3,28 (s,2H), 3,78 (s,3H), 4,44 (m,1 H), 6,96 et 7,09 (m, 3H), 8,54 (d, 1H), 12,66 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 146. | 3,5-difluorophényle | H, H | $CH_3(CH_2)_2$-(S) | $-CH(CH_3)_2$ | $-COR_6$ | $OCH_3$ | - | - | 0,87 (t,3H), 1,27 (d,6H), 1,35 (m,2H), 1,64 (m,2H), 3,54 (s,2H), 3,77 (s,3H), 3,90 (m,1 H), 4,45 (m,1H), 6,96-7,09 (m, 3H), 8,53 (d, 1H), 12,69 (s, 1H)*** |
| 147. | 3,5-difluorophényle | OH, H (S) | $CH_3$ (S) | $-CH(CH_3)_2$ | $-COR_6$ | $OCH_3$ | - | - | 1,18 (d, 6H), 1,32 (d,3H), 3,77 (s,3H), 3,91 (m, 1H), 4,48 (m, 1H), 5,05 (s, 1H), 6,53 (s,1H), 7,12 (m,3H), 8,37 (d, 1H), 12,61 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 148. | 3,5-difluorophényle | H, H | $CH_3$ (S) | $-CH(CH_3)_2$ | $-COR_6$ | $OCH_3$ | - | - | 1,15 (d,6H), 1,32 (d,3H), 3,53 (s,2H), 3,77 (s,3H), 4,04 (m, 1H), 4,47 (m, 1H), 6,97-7,09 (m, 3H), 8,59 (d, 1H), 12,66 (s, 1H)*** |
| 149. | 3,5-difluorophényle | OH, H (R) | $CH_3$ (S) | $-CH(CH_3)_2$ | $-COR_6$ | $OCH_3$ | - | - | 1,17 (d,6H), 1,35 (d,3H), 3,77 (s,3H), 3,91 (m, 1H), 4,49 (m,1H), 5,04 (s, 1H), 6,47 (s,1H), 7,12 (m,3H), 8,37 (d,1H), 12,62 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 150. | 3,5-difluorophényle | OH, H (S) | $CH_3(CH_2)_2$-(S) | $-CH(CH_3)_2$ | $-COR_6$ | $OCH_3$ | - | -73° | 0,81 (t,3H), 1,18 (d,6H), 1,22 (m,2H), 1,71 (m,2H), 3,77 (s,3H), 3,90 (m, 1H), 4,50 (m, 1H), 5,06 (s, 1H), 6,54 (s, 1H), 7,12 (m,3H), 8,30 (d,1 H), 12,66 (s,1H)*** |
| 151. | 3,5-difluorophényle | OH, H (R) | $CH_3(CH_2)_2$-(S) | $-CH(CH_3)_2$ | $-CO_6$ | $OCH_3$ | - | -104° | 0,83 (t,3H), 1,19 (d,6H), 1,22 (m,2H), 1,70 (m,2H), 3,77 (s,3H), 3,91 (m, 1H), 4,48 (m,1 H), 5,07 (s,1 H), 6,42 (s,1 H), 7,12 (m,3H), 8,34 (d, 1H), 12,67 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 152. | 3,5-difluorophényle | H, H | $CH_3(CH_2)_2$-(S) | phényle | -$COR_6$ | $OCH_2CH_3$ | - | -88° | 0,86 (t,3H), 1,19 (t,3H), 1,29 (m,2H), 1,67 (m,2H), 3,57 (S,2H), 4,18 (m,2H), 4,47 (m,1 H), 6,98 et 7,10 (m, 3H), 7,42 et 7,68 (2s,5H), 8,55 (d,1 H), 12,81 (s,1 H)*** |
| 153. | 3,5-difluorophényle | H, H | $CH_3(CH_2)_2$-(S) | -$CH(CH_3)_2$ | -$COR_6$ | $OCH_2CH_3$ | - | -120° | 0,87 (t,3H), 1,18 (d,6H), 1,27 (t,3H), 1,30 (m,2H), 1,65 (m,2H), 3,55 (s,2H), 3,90 (m,1H), 4,25 (m,2H), 4,43 (m,1 H), 6,80-7,13 (m, 3H), 8,53 (d,1 H), 12,66 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 154. | 3,5-difluorophényle | OH, H (S) | $CH_3$ $(CH_2)_2$-(S) | $-(CH_2)_2CH_3$ | $-COR_6$ | $OCH_3$ | - | - | 0,80 (t,3H), 0,90 (t,3H), 1,22 (m,2H), 1,68 et 1,72 (2m,4H), 2,98 (m,2H), 3,78 (s, 3H), 4,52 (m, 1H), 5,06 (s, 1H), 6,55 (s, 1H), 7,17 (m, 3H), 8,31 (d, 1H), 12,68 (s, 1H)*** |
| 155. | 3,5-difluorophényle | OH, H (R) | $CH_3$ $(CH_2)_2$-(S) | $-(CH_2)_2CH_3$ | $-COR_6$ | $OCH_3$ | - | - | 0,80 (t,3H), 0,90 (3,3H), 1,22 (m,2H), 1,66 et 1,73 (2m,4H), 2,98 (m,2H), 3,78 (s, 3H), 4,49 (m,1 H), 5,09 (s,1 H), 6,43 (s,1H), 7,14 (m,5H), 8,38 (d,1H), 12,68 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 156. | 3,5-difluorophényle | H, H | $CH_3(CH_2)_2$-(S) | $-(CH_2)_2CH_3$ | $-COR_6$ | $OCH_3$ | - | -85° | 0,89 (2t,6H), 1,37 (m,2H), 1,63 (m,4H), 2,95 (m,2H), 3,55 (s,2H), 3,76 (s,3H), 4,43 (m,1H), 6,80-7,12 (m, 3H), 8,55 (d, 1H), 12,68 (s, 1H)*** |
| 157. | 3,5-difluorophényle | H, H | $CH_3(CH_2)_2$-(S) | $-CH_2CH_3$ | $-COR_6$ | $OCH_3$ | - | -112° | 0,88 (t,3H), 1,30 (t,3H), 1,37 (m,2H), 1,63 (m,2H), 2,98 (m,1H), 3,56 (s,2H), 3,78 (s,3H), 4,50 (m,1H), 6,81-7,25 (m, 3H), 8,56 (d, 1H), 12,71 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 158. | 3,5-difluorophényle | H, H | $CH_3(CH_2)_2$-(S) | $-CHCH(CH_3)_2$ | $-COR_6$ | $OCH_3$ | - | -99° | 0,89 (t+d,12H), 1,29 (m,2H), 1,66 (m,2H), 2,05 (m,1H), 2,86 (m,2H), 3,56 (s,2H), 3,78 (s,3H), 4,46 (m,1 H), 7,06-7,15 (m, 3H), 8,64 (d,1 H), 12,68 (s, 1H)*** |
| 159. | 3,5-difluorophényle | H, H | $CH_3(CH_2)_2$-(S) | $-CH_3$ | $-COR_6$ | $OCH_3$ | - | -126° | 0,88 (t,3H), 1,29 (m, 2H), 1,65 (m,2H), 2,55 (s,3H), 3,56 (s,2H), 3,78 (s,3H), 4,45 (m, 1H), 6,99-7,14 (m, 5H), 8,55 (d, 1H), 12,68 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 160. | 3,5-difluorophényle | OH, H (S) | $CH_3$ $(CH_2)_2$-(S) | $-CH_2CH_3$ | $-COR_6$ | $OCH_3$ | - | - | 0,82 (t,3H), 1,20 (d,6H), 1,26 (m,2H), 1,72 (m,2H), 2,99 (m,2H), 3,78 (s,3H), 4,49 (m,1H), 5,10 (s,1 H), 6,43 (s,1H), 7,16 (m,3H), 8,36 (d,1H), 12,70 (s,1 H)*** |
| 161. | 3,5-difluorophényle | OH, H (R) | $CH_3$ $(CH_2)_2$-(S) | $-CH_2CH_3$ | $-COR_6$ | $OCH_3$ | - | - | 0,89 (t,3H), 1,24 (d,6H), 1,28 (m,2H), 1,77 (m,2H), 3,04 (m,2H), 3,83 (s,3H), 4,52 (m,1 H), 5,15 (s,1 H), 6,48 (s,1H), 7,18 (m,3H), 8,40 (d,1 H), 12,65 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 162. | 3,5-difluorophényle | 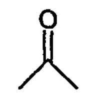 | $CH_3(CH_2)_2$-(S) | $-CH_2CH_3$ | $-COR_6$ | $OCH_3$ | - | - | 0,92 (t,3H), 1,20 (t,3H), 1,38 (m,2H), 1,78 (m,2H), 3,00 (m,2H), 3,80 (s,3H), 4,64 (m,1 H), 7,72 (m,3H), 9,45 (d, 1H), 12,95 (s,1 H)*** |
| 163. | $(CH_3)_3C$- | OH, H (S) | $CH_3(CH_2)_2$-(S) | $-CH_2CH_3$ | $-COR_6$ | $OCH_3$ | - | - | 0,87 (t,3H), 0,91 (s,9H), 1,21 (t,3H), 1,27 (m,2H), 1,69 (m,2H), 3,01 (m,2H), 3,57 (d,1H), 3,78 (s,3H), 4,57 (m, 1H), 5,56 (d, 1H), 7,85 (d,1 H), 12,63 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 164. | $(CH_3)_3C$- | 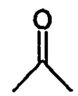 | $CH_3(CH_2)_2$-(S) | $-CH_2CH_3$ | $-COR_6$ | $OCH_3$ | - | - | 0,95 (t,3H), 1,34 (s,9H), 1,35 (t,3H), 1,40 (m,2H), 1,75 (m,2H), 3,04 (m,2H), 3,98 (s,3H), 4,62 (m,1H), 9,03 (d,1H). 12,85 (s,1 H)*** |
| 165. | 3,5-difluorophényle | OH, H (S) | $CH_3(CH_2)_2$-(S) | $-CH_3$ | $-COR_6$ | $OCH_3$ | - | - | 0,82 (t,3H), 1,26 (m,2H), 1,69 (m,2H), 2,63 (s,3H), 3,77 (s,3H), 4,50 (m,1 H), 5,07 (d,1 H), 6,55 (d,1 H), 7,16 (m,3H), 8,31 (d,1H), 12,65 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 166. | 3,5-difluorophényle | H, H | $CH_3(CH_2)_2$-(*S*) | $-CH_2C(CH_3)_3$ | $-COR_6$ | $OCH_3$ | - | - | 0,87 (t,3H), 0,94 (s,9H), 1,31 (m,2H), 1,66 (m,2H), 2,89-3,05 (m, 2H), 3,59 (s, 2H), 3,77 (s, 3H), 4,48 (m, 1H), 6,98 (d, 2H), 7,10 (t, 1H), 8,54 (d, 1H), 12,65 (s, 1H)*** |
| 167. | 3,5-difluorophényle | 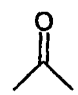 | $CH_3(CH_2)_2$-(S) | $-CH_3$ | $-COR_6$ | $OCH_3$ | - | - | 0,90 (t,3H), 1,38 (m,2H), 1,75 (m,2H), 2,63 (s,3H), 3,78 (s,3H), 4,61 (m,1H), 7,69 (m,3H), 9,43 (d,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 168. | 3,5-difluorophényle | OH, H (S) | $CH_3(CH_2)_2$-(S) | $-CH_2C(CH_3)_3$ | $-COR_6$ | $OCH_3$ | - | - | 0,81 (t,3H), 0,92 (s,9H), 1,20 (m,2H), 1,70 (m,2H), 2,90-2,98 (m, 2H), 3,77 (s, 3H), 4,50 (m,1 H), 5,08 (d,1 H), 6,52 (d,1 H), 7,12 (m, 3H), 8,30 (d, 1H). 12,60 (s, 1H)*** |
| 169. | 3,5-difluorophényle | OH, H (R) | $CH_3(CH_2)_2$-(S) | $-CH_2C(CH_3)_3$ | $-COR_6$ | $OCH_3$ | - | - | 0,84 (t,3H), 0,93 (s,9H), 1,26 (m,2H), 1,71 (m,2H), 2,90-3,00 (m, 2H), 3,80 (s, 3H), 4,47 (m, 1H), 5,08 (d, 1H), 6,43 (d, 1H), 7,17 (m, 3H), 8,35 (d,1 H), 12,60 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 170. | 3,5-difluorophényle | H. H | $CH_3(CH_2)_2$-(S) | 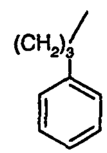 | $-COR_6$ | $OCH_2CH_3$ | - | - | 0,89 (t,3H), 1,27 (t,3H), 1,29 (m,2H), 1,66 (m,2H), 1,97 (m,2H), 2,63 (m,2H), 3,00 (m,2H), 3,57 (s,2H), 4,25 (m,2H), 4,45 (m,1H), 6,99 (d,2H), 7,09 (t,1H), 7,19-7,27 (m, 5H), 8,55 (d,1 H), 12,68 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 171. | 3,5-difluorophényle | OH. H (S) | $CH_3(CH_2)_2$-(S) | 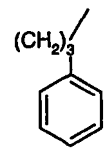 | $-COR_6$ | $OCH_2CH_3$ | - | - | 0,82 (t,3H), 1,18 (m,2H), 1,22 (t,3H), 1,67 (m,2H), 1,90 (m,2H), 2,61 (m,2H), 3,00 (m,2H), 4,23 (m,2H), 4,47 (m,1 H), 5,06 (d,1 H), 6,54 (d,1 H), 7,10-7,32 (m, 8H), 8,28 (d, 1H), 12,60 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 172. | 3,5-difluorophényle | OH, H (R) | $CH_3(CH_2)_2$-(S) | 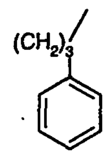 | $-COR_6$ | $OCH_2CH_3$ | - | - | 0,82 (t,3H), 1,24 (t,3H), 1,29 (m,2H), 1,68 (m,2H), 1,91 (m,2H), 2,61 (m,2H), 2,99 (m,2H), 4,21 (m,2H), 4,46 (m,1 H), 5,07 (d,1H), 6,40 (d,1H), 7,07-7,31, (m, 8H), 8,32 (d, 1H), 12,60 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 173. | 3,5-difluorophényle | H, H | $CH_3(CH_2)_2$-(S) | 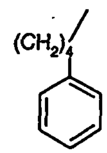 | $-COR_6$ | $OCH_2CH_3$ | - | - | 0,85 (t,3H), 1,26 (t,3H), 1,27 (m,2H), 1,59 (m,8H), 2,56 (m,2H), 2,99 (m,2H), 3,54 (m,2H), 4,23 (m,2H), 4,43 (m,1H), 6,98 (d,2H), 7,09 (t,1H), 7,14-7,30 (m, 5H), 8,52 (d, 1H), 12,82 (s, 1H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 174. | 3,5-difluorophényle | H, H | $CH_3$ $(CH_2)_2$-(S) | $(CH_2)_4CH$ $(CH_3)$ 2 | $-COR_6$ | $OCH_2CH_3$ | - | - | - 0,82 (d,6H), 0,86 (t,3H), 1,18 (m,2H), 1,27 (t,3H), 1,28 (m,2H), 1,30 (m,2H), 1,47 (m,2H), 1,63 (m,4H), 2,96 (m,1H), 3,56 (m,2H), 4,24 (m,2H), 4,44 (m,1H), 6,98 (d,2H), 7,14 (t,1H), 8,53 (d, 1H), 12,67 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 175. | 3,5-difluorophényle | H, H | $CH_3(CH_2)_2$-(S) | $(CH_2)_2CH(CH_3)$ | $-COR_6$ | $OCH_3$ | - | - | 0,88 (t,3H), 0,91 (d,6H), 1,37 (m,2H), 1,52 (m,4H), 1,63 (m,2H), 3,00 (m,1 H), 3,63 (m,2H), 3,78 (s,3H), 4,45 (m,1H), 6,99 (d,2H), 7,10 (t, 1H), 8,56 (d, 1H), 12,67 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 176. | 3,5-difluorophényle | H, H | $CH_3$ $(CH_2)_2$-(S) | $-CH_3$ | $-COR_6$ | $NR_7R_8$ | $CH_3$, 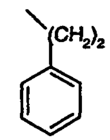 | -103° | 0,92 (t,3H), 1,38 (m,2H), 1,65 (m,2H), 2,14 (s,3H), 2,88 (m,2H), 2,97 (s,3H), 3,63 (s,2H), 3,67 (m,2H), 4,51 (m,1 H), 7,04 (d,2H), 7,12 (t,1H), 7,24-7,30 (m, 5H), 8,55 (d, 1H), 12,46 (s, 1H),*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 177. | 3,5-difluorophényle | H, H | $CH_3(CH_2)_2$-(S) | $-CH_3$ | $-COR_6$ | $NR_7R_8$ | H, 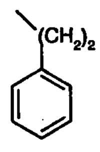 | -97° | 0,91 (t,3H), 1,37 (m,2H), 1,67 (m,2H), 2,45. (s,3H), 2,85 (m,2H), 3,45 (m,2H), 3,60 (s,2H), 4,53 (m,1H), 7,03 (d,2H), 7,14 (t,1H), 7,24-7,32 (m, 5H), 7,99 (d, 1H), 8,55 (d, 1H), 12,44 (s, 1H)*** |
| 178. | 3,5-difluorophényle | H, H | $CH_3(CH_2)_2$-(S) | $-CH_3$ | $-COR_6$ | $NR_7R_8$ | H, $CH_3$ | - | 0,91 (t,3H), 1,37 (m,2H), 1,66, (m,2H), 2,50 (s,3H), 2,75 (d,1 H), 3,62 (s,2H), 4,48 (m,1 H), 7,01 (d,2H), 7,14 (t,1H), 7,91 (d,1H). 8,54 (d,1H), 12,43 (s,1 H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 179. | 3,5-difluorophényle | H, H | $CH_3(CH_2)_2$-(S) | $-COR_6$ | 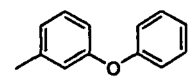 | $OCH_3$ | - | - | 0,91 (t,3H), 1,35 (m,2H), 1,72 (m,2H), 3,60 (m,2H), 3,85 (s,3H), 4,47 (m,1 H), 7,02 (d,2H), 7,09 (t,1 H), 7,10-7,47 (m, 9H), 8,55 (d, 1H), 12,80 (s, 1H)*** |
| 180. | 3,5-difluorophényle | H, H | $CH_3(CH_2)_2$ (S) | $-COR_6$ | 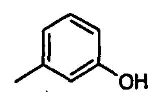 | $OCH_3$ | - | - | 0,93 (t, 3H), 1,32 (m, 2H), 1,70 (m, 2H), 3,66 (m,2H), 3,78 (s, 3H), 3,50 (m, 1 H), 6,90-6,95 (m, 3H), 7,08 (d, 2H), 7,17 (t, 1H), 7,29 (t, 1H). 8,58 (d, 1H). 9,68 (s, 1 H), 12,77 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 181. | 3,5-difluorophényle | H, H | $CH_3(CH_2)_2$-(S) | $-COR_6$ | 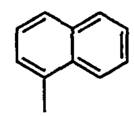 | $OCH_3$ | - | - | 0,92 (t,3H), 1,36 (m,2H), 1,70 (m,2H), 3,49 (s,3H), 3,61 (m,2H), 4,50 (m,1 H), 7,02 (d, 2H), 7,10 (t, 1 H), 7,53-7,62 (m, 5H), 8,04 (m, 2H), 8,55 (d, 1H), 12,87 (s, 1H)*** |
| 182. | 3,5-difluorophényle | H, H | $CH_3(CH_2)_2$-(S) | $-COR_6$ | 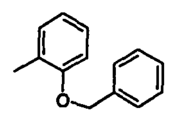 | $OCH_3$ | - | - | 0,93 (t, 3H), 1,39 (m, 2H), 1,68 (m, 2H), 3,57 (m, 5H), 4,49 (m, 1H), 5,15 (s, 2H), 7,06 (d, 2H), 7,19 (t, 1H), 7,34 (m, 5H), 8,60 (s, 1 H), 12,67 (s, 1 H) *** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 183. | 3,5-difluorophényle | H, H | $CH_3$ $(CH_2)_2$-(S) | $-COR_6$ | 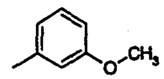 | $OCH_3$ | - | - | 0,93 (t, 3H), 1,39 (m, 2H), 1,68 (m, 2H), 3,61 (m, 2H), 3,73 (s, 3H), 4,82 (s, 3H), 4,47 (m,2H), 7,06 (d, 2H), 7,04-7,14 (m, 6H), 7,40 (t, 1H), 8,56 (s, 1H), 12,72 (s, 1H)*** |
| 184. | 3,5-difluorophényle | H, H | $CH_3$ $(CH_2)_2$-(S) | $-COR_6$ | 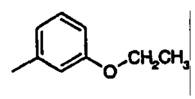 | $OCH_3$ | - | - | 0,91 (t, 3H), 1,34 (m, 2H), 1,39 (s, 3H), 1,69 (m, 2H), 3,57 (m, 2H), 3,71 (s, 3H), 4,10 (m, 2H), 4,47 (m, 1 H), 7,07 (m, 6H), 7,35 (t, 1H), 8,55 (s,1H), 12,78 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 185. | 3,5-difluorophényle | H, H | $CH_3(CH_2)_2$-(S) | $-COR_6$ | $-CH(CH_3)_2$ | $OC(CH_3)_3$ | - | - | 0,89 (t, 3H), 1,31 (d, 6H), 1,37 (m, 2H), 1,55 (s, 9H), 1,64 (m, 2H), 3,59 (m, 2H), 3,95 (m, 2H), 4,45 (m, 1H), 7,09 (d, 2H), 7,12 (t, 1 H), 8,49 (s, 1 H), 12,40 (s, 1 H) *** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 186. | 3,5-difluorophényle | H, H | $CH_3$ $(CH_2)_2$-(S) | $-COR_6$ | $-CH(CH_3)_2$ | $NR_7R_8$ | 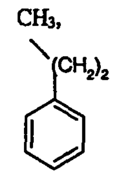 | - | 0,91 (m,3H) ; 1,22 (m,6H), 1,32 (m,2H) ; 1,69 (m,2H) ; 2,89 et 3,01 (2s,3H) ; 2,91 (m,2H) ; 3,07-3,40 (m, 2H) ; 3,70 (m, 2H) ; 3,71 (m, 1H); 4,45-4,51 (2m,1H) ; 7,02 (m,2H) ; 7,18 (m,2H) ; 7,25 (m,2H) ; 7,35 (m,2H) ; 8,53 (m, 1H); 12,25 (s, 1H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 187. | 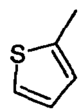 | 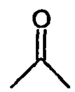 | $CH_3(CH_2)_2$-(S) | -$COR_6$ | -$CH(CH_3)_2$ | $OCH_3$ | - | -56° | 0,96 (t,3H) ; 1,36 (d, 6H); 1,42 (m, 2H); 1,87 (m,2H) ; 3,85 (s,3H) ; 4,07 (m,1H) ; 4,59 (m,1H) ; 7,38 (d, 1H); 8,22 (d, 1H); 8,26 (d, 1H); 9,22 (d,1 H) ; 12,68 (s large, 1H)*** |
| 188. | 3,5-difluorophényle | H, H | $CH_3(CH_2)_2$-(S) | -$COR_6$ | 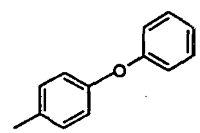 | $OCH_3$ | - | - | 0,92 (t,3H) ; 1,32-1,39 (m, 2H) ; 1,75 (m, 2H) ; 3,64 (m, 2H) ; 3,75 (s, 3H) ; 4,48 (m, 1H) ; 7,06 (m, 4H) ; 7,13 (m, 3H) ; 7,24 (m, 1H) ; 7,49 (m, 2H) ; 7,55 (m, 2H) ; 8,56 (m,1 H) ; 12,77 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 189. | 3,5-difluorophényle | OH, H (S) | $CH_3(CH_2)_2$-(S) | $-COR_6$ | 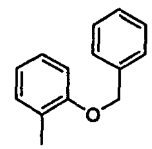 | $OCH_3$ | - | -56° | 0,89 (t,3H) ; 1,29 (m,2H) ; 1,79 (m,2H) ; 3,63 (s,3H) ; 4,54 (m,1H) ; 5,13 (s,1H) ; 5,17 (s,2H) ; 6,62 (s,1 H) ; 7,07 (t,1H) ; 7,23 (m,4H) ; 7,33 (m,6H) ; 7,43 (m,1H) ; 8,35 (d,1H) ; 12,68 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 190. | 3,5-difluorophényle | OH, H (R) | $CH_3(CH_2)_2$-(S) | $-COR_6$ | 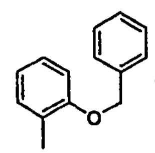 | $OCH_3$ | - | - | 0,94 (t,3H) ; 1,28-1,35 (2m, 2H) ; 1,78 (m, 2H) ; 3,65 (s, 3H) ; 4,49 (m,1 H) ; 5,13 (2s, 3H) ; 6,47 (s, 1H) ; 7,05 (t, 1H) ; 7,21 (m, 4H) ; 7,35 (m, 6H) ; 7,45 (m, 1H) ; 8,38 (d, 1H) ; 12,70 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 191. | 3,5-difluorophényle | 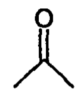 | $CH_3(CH_2)_2$-(S) | $-COR_6$ | 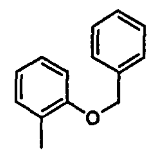 | $OCH_3$ | - | - | 0,99 (t,3H) ; 1,47 (m,2H) ; 1,80-1,86 (2m, 2H) ; 3,64 (s, 3H) ; 4,67 (m, 1H) ; 5,19 (s, 2H) ; 7,08 (t, 1 H) ; 7,24 (d, 1H) ; 7,30-7,50 (m,8H) ; 7,78 (m,2H) ; 9,46 (s,large 1H) ; 12,87 (s,large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 192. | 3,5-difluorophényle | H, H | $CH_3(CH_2)_2$-(S) | $-COR_6$ | 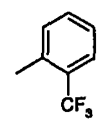 | $OCH_3$ | - | - | 0,98 (t,3H) ; 1,35-1,45 (m, 2H) ; 1,75 (m, 2H) ; 3,60 (m+s,5H) ; 3,50 (m,1 H) ; 7,06 (d,2H) ; 7,15 (t, 1H); 7,60 (d, 1H); 7,77 (m,2H) ; 7,90 (d,1 H) ; 8,55 (s,large 1 H) ; 12,90 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 193. | 3,5-difluorophényle | H, H | $CH_3(CH_2)_2$-(S) | $-COR_6$ | 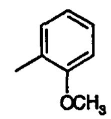 | $OCH_3$ | - | - | 0,97 (t,3H) ; 1,39 (m,2H) ; 1,75 (m,2H) ; 3,64 (m,2H) ; 3,81 (s,3H) ; 4,11 (s,3H) ; 4,51 (m,1H) ; 7,07 (m,3H) ; 7,17 (m,2H) ; 7,38 (d,1H); 7,48 (m,1H); 8,58 (s large, 1H) ; 12,75 (s, large1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 194. | 3,5-difluorophényle | H, H | $CH_3(CH_2)_2$-(S) | $-COR_6$ | 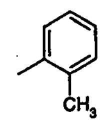 | $OCH_3$ | - | - | 0,94 (t,3H) ; 1,35 (m,1H) ; 1,40 (m,1H) ; 1,71 (m,2H) ; 2,17 (s,3H) ; 3,62 (m,2H) ; 3,73 (s,3H) ; 4,47 (m,1H) ; 7,04 (d,2H) ; 7,15 (t,1H) ; 7,29 (s, large 2H) ; 7,36 (m, 2H) ; 8,56 (d, large 1H) ; 12,78 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 195. | 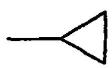 | 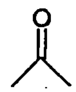 | $CH_3(CH_2)_2$-(S) | $-COR_6$ | $-CH(CH_3)_2$ | $OCH_3$ | - | -28,5° | 0,94 (t,3H) ; 1,07 (m,2H) ; 1,19 (m,2H); 1,32 (d,6H) ; 1,37 (m,2H) ; 1,80 (m,2H) ; 2,86 (m,1H) ; 3,85 (s,3H) ; 4,03 (m,1H) ; 4,50 (m,1H) ; 8,80 (d,1H) ; 12,56 (s,large 1 H)*** |
| 196. | 3,5-difluorophényle | 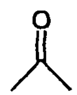 | $CH_3(CH_2)_2$-(S) | $-COR_6$ | 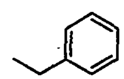 | $OCH_3$ | - | -71° | 0,94 (t,3H) ; 1,37 (m,2H) ; 1,79 (m,2H) ; 3,87 (s,3H) ; 4,54 (s,2H) ; 4,58 (m,1H) ; 7,29 (m,1 H) ; 7,35 (m,4H) ; 7,75 (m,3H) ; 9,38 (m,1H) ; 12,66 (s,large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 197. | 3,5-difluorophényle | H, H | $CH_3(CH_2)_2$-(S) | $-COR_6$ | 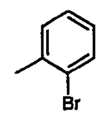 | $OCH_3$ | - | - | 0,95 (t,3H) ; 1,39-1,44 (2m, 2H) ; 1,77 (m, 2H) ; 1,38 et 1,43 (2m,2H) ; 1,75 (m,2H) ; 3,69 (m,2H) ; 3,81 (s,3H) ; 4,48 (m,1H) ; 7,15 (d,2H) ; 7,22 (t,1 H) ; 7,45 (m,1H) ; 7,51 (m,2H) ; 7,79 (d,1H) ; 8,58 (s,large 1H) ; 12,89 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 198. | 3,5-difluorophényle | H,H | $CH_3(CH_2)_7$ (S) | $-COR_6$ | $-CH(CH_3)_2$ | $NR_7R_8$ | H, 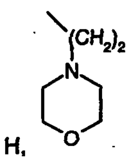 | - | 0,93 (t,3H) ; 1,28 (d,6H) ; 1,38 (m,2H) ; 1,68 (m,2H) ; 2,50 (m,4H) ; 2,56 (m,2H) ; 3,45 (m,2H) ; 3,60 (m,6H) ; 4,28 (m,1 H) ; 4,52 (m,1H) ; 7,06 (d,2H); 7,14 (t,1H) ; 7,73 (m,1H) ; 8,57 (d,1H) ; 12,30 (s,large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 199. | 3,5-difluorophényle | H, H | $CH_3(CH_2)_2$-(S) | $-COR_6$ | 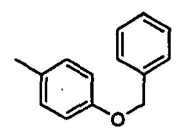 | $OCH_3$ | - | - | 0,94 (t,3H) ; 1,37-1,43 (2m, 2H) ; 1,73 (m, 2H) ; 3,60 (m, 2H) ; 3,74 (s, 3H) ; 4,50 (m, 1H) ; 5,20 (s, 2H) ; 7,06 (d, 2H) ; 7,12 (m, 3H) ; 7,36-7,50 (m,7H) ; 8,56 (d, 1H) ; 12,76 (s,large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 200. | 3,5-difluorophényle | H, H | $CH_3(CH_2)_2$-(S) | $-COR_6$ | 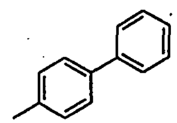 | $OCH_3$ | - | - | 0,93 (t,3H) ; 1,32-1,40 (m, 2H) ; 1,70 (r, 2H) ; 3,60 (m, 2H) ; 3,78 (s, 3H) ; 4,50 (m, 1H); 7,03 (d, 2H) ; 7,12 (t, 1H); 7,40 (m, 1H) ; 7,50 (m, 2H) ; 7,53 (m, 2H) ; 7,80 (m, 4H); 8,58 (d, 1H) ; 12,78 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 201. | $(CH_3)_3C$- | OH, H (S) | $CH_3(CH_2)_2$-(S) | $-COR_6$ | 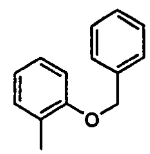 | $OCH_3$ | - | - | 0,93 (t,3H) ; 0,96 (s,9H) ; 1,40 (m, 2H); 1,78 (m,2H) ; 3,62 (s,1H) ; 3,63 (s,3H) ; 4,59 (m,1 H) ; 5,17 (s,2H) ; 5,65 (s,1 H) ; 7,07 (m,1 H) ; 7,21 (m,1H) ; 7,35 (m,6H) ; 7,44 (m, 1H); 7,80 (d, 1H); 12,62 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 202. | $(CH_3)_2CH-$ | OH, H (S) | $CH_3(CH_2)_2$-(S) | $-COR_6$ | 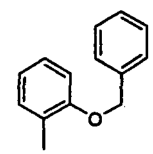 | $OCH_3$ | - | - | 0,78 (d,3H) ; 0,86 (t,3H) ; 0,97 (d,3H) ; 1,34 (m,2H) ; 1,78 (m,2H) ; 2,06 (m,1 H) ; 3,66 (s,3H) ; 3,80 (m,1H) ; 3,59 (m,1H) ; 5,17 (s,2H) ; 5,54 (s,1 H) ; 7,06 (m,1H) ; 7,20 (m,1 H) ; 7,32 (m,6H) ; 7,42 (m,1H) ; 7,89 (d, 1H), 12,66 (s,large 1H) |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 203. | $(CH_3)_2CH-CH_2-$ | OH, H (S) | $CH_3(CH_2)_2$-(S) | $-COR_6$ | 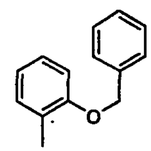 | $OCH_3$ | - | - | 0,94 (d,6H) ; 1,32 (m,2H) ; 1,50 (m,2H) ; 1,80 (m,2H) ; 1,87 (m,2H) ; 3,78 (s,3H) ; 3,99 (m, 1 H) ; 3,62 (m, 1H) ; 5,17 (s,2H) ; 5,60 (s, 1H) ; 7,17 (t,1 H) ; 7,20 (d,1 H) ; 7,30 (m,6H) ; 7,42 (m, 1H) ; 7,95 (d, 1H) ; 12,61 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 204. | 3,5-difluorophényle | H, H | $CH_3(CH_2)_2$-(S) | $-COR_6$ | $-CH(CH_3)_2$ | $NR_7R_8$ | $CH_3$, 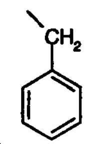 | - | 0,92 (t,3H) ; 1,24-1,29 (2d, 6H) ; 1,40 (m, 2H) ; 1,70 (m, 2H) ; 2,86 et 2,93 (2s,3H); 3,40 (m, 1H); 3,60 (m,2H) ; 4,50 (m, 1H) ; 4,60 (s) et 4,72 (m) 2H ; 7,08 (d,2H) ; 7,14 (t, 1H) ; 7,24 (d,1 H) ; 7,39 (m, 4H) ; 8,55 (d, 1H) ; 12,35 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 205. | 3,5-difluorophényle | H, H | $CH_3$ $(CH_2)_2$-(S) | $-COR_6$ | $-CH(CH_3)_2$ | $NR_7R_8$ | $CH_3$, 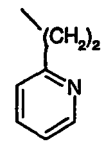 | - | 0,92 (t,3H) ; 1,24 (d,6H) ; 1,32 et 1,41 (2m,2H) ; 1,68 (m,2H) ; 2,86 et 3,04 (2s,3H); 3,07-3,18 (m, 2H) ; 3,36 (m,1 H) ; 3,60 (m, 2H) ; 3,84 (m,1 H) ; 4,51 (m,1 H) ; 7,03 (d, 2H) ; 7,11 (t, 1H) ; 7,2-7,4 (m,2H) ; 7,66 et 7,73 (2t,1H) ; 8,45-8,57 (m, 2H) ; 12,27 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 206. | 3,5-difluorophényle | H, H | $CH_3(CH_2)_2$-(S) | $-COR_6$ | 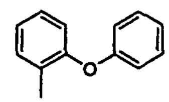 | $OCH_3$ | - | - | 0,91 (t,3H) ; 1,33-1,40 (2m, 2H); 1,67 (m, 2H) ; 3,61 (m, 2H) ; 3,70 (s, 3H) ; 4,47 (m, 1H) ; 6,93 (d, 2H) ; 7,03 (t, 3H) ; 7,14 (t, 2H) ; 7,30 (t, 1H) ; 7,39 (t, 2H) ; 7,48 (t, 1H) ; 7,54 (t, 1H) ; 8,54 (d,1 H) ; 12,78 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 207. | 3,5-difluorophényle | H, H | $CH_3(CH_2)_2$-(S) | $-COR_6$ | 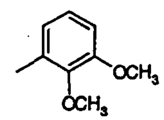 | $OCH_3$ | - | - | 0,92 (t,3H) ; 1,32-1,41 (2m, 2H) ; 1,67 (m, 2H) ; 3,60 (s + m,5H) ; 3,66 (s, 3H) ; 3,88 (s, 3H) ; 4,48 (m,1 H) ; 6,93 (d,1 H) ; 7,03 (d, 2H) ; 7,17 (m, 3H) ; 8,56 (d,1 H) ; 12,70 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 208. | 2-benzyloxy-3,5-difluorophényle | OH, H | $CH_3(CH_2)_2$-(S) | $-COR_6$ | $-CH(CH_3)_2$ | $OCH_3$ | - | - | 0,92 (t,3H) ; 1,31 (d,6H) ; 1,35 (m,2H) ; 1,79 (m, 2H) ; 3,86 (s,3H) ; 4,03 (m, 1H) ; 4,59 (m, 1H) ; 5,32 (d,2H) ; 5,32 (s, 1H) ; 6,80 (m, 1H) ; 7,01 (m, 1H) ; 7,25 (m, 1H) ; 7,40 (m, 5H) ; 12,59 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 209. | 2-hydroxy-3,5-difluorophényle | OH, H | $CH_3(CH_2)_2$-(S) | $-COR_6$ | $-CH(CH_3)_2$ | $OCH_3$ | - | - | 0,94 (t,3H) ; 1,33 (d,6H) ; 1,34 (m,2H) ; 1,82 (m,2H) ; 3,84 (s, 3H) ; 4,04 (m, 1H) ; 4,60 (m,1 H) ; 5,30 (m, 1H) ; 6,71 (m, 1H) ; 6,80-7,20 (m, 2H) ; 8,15 et 8,20 (2d, 1H) ; 9,76 (d, large 1H) ; 12,57 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 210. | 3,5-difluorophényle | H, H | $CH_3$ $(CH_2)_2$-(S) | $-COR_6$ | $-CH(CH_3)_2$ | $NR_7R_8$ | $CH_3$, $-(CH_2)_2OCH_3$ | - | 0,93 (t,3H) ; 1,32 (d,6H) ; 1,34-1,41 (2m, 2H) ; 1,70 (m, 2H); 2,94 et 3,01 (2s,3H); 3,20 et 3,30 (2s,3H) ; 3,32-3,67 (4m, 7H) ; 4,48 (m, 1H) ; 7,04 (d, 2H) ; 7,13 (t, 1H) ; 8,55 (s, large 1H) ; 12,28 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 211. | 3,5-difluorophényle | H, H | $CH_3(CH_2)_2$-(S) | $-COR_6$ | 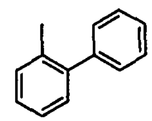 | $OCH_3$ | - | - | 0,91 (t,3H) ; 1,33 (m,2H) ; 1,67 (m,2H) ; 3,61 (s,3H) ; 3,64 (m,2H) ; 4,43 (m,1H) ; 7,04 (m,2H) ; 7,14 (m,1H) ; 7,14 (m,1H) ; 7,22 (m,2H) ; 7,28 (m,1H) ; 7,35 (m,2H) ; 7,48 (m,2H) ; 7,57 (m,1H) ; 8,60 (s, large 1 H) ; 12,65 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 212. | 3,5-difluorophényle | H, H | $CH_3(CH_2)_2$-(S) | $-COR_6$ | $-CH(CH_3)_2$ | $NR_7R_8$ | $CH_3$, 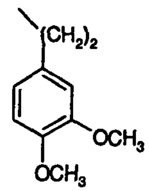 | - | 0,87 (t,3H) ; 1,13 (2d,6H) ; 1,16-1,18 (2m, 2H) ; 1,63 (m, 2H) ; 2,80 (m, 2H) ; 2,82 et 2,98 (2s,3H) ; 2,97 (m,1 H) ; 3,15 (m,1H); 3,38 (m,1H); 3,60 (m,2H) ; 3,60-3,65-3,71 et 3,74 (4s, 6H) ; 4,44 (m, 1H) ; 6,58 (m, 1H); 6,77 (m, 1H) ; 6,86 (m,1 H) ; 6,97 (m, 2H) ; 7,06 (m, 1H) ; 8,46 (s, large 1 H) ; 12,23 (s, large 1 H) |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 213. | $(CH_3)_3C$- | OH, H (S) | $CH_3(CH_2)_2$-(S) | $-COR_6$ | 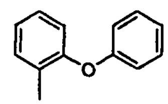 | $OCH_3$ | - | - | 0,91 (t,3H) ; 0,93 (s,9H) ; 1,34 (m,2H) ; 1,77 (m,2H) ; 3,61 (d,2H) ; 3,71 (s,3H) ; 4,58 (m,1H) ; 5,65 (d,1H) ; 6,93 (d,2H) ; 7,03 (d,1H) ; 7,13 (t,1H) ; 7,29 (t,1H) ; 7,36 (t,2H) ; 7,48 (t,1 H) ; 7,51 (d, 1H) ; 7,86 (d,1 H) ; 12,66 (s, large 1 H)*** |

(suite)

| N° | R1 | R2,R'2 | R3 | R4 | R5 | R6 | R7, R8 | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 214. | 3,5-difluorophényle | H, H | $CH_3(CH_2)_2$-(S) | $-COR_6$ | 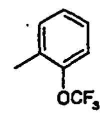 | $OCH_3$ | - | - | 0,93 (t,3H) ; 1,35-1,40 (2m, 2H) ; 1,70 (m, 2H) ; 3,60 (m, 2H) ; 3,68 (s, 3H) ; 4,50 (m, 1H) ; 7,03 (d, 2H) ; 7,12 (t, 1H) ; 7,55 (m, 2H) ; 7,63 (m, 2H) ; 8,57 (s, large 1 H) ; 12,88 (s, large 1 H)*** |
| 215. | 3,5-difluorophényle | OH, H (S) | $CH_3(CH_2)_2$-(S) | $-COR_6$ | 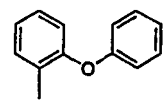 | $OCH_3$ | - | - | 0,85 (t,3H) ; 1,26-1,29 (2m, 2H) ; 1,75 (m, 2H); 3,68 (s, 3H); 4,47 (m, 1H); 5,09 (s, 1H); 6,58 (s, 1H); 6,92 à 7,52 (7m,12H); 8,30 (s, large 1H); 12,68 (s, large 1H)*** |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 216. | $(CH_3)_3C$- | OH, H (S) | $CH_3$ $(CH_2)_2$-(S) | $-COR_6$ | 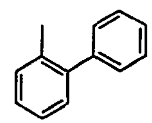 | $OCH_3$ | - | - | 0,91 (t,3H) ; 1,02 (s,9H) ; 1,35 (m,2H) ; 1,75 (m,2H) ; 3,58 (s,3H) ; 3,59 (s, 1H) ; 4,54 (m,1H) ; 5,66 (s, 1H) ; 7,20-7,57 (5m, 9H) ; 7,83 (d, 1H) ; 12,55 (s, large 1H)*** |
| 217. | 3,5-difluorophényle | OH, H | $CH_3$ $(CH_2)_2$-(S) | $-COR_6$ | 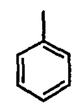 | $NR_7R_8$ | H, H | - | 0,91 (t,3H) ; 1,29 (m,2H) ; 1,79 (m,2H) ; 4,58 (m, 1H) ; 5,15 (d,1 H) ; 6,48 et 6,63 (2s,1H) ; 7,12 (m,3H) ; 7,42 (m,4H) ; 7,58 (m,1H) ; 7,59 (s,2H) ; 8,34 et 8,40 (2d,1H) ; 12,41 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 218. | 3,5-difluorophényle | H, H | $CH_3(CH_2)_2$-(S) | $-COR_6$ | $-CH(CH_3)_2$ | 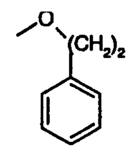 | - | - | 0,92 (t,3H) ; 1,25 (d,6H) ; 1,38 (m,2H) ; 3,05 (m,2H); 3,61 (m,2H); 3,88 (m,1H); 4,43 (m,1H); 4,51 (m,2H); 7,04 (d,2H) ; 7,15 (t,1H); 7,27 (m,1 H) ; 7,36 (s,4H) ; 8,52 (d,1H); 12,55 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 219. | $(CH_3)_2CH-$ | OH, H (S) | $CH_3(CH_2)_2$-(S) | $-COR_6$ | 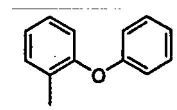 | $OCH_3$ | - | - | - 0,80 (d,3H); 0,87 (t,3H) ; 0,90 (d,3H) ; 1,27 (m,2H); 1,70 (m,2H); 1,98 (m,1 H) ; 3,65 (s,3H); 3,74 (m,1H); 5,48 (d,1 H) ; 6,88 (d,2H) ; 6,97 (d,1 H) ; 7,09 (t,1 H) ; 7,24 (t,1H); 7,20 (m,2H); 7,30 (m,1 H) ; 7,34 (d,1H) ; 7,85 (d,1H) ; 12,63 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 220. | 3,5-difluorophényle | H, H | $CH_3(CH_2)_2$-(S) | $-COR_6$ | 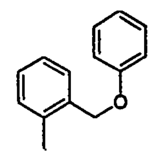 | $OCH_3$ | - | - | 0,86 (t,3H) ; 1,32 (m,2H) ; 1,63 (m,2H) ; 3,55 (m,2H) ; 3,59 (s,3H) ; 4,43 (m,1H) ; 4,91 (s,2H) ; 6,83 (d,2H) ; 6,86 (m,1 H) ; 6,99 (d,2H) ; 7,07 (m,1H) ; 7,18 (m,2H) ; 7,37 (t,1H) ; 7,40 (t,1 H) ; 7,47 (t,1 H) ; 7,57 (d,1H) ; 8,52 (d,1H) ; 12,62 (s,1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 221. | $(CH_3)_3C$- | OH, H (S) | $CH_3(CH_2)_2$-(S) | $-COR_6$ | 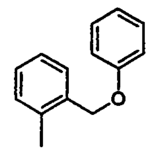 | $OCH_3$ | - | - | 0,87 (t,3H) ; 0,90 (s,9H) ; 1,28 (m,2H) ; 1,70 (m,2H) ; 3,55 (s,1 H) ; 3,59 (s,3H) ; 4,53 (m,1H) ; 4,91 (s,2H) ; 5,59 (d,1 H) ; 6,83 (d,2H) ; 6,90 (t, 1H) ; 7,22 (m,2H) ; 7,34 (d,1 H) ; 7,40 (t, 1H); 7,45 (t,1 H) ; 7,59 (t, 1H) ; 7,82 (d,1 H) ; 12,67 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 222. | $(CH_3)_3C$- | OH, H (S) | $CH_3(CH_2)_2$-(S) | $-COR_6$ | 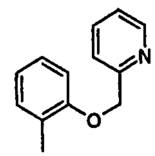 | $OCH_3$ | - | - | 0,96 (t,3H) ; 0,99 (s,9H) ; 1,44 (m,2H) ; 1,77 (m,2H) ; 3,38 (s, 3H); 3,64 (s,1 H) ; 4,64 (m,1H) ; 5,26 (s, 2H); 5,68 (d,1 H) ; 7,12 (t,1H) ; 7,24 (d, 1H); 7,34 (d, 1H); 7,38 (m,1H) ; 7,44 (m, 2H); 7,82 (t,1H) ; 7,90 (d,1H) ; 8,62 (d,1 H) ; 12,68 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 223. | 3,5-difluorophényle | H, H | $CH_3(CH_2)_2$-(S) | $-COR_6$ | 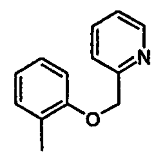 | $OCH_3$ | - | - | 0,99 (t,3H) ; 1,46 (m,2H) ; 1,77 (m,2H) ; 3,63 (s,3H) ; 3,66 (m,2H) ; 4,54 (m,1H) ; 5,28 (s,2H) ; 7,10-7,48 (plusieurs m, 9H) ; 7,83 (t, 1H); 8,63 (m, 2H) ; 12,80 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 224. | $(CH_3)_3C-$ | OH, H (S) | $CH_3(CH_2)_2$-(S) | $-COR_6$ | 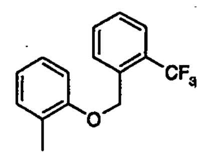 | $OCH_3$ | - | - | 0,85 (t,3H) ; 0,91 (s,9H) ; 1,29 (m,2H) ; 1,68 (m,2H) ; 3,53 (s,3H) ; 3,56 (m,2H) ; 4,52 (m,1 H) ; 5,24 (s,2H) ; 5,58 (d;1 H) ; 7,05 (t,1 H) ; 7,13 (d,1 H) ; 7,36 (d,1 H) ; 7,41 (t,1H) ; 7,54 (d,1H) ; 7,62 (t,1 H) ; 7,73 (d,1 H) ; 7,81 (d,1 H) ; 12,55 (s,1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 225. | $(CH_3)_3C$- | OH, H (S) | $CH_3(CH_2)_2$-(S) | -$COR_6$ | 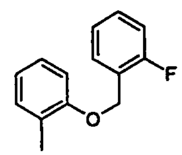 | $OCH_3$ | - | - | 0,87 (t,3H) ; 0,91 (s,9H) ; 1,28 (m,2H) ; 1,70 ; (m,2H) ; 3,56 (s,2H) ; 3,56 (s,3H) ; 4,54 -(m,1 H) ; 5,15 (s,2H) ; 5,60 (d,1 H) ; 7,05 (t,1H) ; 7,20 (m,3H) ; 7,33 (m,3H) ; 7,39 (m,1 H) ; 7,81 (d,1 H) ; 12,54 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 226. | $(CH_3)_3C$- | OH, H (S) | $CH_3(CH_2)_2$-(S) | $-COR_6$ | 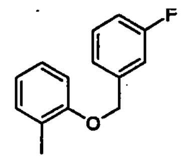 | $OCH_3$ | - | - | 0,96 (t,3H) ; 0,99 (s,9H) ; 1,35 (m,2H) ; 1,80 (m,2H) ; 3,64 (d,1 H) ; 3,65 (s,3H) ; 4,63 (m,1H) ; 5,23 (s,2H) ; 5,68 (d,1H) ; 7,12-7,22 (m, 5H) ; 7,45 (m, 3H) ; 7,92 (d,1 H) ; 12,67 (s, large 1 H)*** |
| 227. | 3,5-difluorophényle | H, H | $CH_3(CH_2)_2$-(S) | $-COR_6$ | 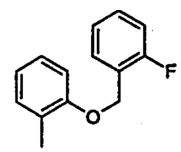 | $OCH_3$ | - | - | 0,87 (t,3H) ; 1,32 (m,2H) ; 1,64 (m,2H) ; 3,57 (s,3H) ; 3,60 (m,2H) ; 4,43 (m,1 H) ; 5,14 (s,2H) ; 6,98-7,40 (m, 11 H) ; 8,51 (d, 1 H) ; 12,63 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 228. | 3,5-difluorophényle | H, H | $CH_3(CH_2)_2$-(S) | $-COR_6$ | 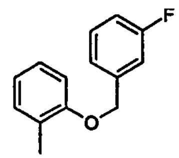 | $OCH_3$ | - | - | 0,95 (t,3H) ; 1,43 (m,2H) ; 1,71 (m,2H) ; 3,66 (m+s, 5H) ; 4,50 (m, 1H) ; 5,18 (s, 2H) ; 7,06-7,19 (m,8H) ; 7,44 (m,3H) ; 8,64 (d,1H) ; 12,72 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 229. | 3,5-difluorophényle | OH, H | $CH_3(CH_2)_2$-(S) | $-COR_6$ | 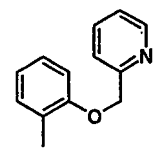 | $OCH_3$ | - | - | 0,83 (t,3H) ; 1,25 (m,2H) ; 1,69 (m,2H) ; 3,55 (s,3H) ; 4,45 (m,1H) ; 5,09 (m,1H) ; 5,16 (s,2H) ; 6,44 et 6,58 (2d,1H) ; 7,02 (t,1H) ; 7,14 (m,4H) ; 7,24-7,37 (m, 4H) ; 7,33 (m, 1H) ; 8,30 et 8,32 (2d,1H) ; 8,52 (s,1H) ; 12,63 et 12,71 (2s,large 1H) *** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 230. | $(CH_3)_3C$- | OH, H (S) | $CH_3(CH_2)_2$-(S) | -$COR_6$ | 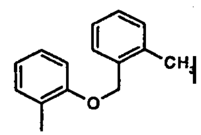 | $OCH_3$ | - | - | 0,97 (t,3H) ; 1,02 (s,9H) ; 1,36 (m,2H) ; 1,80 (m,2H) ; 2,30 (s,3H) ; 3,65 (s,3H) ; 3,67 (m,2H) ; 4,65 (m,1H) ; 5,18 (s,2H) ; 5,70 (d, 1H); 7,10-7,50 (5m, 8H) ; 7,96 (d, 1H); 12,68 (s, large 1 H)*** |
| 231. | 3,5-difluorophényle | H, H | $CH_3(CH_2)_2$-(S) | -$COR_6$ | 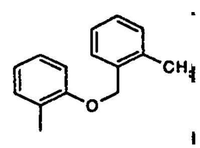 | $OCH_3$ | - | - | 0,96 (t,3H) ; 1,39 (m,2H) ; 1,71 (m, 2H); 2,36 (s,3H) ; 3,60 (s,3H) ; 3,62 (m, 2H); 4,50 (m,1H) ; 5,16 (s,2H) ; 7,06-7,48 (6m, 11H) ; 8,67 (d, 1H); 12,77 (s, 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 232. | $(CH_3)_3C$- | OH, H (S) | $CH_3(CH_2)_2$-(S) | $-COR_6$ | 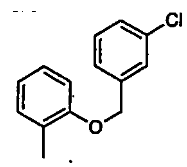 | $OCH_3$ | - | - | 0,95 (t,3H) ; 0,99 (s,9H) ; 1,37 (m, 2H); 1,81 (m,2H) ; 3,64 (m,2H) ; 3,67 (s,3H) ; 4,63 (m, 1H) ; 5,22 (s,2H) ; 5,71 (d,1H); 7,10 (t,1H) ; 7,22 (d,1H) ; 7,33 (d,1H) ; 7,40-7,50 (m, 5H) ; 7,94 (d, 1H) ; 12,62 (s, large 1H)*** |
| 233. | $CF_3$ | OH, H (S) | $CH_3(CH_2)_2$-(S) | $-COR_6$ | 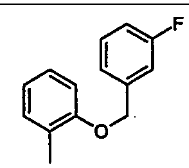 | $OCH_3$ | - | - | 0,96 (t,3H) ; 1,37 (m,2H) ; 1,82 (m,2H) ; 3,66 (s,3H) ; 4,60 (m,1 H) ; 4,72 (m,1 H) ; 5,23 (s,2H); 7,01-7,50 (2m, 8H) ; 8,62 (d, 1H); 12,86 (s, large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 234. | 3,5-difluorophényle | H, H | $CH_3(CH_2)_2$-(S) | $CH_3$ | $-COR_6$ | $NR_7R_8$ | $CH_3$, $CH_3$ | - | 0,96 (t,3H) ; 1,40 (m,2H) ; 1,72 (m,2H) ; 2,32 (s,3H); 3,06 (s,6H) ; 3,62 (m,2H) ; 4,52 (m, 1H); 7,08 (d,2H) ; 7,16 (t,1H); 8,58 (d, 1 H); 12,42 (s,large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 235. | 3,5-difluorophényle | H, H | $CH_3(CH_2)_2$-(S) | $CH_3$ | $-COR_6$ | $NR_7R_8$ | H, 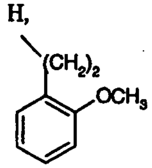 | - | 0,85 (t,3H) ; 1,27 (m,2H) ; 1,62 (m,2H) ; 2,41 (s,3H) ; 2,78 (t,2H) ; 3,37 (t,2H); 3,55 (m,2H); 3,78 (s,3H) ; 4,43 (m,1H); 6,87 (t,1H); 7,08 (m,3H) ; 7,11 (m,2H); 7,20 (t, 1H); 7,95 (s,large 1H); 8,51 (d,1 H) ; 12,38 (s, large 1 H) |

(suite)

| N° | $R_1$ | $R_2$,R'$_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 236. | 3,5-difluorophényle | H, H | $CH_3$ $(CH_2)_2$-(S) | H | $-COR_6$ | $OCH_3$ | - | - | 0,93 (t,3H); 1,30 et 1,41 (2m,2H); 1,70 (m,2H) ; 3,61 (m,2H); 3,85 (s, 3H); 4,53 (m,1 H) ; 7,04 (d, 2H); 7,17 (t,1 H) ; 8,21 (s, 1H) ; 8,63 (d, 1H); 12,82 (s, large 1 H)*** |
| 237. | $(CH_3)_3C$- | OH, H (S) | $CH_3$ $(CH_2)_2$-(*S*) | H | $-COR_6$ | $NR_7R_8$ | $CH_3$, 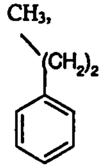 | - | 0,91 (t,3H) ; 0,95 (s,9H) ; 1,32 et 1,35 (2m,2H) ; 1,74 (m,2H) ; 2,90 (m,2H) ; 3,18 (s,large 3H) ; 3,60 (d,1H) ; 3,71 (m,2H) ; 3,61 (m,1H) ; 5,63 (d,1H) ; 7,22-7,35 (m, 5H) ; 7,90 (d, 1H) ; 12,49 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 238. | $(CH_3)_3C$- | OH, H (S) | $CH_3(CH_2)_2$-(*S*) | $-COR_6$ | 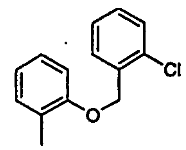 | $OCH_3$ | - | - | 0,96 (t,3H) ; 1,14 (s,9H) ; 1,39 (m,2H) ; 1,82 (m,2H) ; 3,65 (s,3H) ; 3,67 (s,1H) ; 4,64 (m,1H) ; 5,27 (s,2H) ; 5,73 (d,1H) ; 7,16 (m,1H) ; 7,28 (d,1H) ; 7,40-7,50 (4m, 6H) 8,01 (d, 1H); 12,70 (s, large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 239. | $(CH_3)_3C$- | OH, H (S) | $CH_3(CH_2)_2$-(S) | $-COR_6$ | 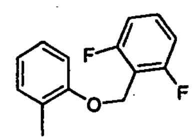 | $OCH_3$ | - | - | 0,94 (t,3H) ; 0,97 (s,3H) ; 1,34 (m,2H) ; 3,62 (s,3H) ; 3,63 (s,1H) ; 5,67 (m,2H) ; 5,15 (s,2H) ; 7,15 (m,3H) ; 7,41 (d,2H) ; 7,50 (m,2H) ; 7,89 (d,1H) ; 12,67 (s large 1H)*** |
| 240. | $(CH_3)_3C$- | OH, H (S) | $CH_3(CH_2)_2$-(S) | $-COR_6$ | 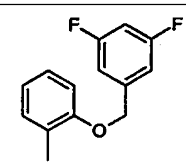 | $OCH_3$ | - | - | 0,97 (t,3H) ; 0,99 (s,9H) ; 1,39 (m,2H) ; 1,80 (m,2H) ; 3,65 (s,3H) ; 3,66 (s,1H) ; 4,63 (m,1 H) ; 5,24 (s,2H) ; 5,70 (d,1 H) ; 7,05-7,27 (3m, 5H) ; 7,45-7,49 (2m,2H) ; 7,93 (d,1 H) ; 12,70 (s large 1H)*** |

(suite)

| N° | $R_1$ | $R_2,R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 241 | $(CH_3)_3C$- | OH, H (S) | $CH_3(CH_2)_2$-(S) | $-COR_6$ | 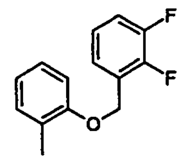 | $OCH_3$ | - | - | 0,87 (t,3H) ; 0,91 (s,9H) ; 1,30 (m,2H) ; 1,73 (m,2H) ; 3,56 (2s,4H) ; 4,54 (m,1 H) ; 5,19 (s,2H) ; 5,60 (d,1H) ; 7,06 (m,1H) ; 7,16 (m,2H) ; 7,23 (d,1H) 7,32-7,41 (m, 3H) ; 7,82 (d,1 H) ; 12,70 (s large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 242. | $(CH_3)_3C$- | OH, H (S) | $CH_3(CH_2)_2$-(S) | $-COR_6$ | 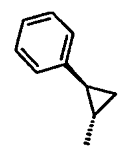 | $OCH_3$ | - | - | 0,87 (t,3H) ; 0,91 (s,9H) ; 1,27 (m,2H) ; 1,45 (m,3H) ; 1,69 (m,1 H) ; 2,32 (m,1 H) ; 3,13 (m,1H) ; 3,56 (d,1H) ; 3,70 (s,3H) ; 4,52 (m,1H) ; 5,57 (d,1H) ; 7,19 (m,3H) ; 7,29 (m,2H) ; 7,80 (d,1H) ; 12,47 (s large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 243. | 3,5-difluorophényle | H, H | $CH_3(CH_2)_2$-(S) | $-COR_6$ | 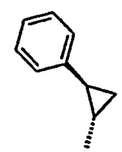 | $OCH_3$ | - | - | 0,95 (t,3H) ; 1,36-1,41 (m, 2H) ; 1,51 (m, 1H) ; 1,72 (m, 3H) ; 2,38 (m, 1H) ; 3,20 (m, 1H) ; 3,63 (m, 2H) ; 3,76 (s, 3H) ; 7,26 (m, 3H) ; 7,39 (m, 2H) ; 8,54 (s large 1 H) ; 12,65 (s large 1 H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 244. | 3,5-difluorophényle | H, H | CH3 $(CH_2)_2$-(S) | $-COR_6$ | 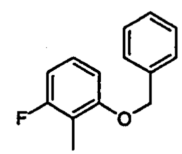 | $OCH_3$ | - | - | 0,94 (t,3H) ; 1,39 (m,2H) ; 1,70 (m,2H) ; 3,62 (m+s, 5H) ; 4,48 (m, 1H) ; 4,91 (s, 2H) ; 7,04 (d, 2H) ; 7,12 (m, 3H) ; 7,22 (m, 5H) ; 7,40 (m, 1H) ; 8,57 (d large 1H) ; 12,77 (s large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 245. | $(CH_3)_3C$- | OH, H (S) | $CH_3(CH_2)_2$-(S) | $-COR_6$ | 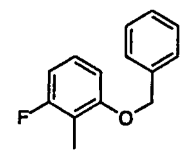 | $OCH_3$ | - | - | 0,96 (t,3H) ; 0,98 (s,9H) ; 1,39 (m,2H) ; 1,80 (m,2H) ; 3,65 (m,1H) ; 3,66 (s,3H) ; 4,64 (m,1H) ; 4,95 (s,2H) ; 5,65 (s,1H) ; 7,15 (m,2H) ; 7,27 (m,5H) ; 7,45 (m,1H) ; 7,91 (d,1 H) ; 12,70 (s large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 246. | $(CH_3)_3C$- | OH, H (S) | $CH_3(CH_2)_2$-(S) | -$COR_6$ | 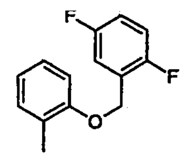 | $OCH_3$ | - | - | 0,95 (t,3H); 0,98 (s,9H); 1,37 (m,2H); 1,80 (m,2H) ; 3,63 (d+s,5H) ; 4,61 (m,1 H) ; 5,22 (s,2H) ; 5,66 (d,1H) ; 7,12 (t,1H) ; 7,16 (m,1 H) ; 7,29 (m,3H); 7,40 (d,1H) ; 7,50 (m,1H); 7,89 (d,1H); 12,65 (s large 1H)*** |

(suite)

| N° | $R_1$ | $R_2$,$R'_2$ | $R_3$ | $R_4$ | $R_5$ | $R_6$ | $R_7$, $R_8$ | alpha D (C = 1, MeOH) | RMN (DMSO d6 quand non précisé) * signifie 300MHz - ** signifie 360MNz -*** signifie 500MHz |
|---|---|---|---|---|---|---|---|---|---|
| 247. | 3,5-difluorophényle | OH, H | $CH_3$ $(CH_2)_2$-(S) | H | $-COR_6$ | $NR_7R_8$ | 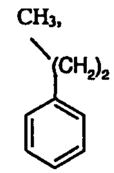 | - | 0,82 (t,3H) ; 1,16-1,28 (m, 2H) ; 1,67 (m, 2H) ; 2,86 (s large 2H) ; 3,29 (s large 3H) ; 3,66 (s large 2H) ; 4,52 (m, 1H) ; 5,08 (2d, 1H) ; 6,40 et 6,55 (2d,1H) ; 7,09-7,28 (3m, 8H) ; 7,60 (s large 1H); 8,27 et 8,34 (2d, 1H) ; 12,44 (s large 1H)**** |

Dans le tableau, (S) ou (R) dans les colonnes « $R_3$ » et «$R_2$, $R_{2'}$ » indiquent la stéréochimie du carbone asymétrique, portant $R_3$ ou $R_2$, dans la formule (I). Pour le carbone portant $R_2$, l'indication (S) ou (R) ne concerne pas le cas où $R_2$ et $R_{2'}$ forment ensemble un groupe oxo.

Les composés de l'invention ont fait l'objet d'essais pharmacologiques qui ont montré leur intérêt comme substances actives en thérapeutique.

Ils ont en particulier été testés quant à leurs effets d'inhibition de la production du peptide β-amyloïde (β-A4).

Le peptide β-amyloïde (β-A4) est un fragment d'une protéine précurseur plus importante appelée APP (Amyloid Precursor Protein). Cette dernière est produite et présente dans différentes cellules de tissu animal ou humain. Toutefois, son clivage, dans le tissu cérébral, par des enzymes de type protéase, conduit à la formation du peptide β-A4 qui s'accumule sous forme de plaque amyloïde. Les deux protéases responsables de la production du peptide amyloide sont connues sous le nom de beta et gamma-secrétases (Wolfe MS, Secretase targets for Alzheimer's disease: identification and therapeutic potential, J Med Chem. 2001 Jun 21;44(13):2039-60).

Or il a été démontré que ce dépôt progressif du peptide β-A4 est neurotoxique et pourrait jouer un rôle important dans la maladie d'Alzheimer.

Ainsi, les composés de la présente invention, en tant qu'inhibiteur de la production du peptide β-amyloïde (β-A4) par inhibition de la gamma protéase, peuvent être utilisés dans le traitement de pathologies comme la démence sénile, la maladie d'Alzheimer, le syndrome de Down, la maladie de Parkinson, l'angiopathie amyloïde et/ou les désordres cérébro-vasculaires.

Les tests ont été réalisés selon le protocole décrit ci-après.

Pour l'essai cellulaire □ amyloïde, la lignée CHO-K1 coexprimant le CT100 de APP et PS1 M146L clone 30-12 est utilisée. La lignée cible l'inhibition de gamma secrétase. La préséniline est liée à l'activité gamma-secrétase (Wolfe MS, Haass C., The Role of presenilins in gamma-secretase activity, J Biol Chem. 2001 Feb 23;276(8):5413-6) et sa coexpression avec la protéine amyloïde ou son fragment N-terminal entraîne une augmentation de secrétion du peptide A1-42 (β-A4) générant ainsi un outil pharmacologique permettant d'évaluer l'inhibition par les composés de formule (1) de la production du peptide β-A4. L'ensemencement des plaques de culture de 96 puits est réalisé à raison de $1x10^5$ cellules par puits dans 150□l de milieu d'incubation. La présence d'un pourcentage minimal (1,3% final) de sérum permet l'adhésion cellulaire au plastique après 2-3 heures d'incubation à 37°C, en présence de 5% $CO_2$. Les produits (15μl) sont testés à 10μM DMSO 1% final et sont incubés durant 24-25h à 37°C en présence de 5% $CO_2$ et de 100% d'humidité. Après cette incubation de 24-25h, les surnageants cellulaires (100μl) sont transférés dans les plaques ELISA, traitées avec l'anticorps de capture 6E10 (6E10, épitope: aa1-17, INTERCHIM/SENETEK 320-10), pour déterminer le taux de peptides amyloïdes sécrété par les cellules en présence de composés selon l'invention. Une gamme de peptide contrôle, « peptide 1-40 », synthétique à 5 et 10 ng/ml est traitée parallèlement. Les plaques ELISA sont incubées pendant une nuit à 4°C.

La quantité de peptide fixé est détectée de façon indirecte en présence d'un compétiteur correspondant au peptide tronqué, le peptide 1-28 couplé à la biotine qui est ensuite détectée par la streptavidine couplée à la phosphatase alcaline. Le substrat, le p-Nitrophényl Phosphate (pNPP *FAST* p-Nitrophényl Phosphate, Sigma N2770), donne un produit de réaction soluble jaune lisible à 405nm. La réaction est stoppée avec une solution d'EDTA 0,1 M. Pour cela, après fixation du peptide amyloïde dans la plaque ELISA, 50μl de peptide 1-28 biotinylé sont ajoutés aux 100μl de surnageant cellulaire et incubés 30 minutes à température ambiante. Les plaques ELISA sont ensuite lavées 3 fois. Après séchage par inversion sur papier absorbant, 100μl de streptavidine-Alcaline Phosphatase (Interchim/Jackson ImmunoResearch Laboratories 016-050-084), sont ajoutés par puits et incubés 1 heure à température ambiante. Les plaques sont à nouveau lavées puis le substrat de la phosphatase alcaline (pNPP 1mg/ml) est ajouté à raison de 100μl par puits. Après une incubation de 30 minutes à température ambiante, la réaction est stoppée par ajout de 100μl par puits d'EDTA 0,1 M et la lecture est effectuée à 405nm.

Les composés de formule (I) selon la présente invention ont montré une CI50 (concentration inhibitrice à 50%) inférieure à 500 nM, plus particulièrement inférieure à 100 nM.

Les résultats des tests biologiques montrent que les composés sont des inhibiteurs de la formation du peptide β-amyloïde (β-A4).

Ainsi, ces composés peuvent être employés dans le traitement des pathologies dans lesquelles un inhibiteur de la formation du peptide β-amyloïde (β-A4) apporte un bénéfice thérapeutique. Notamment de telles pathologies sont la démence sénile, la maladie d'Alzheimer, le syndrome de Down, la maladie de Parkinson, l'angiopathie amyloïde et les désordres cérébro-vasculaires.

L'utilisation des composés selon l'invention pour la préparation d'un médicament destiné à traiter les pathologies ci-dessus mentionnées fait partie intégrante de l'invention.

L'invention a également pour objet des médicaments qui comprennent un composé de formule (I), ou un sel d'addition de ce dernier à un acide pharmaceutiquement acceptable ou encore un hydrate ou un solvat du composé de formule (I). Ces médicaments trouvent leur emploi en thérapeutique, notamment dans le traitement des pathologies ci-dessus mentionnées.

Selon un autre de ses aspects, la présente invention concerne des compositions pharmaceutiques renfermant en tant que principe actif, au moins un composé selon l'invention. Ces compositions pharmaceutiques contiennent une dose efficace d'un composé selon l'invention, ou d'un sel pharmaceutiquement acceptable, d'un hydrate ou d'un solvat dudit composé, et éventuellement un ou plusieurs excipients pharmaceutiquement acceptables. Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, son sel, son solvat ou son hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules, les chewing-gums et les solutions ou suspensions orales, les formes d'administration sublinguale, buccale, intratrachéale, inraoculaire, intranasale, par inhalation, les formes d'administration sous-cutanée, intramusculaire ou intraveineuse et les formes d'administration rectale ou vaginale. Pour l'application topique, on peut utiliser les composés selon l'invention dans des crèmes, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Afin d'obtenir l'effet prophylactique ou thérapeutique désiré, la dose de principe actif peut varier entre 0,1 mg et 200 mg par kg de poids du corps et par jour. Bien que ces dosages soient des exemples de situation moyenne, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, le poids et la réponse dudit patient.

Chaque dose unitaire peut contenir de 0,1 à 1000 mg, de préférence de 0,1 à 500 mg, de principe actif en combinaison avec un ou plusieurs excipients pharmaceutiques. Cette dose unitaire peut être administrée 1 à 5 fois par jour de façon à administrer un dosage journalier de 0,5 à 5000 mg, de préférence de 0,5 à 2500 mg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule générale (I) :

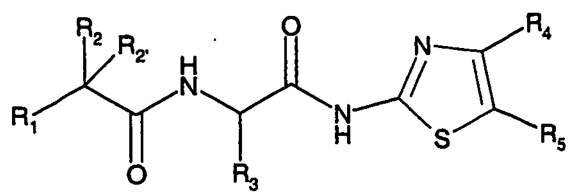

(I)

dans laquelle,

$R_1$ représente :

un $C_{1-6}$ alkyle éventuellement substitué par un à trois substituants choisis parmi un halogène, un trifluorométhyle, un hydroxy, un $C_{1-6}$ alcoxy, un $C_{1-6}$ thioalkyle, un thiophène ou un phényle ; ou
un $C_{3-7}$ cycloalkyle, un thiophène, un benzothiophène, un pyridinyle, un furanyle ou un phényle ;
les groupes phényle étant éventuellement substitués par un à trois substituants choisis parmi un atome d'halogène, un $C_{1-6}$-alkyle, un $C_{1-6}$ alcoxy, un hydroxy, un méthylènedioxy, un phénoxy ou un benzyloxy ;

$R_2$ et $R'_2$ représentent indépendamment l'un de l'autre un atome d'hydrogène, un atome d'halogène, un hydroxy, un $C_{1-3}$ alcoxy, un $C_{1-3}$ alkyle, un $C_{3-7}$ cycloalkyle, un groupe O-C(O)-$C_{1-6}$ alkyle, ou $R_2$ et $R'_2$ forment ensemble un groupe oxo ;
$R_3$ représente un atome d'hydrogène, un $C_{1-6}$ alkyle éventuellement substitué par un hydroxy ou un $C_{1-3}$ alcoxy ;
$R_4$ et $R_5$ représentent, indépendamment l'un de l'autre :

un atome d'hydrogène, un $C_{1-7}$ alkyle éventuellement substitué par un $C_{3-7}$ cycloalkyle ou phényle ; ou
un $C_{3-7}$ cycloalkyle, un phényle, un naphtyle ou un -C(X)$R_6$;
les groupes $C_{3-7}$ cycloalkyle et phényle étant éventuellement substitués par un ou plusieurs groupes choisis parmi un atome d'halogène, un hydroxy, un $C_{1-3}$ alkyle, un $C_{1-3}$ alcoxy, un trifluorométhyle, un trifluorométhoxy, un phényle, un phénoxy, un benzyloxy, un -$CH_2$O-phényle, un -O$CH_2$-pyridinyle ;
le groupe benzyloxy étant éventuellement substitué par un à trois groupes choisis parmi un halogène, un trifluorométhyle, un méthyle;
à la condition qu'au moins un groupe $R_4$ ou $R_5$ représente un groupe -C(X)$R_6$; X représente un atome d'oxygène ou un atome de soufre ;

$R_6$ représente un groupe $C_{1-6}$ alcoxy, un hydroxy ou un groupe -N$R_7R_8$; le groupe $C_{1-6}$ alcoxy étant éventuellement substitué par un phényle ;
$R_7$ et $R_8$ représentent, indépendamment l'un de l'autre :

un atome d'hydrogène, un groupe $C_{1-6}$ alkyle éventuellement substitué par un $C_{3-7}$ cycloalkyle, un $C_{3-7}$ cycloalkènyle, $C_{1-3}$ alcoxy un phényle, un morpholinyle ou un pyridinyle ; ou
un $C_{3-7}$ cycloalkyle, $C_{1-6}$ alcoxy ou un phényle ; ou
$R_7$ et $R_8$, avec l'atome d'azote qui les porte, forment un cycle aziridine, azétidine, pyrrolidine, pipéridine ou morpholine ;
les groupes $C_{3-7}$ cycloalkyle et phényle étant éventuellement substitués par un ou deux groupes choisis parmi un $C_{1-3}$ alkyle, un hydroxy, un $C_{1-3}$ alcoxy ou un atome d'halogène ;

à l'état de base, de sel d'addition à un acide, d'hydrate ou de solvat.

**2.** Composé selon la revendication 1, **caractérisé en ce que** :

$R_1$ représente :

un $C_{1-5}$ alkyle éventuellement substitué par un à trois substituants choisis parmi un fluor, un trifluorométhyle, un hydroxy, un $C_{1-4}$ thioalkyle, un thiophène ou un phényle; ou
un $C_{4-7}$ cycloalkyle, un furanyle, un thiophène, un benzothiophène, un pyridinyle ou un phényle ; le phényle étant éventuellement substitué par un à trois substituants choisis parmi un atome d'halogène, un hydroxy, un benzyloxy ou un méthylènedioxy;

$R_2$ et $R'_2$ représentent indépendamment l'un de l'autre un atome d'hydrogène, un atome d'halogène, un hydroxy, un $C_{1-3}$ alkyle, un $C_{3-7}$ cycloalkyle, un $C_{1-3}$ alcoxy, un groupe O-C(O)-$C_{1-4}$ alkyle, ou $R_2$ et $R'_2$ forment ensemble un groupe oxo ;
$R_3$ représente un $C_{1-4}$ alkyle éventuellement substitué par un $C_{1-3}$ alcoxy ;
$R_4$ et $R_5$ représentent, indépendamment l'un de l'autre :

un atome d'hydrogène, un $C_{1-7}$ alkyle éventuellement substitué par un phényle ou un $C_{3-7}$cycloalkyle; ou un $C_{3-7}$ cycloalkyle, un phényle, un naphtyle ou un -C(X)$R_6$; le phényle étant éventuellement substitué par un ou deux groupes choisis parmi un halogène, un $C_{1-3}$ alkyle, un hydroxy, un $C_{1-3}$ alcoxy, un trifluorométhyle, un trifluorométhoxy, un phényle, un phénoxy, un benzyloxy, un -$CH_2$O-phényle, un -O$CH_2$-pyridinyle ;
le groupe benzyloxy étant éventuellement substitué par un groupe choisi parmi un halogène, un trifluorométhyle ou un méthyle ;

à la condition qu'au moins un groupe $R_4$ ou $R_5$ représente un groupe $-C(X)R_6$;
X représente un atome d'oxygène ;
$R_6$ représente un groupe $C_{1-6}$ alcoxy, un hydroxy ou un groupe $-NR_7R_{8;}$ le groupe $C_{1-6}$ alcoxy étant éventuellement substitué par un phényle ;
$R_7$ et $R_8$ représentent, indépendamment l'un de l'autre :

un atome d'hydrogène, un groupe $C_{1-3}$ alkyle éventuellement substitué par un $C_{1-3}$ alcoxy, un $C_{3-7}$ cycloalkènyle, un phényle, un morpholinyle ou un pyridinyle ; un $C_{1-3}$ alcoxy; ou
un $C_{3-6}$cycloalkyle ; ou
$R_7$ et $R_8$, avec l'atome d'azote qui les porte, forment un cycle azétidine, pipéridine ou morpholine ;
le groupe phényle étant éventuellement substitué par un ou deux groupes $C_{1-3}$ alcoxy ;

à l'état de base, de sel d'addition à un acide, d'hydrate ou de solvat.

**3.** Composé de formule (I) selon la revendication 1 **caractérisé en ce que** :

$R_1$ représente :

un $C_{1-6}$ alkyle éventuellement substitué par un ou deux substituants choisis parmi un hydroxy, un $C_{1-6}$ alcoxy, un $C_{1-6}$ thioalkyle, un trifluorométhyle, un thiophène, ou un phényle; ou
un $C_{3-7}$ cycloalkyle, un thiophène, un benzothiophène, un pyridinyle, un furanyle ou un phényle;
les groupes phényle étant éventuellement substitués par un à trois substituants choisis parmi un atome d'halogène, un $C_{1-6}$ alkyle, un $C_{1-6}$ alcoxy, un hydroxy, un méthylènedioxy, un phénoxy ou un benzyloxy;

$R_2$ et $R'_2$ représentent indépendamment l'un de l'autre un atome d'hydrogène, un atome d'halogène, un hydroxy, un $C_{1-3}$ alcoxy, un $C_{1-3}$ alkyle, un $C_{3-7}$ cycloalkyle, un groupe $O-C(O)-C_{1-6}$ alkyle, ou $R_2$ et $R'_2$ forment ensemble un groupe oxo ;
$R_3$ représente un atome d'hydrogène ou un $C_{1-6}$ alkyle éventuellement substitué par un hydroxy ou un $C_{1-3}$ alcoxy ;
$R_4$ et $R_5$ représentent, indépendamment l'un de l'autre :

un atome d'hydrogène, un $C_{1-7}$ alkyle éventuellement substitué par un $C_{3-7}$ cycloalkyle ou phényle; ou
un $C_{3-7}$ cycloalkyle, un phényle, un naphtyl ou un $-C(X)R_6$;
les groupes $C_{3-7}$ cycloalkyle et phényle étant éventuellement substitués par un ou plusieurs groupes choisis parmi un atome d'halogène, un hydroxy, un $C_{1-3}$ alkyle, un $C_{1-3}$ alcoxy, un phényl, un phénoxy, un benzyloxy ;

à la condition qu'au moins un groupe $R_4$ ou $R_5$ représente un groupe $-C(X)R_6$;
X représente un atome d'oxygène ou un atome de soufre ;
$R_6$ représente un groupe $C_{1-6}$ alcoxy, un hydroxy ou un groupe $-NR_7R_8$;
$R_7$ et $R_8$ représentent, indépendamment l'un de l'autre :

un atome d'hydrogène, un groupe $C_{1-6}$ alkyle éventuellement substitué par un $C_{3-7}$ cycloalkyle, un $C_{3-7}$ cycloalkènyle, un phényle ou un pyridinyle; ou
un $C_{3-7}$ cycloalkyle, $C_{1-6}$ alcoxy ou un phényle ; ou
$R_7$ et $R_8$, avec l'atome d'azote qui les porte, forment un cycle aziridine, azétidine, pyrrolidine, pipéridine ou morpholine ;
les groupes $C_{3-7}$ cycloalkyle et phényle étant éventuellement substitués par un groupe $C_{1-3}$ alkyle, un hydroxy, un $C_{1-3}$ alcoxy ou un atome d'halogène ;

à l'état de base, de sel d'addition à un acide, d'hydrate ou de solvat.

**4.** Composé de formule (I) selon l'une quelconque des revendications 1 à 3, **caractérisé en ce que** :

$R_1$ représente un $C_{1-4}$ alkyle ou un phényle substitué par deux atomes de fluor ;
$R_2$ représente un hydroxy et $R'_2$ représente un atome d'hydrogène ;
$R_3$ représente un $C_{1-4}$ alkyle ;
X représente un atome d'oxygène ;

à l'état de base, de sel d'addition à un acide, d'hydrate ou de solvat.

5. Procédé de préparation d'un composé de formule (I) selon l'une quelconque des revendications 1 à 4, comprenant l'étape consistant à

réaliser un couplage peptidique du 2-amino-thiazole de formule (III)

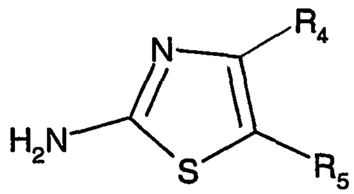

(III)

avec l'acylaminoacide de formule (II)

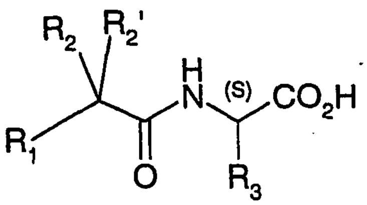

(II)

dans lesquelles $R_1$, $R_2$, $R'_2$, $R_3$, $R_4$, $R_5$ sont tels que définis dans la formule (I) de la revendication (I).

6. Procédé de préparation d'un composé de formule (I) selon l'une quelconque des revendications 1 à 4, comprenant l'étape consistant à
réaliser un couplage peptidique du composé de formule (IV)

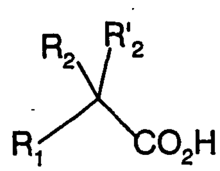

(IV)

avec l'amine de formule (VI)

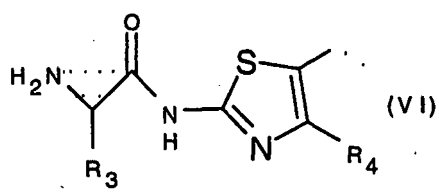

(VI)

dans lesquelles $R_1$, $R_2$, $R'_2$, $R_3$, $R_4$, $R_5$ sont tels que définis dans la formule (I) de la revendication (I).

7. Médicament **caractérisé en ce qu'**il comprend un composé de formule (I) selon l'une quelconque des revendications 1 à 4, à l'état de base, de sel, d'hydrate ou de solvat pharmaceutiquement acceptable.

8. Composition pharmaceutique contenant au moins un composé de formule (I) selon l'une quelconque des revendications 1 à 4, à l'état de base, de sel, d'hydrate ou de solvat pharmaceutiquement acceptable et éventuellement un ou plusieurs excipients pharmaceutiquement acceptables.

**9.** Utilisation d'un composé de formule (I) selon l'une quelconque des revendications 1 à 4, à l'état de base, de sel, d'hydrate ou de solvat pharmaceutiquement acceptable, pour la préparation d'un médicament destiné à traiter une pathologie dans laquelle un inhibiteur de la formation du peptide β-amyloïde β-A4 apporte un bénéfice thérapeutique.

**10.** Utilisation d'un composé de formule (I) selon l'une quelconque des revendications 1 à 4, à l'état de base, de sel, d'hydrate ou de solvat pharmaceutiquement acceptable, pour la préparation d'un médicament destiné à traiter la démence sénile, la maladie d'Alzheimer, le syndrome de Down, la maladie de Parkinson, l'angiopathie amyloïde et/ou les désordres cérébro-vasculaires.

## Claims

**1.** Compound conforming to the general formula (I):

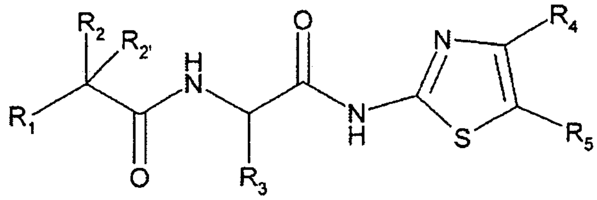

(I)

in which

$R_1$ represents:

a $C_{1-6}$ alkyl optionally substituted by one to three substituents selected from a halogen, a trifluoromethyl, a hydroxyl, a $C_{1-6}$ alkoxy, a $C_{1-6}$ thioalkyl, a thiophene or a phenyl; or
a $C_{3-7}$ cycloalkyl, a thiophene, a benzothiophene, a pyridinyl, a furanyl or a phenyl;
the phenyl groups being optionally substituted by one to three substituents selected from a halogen atom, a $C_{1-6}$ alkyl, a $C_{1-6}$ alkoxy, a hydroxyl, a methylenedioxy, a phenoxy or a benzyloxy;
$R_2$ and $R'_2$ represent independently of one another a hydrogen atom, a halogen atom, a hydroxyl, a $C_{1-3}$ alkoxy, a $C_{1-3}$ alkyl, a $C_{3-7}$ cycloalkyl, an O-C(O)-$C_{1-6}$ alkyl group, or $R_2$ and $R'_2$ together form an oxo group;

$R_3$ represents a hydrogen atom, a $C_{1-6}$ alkyl optionally substituted by a hydroxyl or a $C_{1-3}$ alkoxy;
$R_4$ and $R_5$ represent independently of one another:

a hydrogen atom, a $C_{1-7}$ alkyl optionally substituted by a $C_{3-7}$ cycloalkyl or phenyl; or a $C_{3-7}$ cycloalkyl, a phenyl, a naphthyl or a -C(X)$R_6$;
the $C_{3-7}$ cycloalkyl and phenyl groups being optionally substituted by one or more groups selected from a halogen atom, a hydroxyl, a $C_{1-3}$ alkyl, a $C_{1-3}$ alkoxy, a trifluoromethyl, a trifluoromethoxy, a phenyl, a phenoxy, a benzyloxy, a -$CH_2$O-phenyl, an -O$CH_2$-pyridinyl;
the benzyloxy group being optionally substituted by one to three groups selected from a halogen, a trifluoromethyl, a methyl;
on condition that at least one group $R_4$ or $R_5$ represents a group -C(X)$R_6$;

X represents an oxygen atom or a sulphur atom;
$R_6$ represents a $C_{1-6}$ alkoxy group, a hydroxyl or a group -$NR_7R_8$; the $C_{1-6}$ alkoxy group being optionally substituted by a phenyl;
$R_7$ and $R_8$, represent independently of one another:

a hydrogen atom, a $C_{1-6}$ alkyl group optionally substituted by a $C_{3-7}$ cycloalkyl, a $C_{3-7}$ cycloalkenyl, $C_{1-3}$ alkoxy, a phenyl, a morpholinyl or a pyridinyl; or
a $C_{3-7}$ cycloalkyl, $C_{1-6}$ alkoxy or a phenyl; or
$R_7$ and $R_8$, with the nitrogen atom which bears them, form an aziridine, azetidine, pyrrolidine, piperidine or

morpholine ring;
the $C_{3-7}$ cycloalkyl and phenyl groups being optionally substituted by one or two groups selected from a $C_{1-3}$ alkyl, a hydroxyl, a $C_{1-3}$ alkoxy or a halogen atom;
in the form of a base, addition salt with an acid, hydrate or solvate.

**2.** Compound according to Claim 1, **characterized in that**:

$R_1$ represents:

a $C_{1-5}$ alkyl, optionally substituted by one to three substituents selected from a fluorine, a trifluoromethyl, a hydroxyl, a $C_{1-4}$ thioalkyl, a thiophene or a phenyl; or
a $C_{4-7}$ cycloalkyl, a furanyl, a thiophene, a benzothiophene, a pyridinyl or a phenyl; the phenyl being optionally substituted by one to three substituents selected from a halogen atom, a hydroxyl, a benzyloxy or a methylenedioxy;
$R_2$ and $R'_2$ represent independently of one another a hydrogen atom, a halogen atom, a hydroxyl, a $C_{1-3}$ alkyl, a $C_{3-7}$ cycloalkyl, a $C_{1-3}$ alkoxy, an O-C(O)-$C_{1-4}$ alkyl group, or $R_2$ and $R'_2$ together form an oxo group;

$R_3$ represents a $C_{1-4}$ alkyl, optionally substituted by a $C_{1-3}$ alkoxy;
$R_4$ and $R_5$ represent independently of one another:

a hydrogen atom, a $C_{1-7}$ alkyl, optionally substituted by a phenyl or a $C_{3-7}$ cycloalkyl; or
a $C_{3-7}$ cycloalkyl, a phenyl, a naphthyl or a - $C(X)R_6$; the phenyl being optionally substituted by one or two groups selected from a halogen, a $C_{1-3}$ alkyl, a hydroxyl, a $C_{1-3}$ alkoxy, a trifluoromethyl, a trifluoromethoxy, a phenyl, a phenoxy, a benzyloxy, an $-CH_2O$-phenyl, a $-OCH_2$-pyridinyl;
the benzyloxy group being optionally substituted by a group selected from a halogen, a trifluoromethyl or a methyl;
on condition that at least one group $R_4$ or $R_5$ represents a group $-C(X)R_6$;

X represents an oxygen atom;
$R_6$ represents a $C_{1-6}$ alkoxy group, a hydroxyl or a group $-NR_7R_8$; the $C_{1-6}$ alkoxy group being optionally substituted by a phenyl;
$R_7$ and $R_8$ represent independently of one another:

a hydrogen atom, a $C_{1-3}$ alkyl group, optionally substituted by a $C_{1-3}$ alkoxy, a $C_{3-7}$ cycloalkenyl, a phenyl, a morpholinyl or a pyridinyl; a $C_{1-3}$ alkoxy; or
a $C_{3-6}$ cycloalkyl; or

$R_7$ and $R_8$, with the nitrogen atom which bears them, form an azetidine, piperidine or morpholine ring;
the phenyl group being optionally substituted by one or two $C_{1-3}$ alkoxy groups;
in the form of a base, addition salt with an acid, hydrate or solvate.

**3.** Compound of formula (I) according to Claim 1,
**characterized in that**:

$R_1$ represents:

a $C_{1-6}$ alkyl optionally substituted by one or two substituents selected from a hydroxyl, a $C_{1-6}$ alkoxy, a $C_{1-6}$ thioalkyl, a trifluoromethyl, a thiophene or a phenyl; or
a $C_{3-7}$ cycloalkyl, a thiophene, a benzothiophene, a pyridinyl, a furanyl or a phenyl;
the phenyl groups being optionally substituted by one to three substituents selected from a halogen atom, a $C_{1-6}$ alkyl, a $C_{1-6}$ alkoxy, a hydroxyl, a methylenedioxy, a phenoxy or a benzyloxy;

$R_2$ and $R'_2$ represent independently of one another a hydrogen atom, a halogen atom, a hydroxyl, a $C_{1-3}$ alkoxy, a $C_{1-3}$ alkyl, a $C_{3-7}$ cycloalkyl, an O-C(O)-$C_{1-6}$ alkyl group, or $R_2$ and $R'_2$ together form an oxo group;
$R_3$ represents a hydrogen atom or a $C_{1-6}$ alkyl optionally substituted by a hydroxyl or a $C_{1-3}$ alkoxy;
$R_4$ and $R_5$ represent independently of one another:

a hydrogen atom, a $C_{1-7}$ alkyl optionally substituted by a $C_{3-7}$ cycloalkyl or phenyl; or

a $C_{3-7}$ cycloalkyl, a phenyl, a naphthyl or a - C (X) $R_6$;
the $C_{3-7}$ cycloalkyl and phenyl groups being optionally substituted by one or more groups selected from a halogen atom, a hydroxyl, a $C_{1-3}$ alkyl, a $C_{1-3}$ alkoxy, a phenyl, a phenoxy, a benzyloxy;
on condition that at least one group $R_4$ or $R_5$ represents a group $-C(X)R_6$;

X represents an oxygen atom or a sulphur atom;
$R_6$ represents a $C_{1-6}$ alkoxy group, a hydroxyl or a group $-NR_7R_8$;
$R_7$ and $R_8$ represent independently of one another:

a hydrogen atom, a $C_{1-6}$ alkyl group optionally substituted by a $C_{3-7}$ cycloalkyl, a $C_{3-7}$ cycloalkenyl, a phenyl or a pyridinyl; or
a $C_{3-7}$ cycloalkyl, $C_{1-6}$ alkoxy or a phenyl; or
$R_7$ and $R_8$, with the nitrogen atom which bears them, form an aziridine, azetidine, pyrrolidine, piperidine or morpholine ring;
the $C_{3-7}$ cycloalkyl and phenyl groups being optionally substituted by a $C_{1-3}$ alkyl group, a hydroxyl, a $C_{1-3}$ alkoxy or a halogen atom;
in the form of a base, addition salt with an acid, hydrate or solvate.

4. Compound of formula (I) according to any one of Claims 1 to 3, **characterized in that**:

$R_1$ represents a $C_{1-4}$ alkyl or a phenyl substituted by two fluorine atoms;
$R_2$ represents a hydroxyl and $R'_2$ represents a hydrogen atom;
$R_3$ represents a $C_{1-4}$ alkyl;
X represents an oxygen atom;
in the form of a base, addition salt with an acid, hydrate or solvate.

5. Process for preparing a compound of formula (I) according to any one of Claims 1 to 4, comprising the step consisting in subjecting the 2-aminothiazole of formula (III)

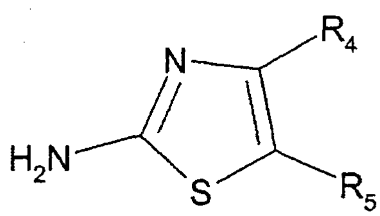

(III)

to peptide coupling with the acylamino acid of formula (II)

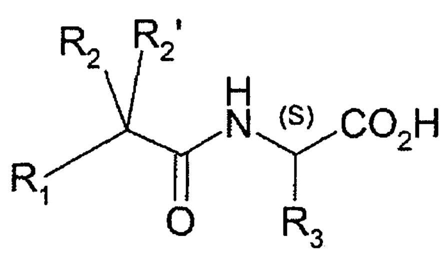

(II)

in which $R_1$, $R_2$, $R'_2$, $R_3$, $R_4$ and $R_5$ are as defined in the formula (I) of Claim 1.

6. Process for preparing a compound of formula (I) according to any one of Claims 1 to 4, comprising the step consisting in

subjecting the compound of formula (IV)

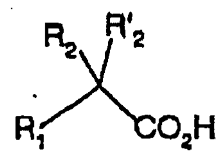

(IV)

to peptide coupling with the amine of formula (VI)

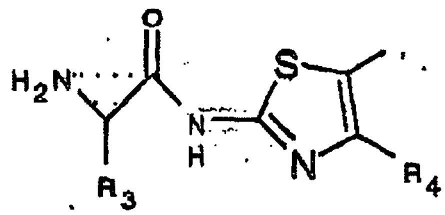

(VI)

in which $R_1$, $R_2$, $R'_2$, $R_3$, $R_4$ and $R_5$ are as defined in the formula (I) of Claim 1.

**7.** Medicament **characterized in that** it comprises a compound of formula (I) according to any one of Claims 1 to 4, in the form of a pharmaceutically acceptable base, salt, hydrate or solvate.

**8.** Pharmaceutical composition comprising at least one compound of formula (I) according to any one of Claims 1 to 4, in the form of a pharmaceutically acceptable base, salt, hydrate or solvate, and optionally one or more pharmaceutically acceptable excipients.

**9.** Use of a compound of formula (I) according to any one of Claims 1 to 4, in the form of a pharmaceutically acceptable base, salt, hydrate or solvate, for preparing a medicament intended for treating a pathology in which a β-amyloid peptide β-A4 inhibitor provides a therapeutic benefit.

**10.** Use of a compound of formula (I) according to any one of Claims 1 to 4, in the form of a pharmaceutically acceptable base, salt, hydrate or solvate, for preparing a medicament intended for treating senile dementia, Alzheimer's disease, Down's syndrome, Parkinson's disease, amyloid angiopathy and/or cerebrovascular disorders.

**Patentansprüche**

**1.** Verbindung nach der allgemeinen Formel (I):

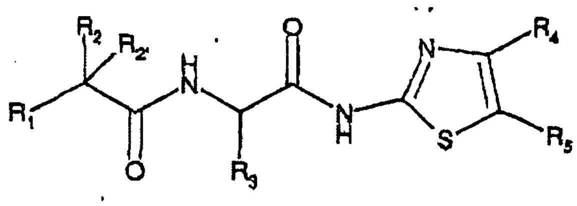

(I)

in welcher

$R_1$ für Folgendes steht:

ein $C_{1-6}$-Alkyl, welches möglicherweise mit einem bis drei Substituenten substituiert ist, die aus einem Halogen, einem Trifluormethyl, einem Hydroxyl, einem $C_{1-6}$-Alkoxy, einem $C_{1-6}$-Thioalkyl, einem Thiophen oder einem Phenyl gewählt sind; oder

ein $C_{3-7}$-Cycloalkyl, ein Thiophen, ein Benzothiophen, ein Pyridinyl, ein Furanyl oder ein Phenyl;

wobei die Phenylgruppen möglicherweise mit einem bis drei Substituenten substituiert sind, die aus einem Halogenatom, einem $C_{1-6}$-Alkyl, einem $C_{1-6}$-Alkoxy, einem Hydroxy, einer Methylendioxy, einem Phenoxy oder einem Benzyloxy gewählt sind;
$R_2$ und $R'_2$ unabhängig voneinander für ein Wasserstoffatom, ein Halogenatom, ein Hydroxyl, ein $C_{1-3}$-Alkoxy, ein $C_{1-3}$-Alkyl, ein $C_{3-7}$-Cycloalkyl, eine O-C(O)-$C_{1-6}$-Alkylgruppe stehen; oder $R_2$ und $R'_2$ gemeinsam eine Oxogruppe bilden;
$R_3$ für ein Wasserstoffatom, ein $C_{1-6}$-Alkyl, welches möglicherweise mit einem Hydroxyl oder einem $C_{1-3}$-Alkoxy substituiert ist, steht;
$R_4$ und $R_5$ unabhängig voneinander für Folgendes stehen:

ein Wasserstoffatom, ein $C_{1-7}$-Alkyl, welches möglicherweise mit einem $C_{3-7}$-Cycloalkyl oder Phenyl substituiert ist; oder
ein $C_{3-7}$-Cycloalkyl, ein Phenyl, ein Naphthyl oder ein -C(X)$R_6$;
wobei die $C_{3-7}$-Cycloalkyl- und Phenylgruppen möglicherweise mit einer oder mehreren Gruppen substituiert sind, welche aus einem Halogenatom, einem Hydroxyl, einem $C_{1-3}$-Alkyl, einem $C_{1-3}$-Alkoxy, einem Trifluormethyl, einem Trifluormethoxy, einem Phenyl, einem Phenoxy, einem Benzyloxy, einem -$CH_2$O-Phenyl, einem -$OCH_2$-Pyridinyl gewählt sind;
wobei die Benzyloxygruppe möglicherweise mit einer bis drei Gruppen substituiert ist, welche aus einem Halogen, einem Trifluormethyl, einem Methyl gewählt sind;
mit der Maßgabe, dass mindestens eine der Gruppen $R_4$ oder $R_5$ für eine -C(X)$R_6$-Gruppe steht;

X für ein Sauerstoffatom oder ein Schwefelatom steht;
$R_6$ für eine $C_{1-6}$-Alkoxygruppe, ein Hydroxyl oder eine -$NR_7R_8$-Gruppe steht; wobei die $C_{1-6}$-Alkoxygruppe möglicherweise mit einem Phenyl substituiert ist;
$R_7$ und $R_8$ unabhängig voneinander für folgendes stehen:

ein Wasserstoffatom, eine $C_{1-6}$-Alkylgruppe, die möglicherweise mit einem $C_{3-7}$-Cycloalkyl, einem $C_{3-7}$-Cycloalkenyl, $C_{1-3}$-Alkoxy, einem Phenyl, einem Morpholinyl oder einem Pyridinyl substituiert ist; oder
ein $C_{3-7}$-Cycloalkyl, $C_{1-6}$-Alkoxy oder ein Phenyl; oder
$R_7$ und $R_8$ mit dem Stickstoffatom, an welches sie gebunden sind, einen Aziridin-, Azetidin-, Pyrrolidin-, Piperidin- oder Morpholinring bilden;

wobei die $C_{3-7}$-Cycloalkyl- und Phenylgruppen möglicherweise mit einer oder zwei Gruppen substituiert sind, die aus einem $C_{1-3}$-Alkyl, einem Hydroxyl, einem $C_{1-3}$-Alkoxy oder einem Halogenatom gewählt sind;
in Form einer Base, eines Salzes, das bei Säurezusatz gebildet wird, eines Hydrates oder eines Solvates.

**2.** Verbindung gemäß Anspruch 1, **dadurch gekennzeichnet, dass**:

$R_1$ für Folgendes steht:

ein $C_{1-5}$-Alkyl, welches möglicherweise mit einem bis drei Substituenten substituiert ist, die aus einem Fluor, einem Trifluormethyl, einem Hydroxyl, einem $C_{1-4}$-Thioalkyl, einem Thiophen oder einem Phenyl gewählt sind; oder
ein $C_{4-7}$-Cycloalkyl, ein Furanyl, ein Thiophen, ein Benzothiophen, ein Pyridinyl oder ein Phenyl; wobei das Phenyl möglicherweise mit einem bis drei Substituenten substituiert ist, die aus einem Halogenatom, einem Hydroxy, einem Benzyloxy oder einem Methylendioxy gewählt sind;

$R_2$ und $R'_2$ unabhängig voneinander für ein Wasserstoffatom, ein Halogenatom, ein Hydroxyl, ein $C_{1-3}$-Alkyl, ein $C_{3-7}$-Cycloalkyl, ein $C_{1-3}$-Alkoxy, eine O-C(O)-$C_{1-4}$-Alkylgruppe stehen oder $R_2$ und $R'_2$ gemeinsam eine Oxogruppe bilden;
$R_3$ für ein $C_{1-4}$-Alkyl stehen, welches möglicherweise mit einem $C_{1-3}$-Alkoxy substituiert ist;
$R_4$ und $R_5$ unabhängig voneinander für Folgendes stehen:

ein Wasserstoffatom, ein $C_{1-7}$-Alkyl, welches möglicherweise mit einem Phenyl oder einem $C_{3-7}$-Cycloalkyl substituiert ist; oder
ein $C_{3-7}$-Cycloalkyl, ein Phenyl, ein Naphthyl oder ein -C(X)$R_6$; wobei das Phenyl möglicherweise mit einer

oder zwei Gruppen substituiert ist, die aus einem Halogen, einem $C_{1-3}$-Alkyl, einem Hydroxyl, einem $C_{1-3}$-Alkoxy, einem Trifluormethyl, einem Trifluormethoxy, einem Phenyl, einem Phenoxy, einem Benzyloxy einem -$CH_2$O-Phenyl, einem -$OCH_2$-Pyridinyl gewählt sind;

wobei die Benzyloxygruppe möglicherweise mit einer Gruppe substituiert ist, die aus einem Halogen, einem Trifluormethyl oder einem Methyl gewählt ist;
mit der Maßgabe, dass mindestens eine der Gruppen $R_4$ oder $R_5$ für eine -C(X)$R_6$-Gruppe steht;
X für ein Sauerstoffatom steht;
$R_6$ für eine $C_{1-6}$-Alkoxygruppe, ein Hydroxyl oder eine -$NR_7R_8$-Gruppe steht; wobei die $C_{1-6}$-Alkoxygruppe möglicherweise mit einem Phenyl substituiert ist;
$R_7$ und $R_8$ unabhängig voneinander für Folgendes stehen:

ein Wasserstoffatom, eine $C_{1-3}$-Alkylgruppe, die möglicherweise mit einem $C_{1-3}$-Alkoxy, einem $C_{3-7}$-Cycloalkenyl, einem Phenyl, einem Morpholinyl oder einem Pyridinyl substituiert ist; ein $C_{1-3}$-Alkoxy; oder ein $C_{3-6}$-Cycloalkyl; oder
$R_7$ und $R_8$ mit dem Stickstoffatom, an welches sie gebunden sind, einen Azetidin-, Piperidin- oder Morpholinring bilden;

wobei die Phenylgruppe möglicherweise mit einer oder zwei $C_{1-3}$-Alkoxygruppen substituiert ist;
in Form einer Base, eines Salzes, das bei Säurezusatz gebildet wird, eines Hydrates oder eines Solvates.

3. Verbindung nach Formel (I) gemäß Anspruch 1, **dadurch gekennzeichnet, dass**:

$R_1$ für Folgendes steht:

ein $C_{1-6}$-Alkyl, welches möglicherweise mit einem oder zwei Substituenten substituiert ist, die aus einem Hydroxyl, einem $C_{1-6}$-Alkoxy, einem $C_{1-6}$-Thioalkyl, einem Trifluormethyl, einem Thiophen oder einem Phenyl gewählt sind; oder
ein $C_{3-7}$-Cycloalkyl, ein Thiophen, ein Benzothiophen, ein Pyridinyl, ein Furanyl oder ein Phenyl;
wobei die Phenylgruppen möglicherweise mit einem bis drei Substituenten substituiert sind, die aus einem Halogenatom, einem $C_{1-6}$-Alkyl, einem $C_{1-6}$-Alkoxy, einem Hydroxyl, einer Methylendioxy, einem Phenoxy oder einem Benzyloxy gewählt sind;

$R_2$ und $R'_2$ unabhängig voneinander für ein Wasserstoffatom, ein Halogenatom, ein Hydroxyl, ein $C_{1-3}$-Alkoxy, ein $C_{1-3}$-Alkyl, ein $C_{3-7}$-Cycloalkyl, eine O-C(O)-$C_{1-6}$-Alkylgruppe stehen oder $R_2$ und $R'_2$ gemeinsam eine Oxogruppe bilden;
$R_3$ für ein Wasserstoffatom oder ein $C_{1-6}$-Alkyl, welches möglicherweise mit einem Hydroxyl oder einem $C_{1-3}$-Alkoxy substituiert ist, steht;
$R_4$ und $R_5$ unabhängig voneinander für Folgendes stehen:

ein Wasserstoffatom, ein $C_{1-7}$-Alkyl, welches möglicherweise mit einem $C_{3-7}$-Cycloalkyl oder Phenyl substituiert ist; oder
ein $C_{3-7}$-Cycloalkyl, ein Phenyl, ein Naphthyl oder ein -C(X)$R_6$;

wobei die $C_{3-7}$-Cycloalkyl- und Phenylgruppen möglicherweise mit einer oder mehreren Gruppen substituiert sind, welche aus einem Halogenatom, einem Hydroxyl, einem $C_{1-3}$-Alkyl, einem $C_{1-3}$-Alkoxy, einem Phenyl, einem Phenoxy, einem Benzyloxy gewählt sind;
mit der Maßgabe, dass mindestens eine der Gruppen $R_4$ oder $R_5$ für eine -C(X)$R_6$-Gruppe steht;
X für ein Sauerstoffatom oder ein Schwefelatom steht;
$R_6$ für eine $C_{1-6}$-Alkoxygruppe, ein Hydroxyl oder eine -$NR_7R_8$-Gruppe steht;
$R_7$ und $R_8$ unabhängig voneinander für Folgendes stehen:

ein Wasserstoffatom, eine $C_{1-6}$-Alkylgruppe, die möglicherweise mit einem $C_{3-7}$-Cycloalkyl, einem $C_{3-7}$-Cycloalkenyl, einem Phenyl, oder einem Pyridinyl substituiert ist; oder
ein $C_{3-7}$-Cycloalkyl, $C_{1-6}$-Alkoxy oder ein Phenyl; oder
$R_7$ und $R_8$ mit dem Stickstoffatom, an welches sie gebunden sind, einen Aziridin-, Azetidin-Pyrrolidin-, Piperidin- oder Morpholinring bilden;

wobei die $C_{3-7}$-Cycloalkyl- und Phenylgruppen möglicherweise mit einer $C_{1-3}$-Alkyl-Gruppe, einem Hydroxyl, einem $C_{1-3}$-Alkoxy oder einem Halogenatom substituiert sind;
in Form einer Base, eines Salzes, das bei Säurezusatz gebildet wird, eines Hydrates oder eines Solvates.

**4.** Verbindung nach Formel (I) gemäß einem beliebigen der Ansprüche 1 bis 3, **dadurch gekennzeichnet, dass**:

$R_1$ für ein $C_{1-4}$-Alkyl oder ein Phenyl, welches mit zwei Fluoratomen substituiert ist, steht;
$R_2$ für ein Hydroxyl steht und $R'_2$ für ein Wasserstoffatom steht;
$R_3$ für ein $C_{1-4}$-Alkyl steht;
X für ein Sauerstoffatom steht;
in Form einer Base, eines Salzes, das bei Säurezusatz gebildet wird, eines Hydrates oder eines Solvates.

**5.** Herstellungsverfahren für eine Verbindung nach Formel (I) gemäß einem beliebigen der Ansprüche 1 bis 4, welches einen Schritt umfasst, der darin besteht,

eine Peptidbindung zwischen dem 2-Aminothiazol nach Formel (III)

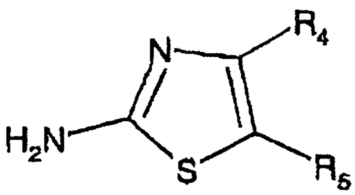

(III)

und der Acylaminosäure nach Formel (II)

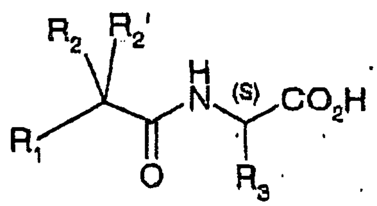

(II)

herzustellen, wobei $R_1$, $R_2$, $R'_2$, $R_3$, $R_4$, $R_5$ den Begriffsbestimmungen in der Formel (I) des Anspruchs (I) entsprechen.

**6.** Herstellungsverfahren für eine Verbindung nach Formel (I) gemäß einem beliebigen der Ansprüche 1 bis 4, welches einen Schritt umfasst, der darin besteht,
eine Peptidbindung zwischen der Verbindung nach Formel (IV)

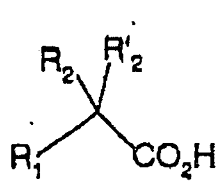

(IV)

und dem Amin nach Formel(VI)

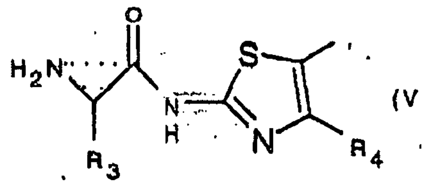

(VI)

herzustellen, wobei $R_1$, $R_2$, $R'_2$, $R_3$, $R_4$, $R_5$ den Begriffsbestimmungen in der Formel (I) des Anspruchs (I) entsprechen.

**7.** Arzneimittel, **dadurch gekennzeichnet, dass** es eine Verbindung nach Formel (I) gemäß einem beliebigen der Ansprüche 1 bis 4 umfasst, welche in Form einer Base, eines Salzes, eines Hydrates oder eines pharmazeutisch unbedenklichen Solvates vorliegt.

**8.** Pharmazeutische Zusammensetzung, welche mindestens eine Verbindung nach Formel (I) gemäß einem beliebigen der Ansprüche 1 bis 4, die in Form einer Base, eines Salzes, eines Hydrates oder eines pharmazeutisch unbedenklichen Solvates vorliegt, sowie möglicherweise einen oder mehrere pharmazeutisch unbedenkliche Trägerstoff(e) enthält.

**9.** Verwendung einer Verbindung nach Formel (I) gemäß einem beliebigen der Ansprüche 1 bis 4, welche in Form einer Base, eines Salzes, eines Hydrates oder eines pharmazeutisch unbedenklichen Solvates vorliegt, zur Herstellung eines Arzneimittels, das dazu bestimmt ist, ein Krankheitsbild zu behandeln, bei welchem ein Stoff, der die Bildung des β-amyloiden Peptides β-A4 hemmt, von therapeutischem Nutzen ist.

**10.** Verwendung einer Verbindung nach Formel (I) gemäß einem beliebigen der Ansprüche 1 bis 4, welche in Form einer Base, eines Salzes, eines Hydrates oder eines pharmazeutisch unbedenklichen Solvates vorliegt, zur Herstellung eines Arzneimittels, das dazu bestimmt ist, Altersdemenz, die Alzheimer-Krankheit, das Down-Syndrom, die Parkinson-Krankheit, die Amyloid-Angiopathie und/oder Störungen der Hirngefäße zu behandeln.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 9822430 A


### Littérature non-brevet citée dans la description


- **GREENE et al.** Protective groups in Organic Synthesis. John Wiley & Sons, Inc,
- **TAKEDA.** *Bull. Chem. Soc. JP,* 1970, vol. 43, 2997
- **A. BARTON ; BREUKELMAN, KAYE.** *J.C.S Perkin I,* 1982, 159
- **L. CROMBIE ; R.C.F. JONES ; C.J. PALMER.** *J.C.S Perkin Trans I,* 1987, 323
- **D.W. BROOKS ; L.D.-L. LU ; S. MASAMUNE.** *Angew. Chem. Int. Ed. Engl.,* 1979, vol. 18, 72
- *Tetrahedron Letters,* 2001, vol. 42, 2101
- **D.A. EVANS.** *J. C. S., Chem. Comm.,* 1973, 55
- **I. SHINN.** *J. Org. Chem.,* vol. 200 (65), 7667
- **WOLFE MS.** Secretase targets for Alzheimer's disease: identification and therapeutic potential. *J Med Chem.,* 21 Juin 2001, vol. 44 (13), 2039-60
- **WOLFE MS ; HAASS C.** The Role of presenilins in gamma-secretase activity. *J Biol Chem.,* 23 Février 2001, vol. 276 (8), 5413-6